(19) Europäisches Patentamt
European Patent Office
Office européen des brevets

(11) **EP 3 257 513 A1**

(12) **EUROPEAN PATENT APPLICATION**

(43) Date of publication:
**20.12.2017 Bulletin 2017/51**

(21) Application number: **17176645.4**

(22) Date of filing: **19.06.2017**

(51) Int Cl.:
*A61K 31/7004* (2006.01)   *A61K 31/198* (2006.01)
*A61K 31/704* (2006.01)   *A61K 38/00* (2006.01)
*A61P 1/16* (2006.01)   *A61P 25/00* (2006.01)
*A61P 25/32* (2006.01)   *A61P 37/04* (2006.01)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **18.06.2016   IN 201641020935**

(71) Applicant: **Chigurupati, Harsha Hyderabad 500033 (IN)**

(72) Inventors:
• **CHIGURUPATI, HARSHA 500033 Hyderabad (IN)**

• **BIYANI, MANISH RADHESHYAM 500033 Hyderabad (IN)**
• **AUDDY, BISWAJIT 500033 Hyderabad (IN)**
• **CHAKRABARTI, SHRABANA 500033 Hyderabad (IN)**

(74) Representative: **dompatent von Kreisler Selting Werner - Partnerschaft von Patent- und Rechtsanwälten mbB Deichmannhaus am Dom Bahnhofsvorplatz 1 50667 Köln (DE)**

(54) **COMPOSITION TO REDUCE DNA AND HEPATIC DAMAGE AND TO ENHANCE REPAIR THEREOF**

(57)    The invention relates to a composition to reduce DNA and hepatic damage and to enhance repair thereof. More particularly the invention relates to a synergistic composition comprising combination of active ingredients which can be used in a beverage composition and also relates to a beverage composition comprising said synergistic composition of active ingredients, wherein each active ingredient in the combination composition and/or beverage composition in appropriate concentration synergistically reduces the DNA damage as well as hepatic damage due to alcohol consumption and/or due to other reasons. The composition also enhances repair of the DNA and hepatic which has already been damaged. The composition also synergistically reduces hangover, modulates and/or alleviates immunology parameters and CNS parameters due to alcohol consumption and due to other reasons. Further a beverage composition comprising above synergistic composition and method of preparation thereof is provided.

Figure-1

STABILITY RESULTS FOR 45 DAYS (TABLE 4)

**Description**

**CROSS REFERENCE TO RELATED APPLICATION**

This application claims priority from Provisional Patent Application No. 201641020935 dated June 18, 2016, filed in Indian Patent Office.

**FIELD OF INVENTION**

The invention relates to a composition to reduce DNA and hepatic damage and to enhance repair thereof. More particularly the invention relates to a synergistic composition comprising combination of active ingredients which can be used in a beverage composition and also relates to a beverage composition comprising said synergistic composition of active ingredients, wherein each active ingredient in the combination composition and/or beverage composition in appropriate concentration synergistically reduces the DNA damage as well as hepatic damage due to alcohol consumption and/or due to other reasons. The composition also enhances repair of the DNA and hepatic which has already been damaged. The composition also synergistically reduces hangover, modulates and/or alleviates immunology parameters and CNS parameters due to alcohol consumption and due to other reasons. Further a beverage composition comprising above synergistic composition and method of preparation thereof is provided.

**BACKGROUND OF THE INVENTION**

Deoxyribo Nucleic Acid (DNA) which is an important part of the human cell constantly gets eroded or damaged by various chemicals and agents. This process is called DNA damage. The DNA suffers millions of damaging events each day. DNA being a part of the cell transmits genetic information to the next generation with high fidelity. (Tuteja, Narendra, Mohan B. Singh, Mithilesh K. Misra, Prem L. Bhalla, and Renu Tuteja. "Molecular Mechanisms of DNA Damage and Repair: Progress in Plants." Critical Reviews in Biochemistry and Molecular Biology 2001, 36,4, 337-97.).

Each of the approximately $10^{13}$ cells of the human body is constantly exposed to thousands of DNA lesions everyday from both endogenous and exogenous sources. The lesions block genome replication and transcription and if they are not repaired or are repaired incorrectly shall lead to mutations or wider-scale genome aberrations in important protein coding sequences. This leads to production of mutated protein and affects various biological processes leading to genome instability or organism viability. (Jackson, Stephen P., and Jiri Bartek. "The DNA-damage Response In Human Biology And Disease." Nature: 1071-078.)

**DNA Damage**

DNA damage has genotoxic and cytotoxic effects on the cell. The biological consequences of the damaged cells depend upon the chemical nature of the lesion. If the lesions occur in germ cells they are heritable and will be harmful to the next generation in passing on a heritable disease. Damage in somatic cells plays an important role in cancer and aging. In order to maintain the integrity of the genome, the prokaryotic and eukaryotic organisms are well equipped with several DNA repair mechanism pathways. (Tuteja, Narendra, Mohan B. Singh, Mithilesh K. Misra, Prem L. Bhalla, and Renu Tuteja. "Molecular Mechanisms of DNA Damage and Repair: Progress in Plants." Critical Reviews in Biochemistry and Molecular Biology 2001, 36,4, 337-97.).

**Types And Sources Of DNA Damage**

All livings cells of the human body are continuously challenged as they are sensitive to spontaneous hydrolysis, which leads to DNA damage. The free radical associated with oxidatively induced DNA damage chemical carcinogen typically is 8-oxo-2'-deoxyguanosine (8-OHdG). UV damage is associated with DNA single strand breaks (SSBs) or DNA double strand breaks (DSBs). Some DNA aberrations arise via physiological processes, such as a) DNA mismatches (occasionally during DNA replication), b) DNA strand breaks caused by abortive topoisomerase I and topoisomerase II activity c) Hydrolytic DNA damage associated with Reactive Oxygen Species (ROS) involves base deamination (apurinic and apyrimidinic sites) and d) non-enzymatic methylations generate thousands of DNA-base lesions per cell per day.

Reactive oxygen species (ROS) formed continuously as a consequence of by-products from oxidative respiration or through redox-cycling events involving environmental toxic agents and Fenton reactions mediated by heavy metals leads to DNA damage. Reactive oxygen and nitrogen species are also produced by macrophages and neutrophils at sites of inflammation and infections. Such chemicals attack DNA, leading to adduct that impair base pairing and/or block DNA replication and transcription, base loss, or DNA single-strand breaks (SSBs). It is estimated that about 100-500, 8-Hydroxy-2'-deoxyguanosine (8-OH-dG) lesions due to oxidative damage form per day in a human cell. The formamido pyrimidine lesions, 2, 6-diamino-4-hydroxy-5-formamidopyrimidine (FapyG) and 4,6-diamino-5-formamido pyrimidine are also formed at similar rates as 8-OH-dG after oxidative stress. (Dizdaroglu, Miral, Pawel Jaruga, Mustafa Birincioglu, and Henry Rodriguez. "Free Radical-induced Damage to DNA: Mechanisms and Measurement." This Article Is Part of a Series of Reviews on "Oxidative DNA Damage and Repair." Free Radical Biology and Medicine 2002, 32, 11, 1102-115.).

Apart from endogenous sources, DNA can also be damaged by exogenous agents from the environment. These include physical genotoxic stresses such as UV light from sunlight which induces a variety of mutagenic and cytotoxic DNA lesions such as cyclobutane-pyrimidine dimers (CPDs) and 6-4 photoproducts (6- 4PPs).

DNA damage in the form of double strand breaks (DSBs) is incurred as a result of medical treatments like radiotherapy, ionising radiation (IR) exposure from cosmic radiation. More recently, the radiation leakage from the Fukushima power plant in Japan are examples of other sources of severe exposure to exogenous radiation.

Chemical sources of DNA damage include chemotherapeutic drugs used in cancer therapy or for other medical conditions. Alkylating agents such as methyl methanesulfonate (MMS) induce alkylation of bases, whereas drugs such as mitomycin C, cisplatin and nitrogen mustard cause DNA interstrand cross links (ICLs), and DNA intrastrand cross links. Chemotherapeutic drugs like camptothecin and etoposide are topoisomerse I and II inhibitors respectively, and gives rise to SSBs or DSBs by trapping topoisomerase-DNA complexes.

Other exogenous DNA damaging sources, that are carcinogenic as well, are foods contaminated with fungal toxins, such as the aflatoxin and overcooked meat products containing heterocyclic amines. Another common source of environmental mutagen is tobacco smoke, which generates DNA lesions in the form of aromatic adducts on DNA and SSBs also micronutrient deficiency can mimic radiation (or chemicals) in damaging DNA by SSBs or DSBs, or oxidative lesions, or both. Those micronutrients whose deficiency mimics radiation are folic acid, $B_{12}$, $B_6$, niacin, C, E, iron, and zinc. ( Vaidehi Krishnan, Baohua Liu and Zhongjun Zhou. "DNA Repair, Human Diseases and Aging", DNA Repair and Human Health, Dr. Sonya Vengrova (Ed.), 2011, ISBN: 978-953-307-612-6, InTech,). ( Ames, Bruce N. "Micronutrient Deficiencies: A Major Cause of DNA Damage." Annals NY Acad Sci Annals of the New York Academy of Sciences: 87-106.)

**DNA damage detection strategies**

There are number of strategies such as PCR (polymerase chain reaction), comet assay, Enzyme-linked immunosorbent assay (ELISA), Radio immunoassay (RIA), etc., that are commonly used to detect DNA damage in various organisms. (Sunita Kumari, Rajesh P. Rastogi, Kanchan L. Singh, Shailendra P. Singh and Rajeshwar P. Sinha. "DNA Damage: Detection Strategies" EXCLI Journal 2008; 7:44-62.)

**Types of DNA repair**

There are four major classes of DNA repair pathways. They are 1. Direct repair 2. Base excision repair 3. Nucleotide excision repair and 4. Mismatch repair.

An example of direct repair is $O^6$-methylguanine methyltransferase. Base excision repair pathway addresses ethenobase adducts. Nucleotide excision repair addresses malondialdehyde-dG lesion. Mismatch repair system is primarily responsible for repair of DNA replication errors. DNA replication after chronic alcohol abuse burdens the mismatch repair system.

**Ailments caused due to DNA damage**

Cells posses a mechanism called DNA Damage Response (DDR) to detect DNA lesions, signal their presence and promote their repair. Cells defective in these mechanisms display heightened sensitivity towards DNA damaging agents and many such defects cause human disease. (Jackson, Stephen P., and Jiri Bartek. "The DNA-damage Response In Human Biology And Disease." Nature: 1071-078.) Different diseases like cancer, atherosclerosis, diabetes, Alzheimer's disease are caused by changes in biomolecules like DNA. Other serious chronic diseases including ocular disorders such as cataract, glaucoma and age-related macular degeneration (AMD) are related to changes in biomolecules like DNA.

In a model for the DDR, the presence of a lesion in the DNA, which leads to replication stalling, is recognized by various sensor proteins. These sensors initiate signaling pathways that have an impact on a wide variety of cellular processes.

**DNA damage and cancer:**

Among the targets of ROS, DNA appears most important in tumor biology since it is firmly established that cancer is a genetic disease. Elevated levels of damage are purported to arise as a consequence lower levels in antioxidant enzymes and higher levels of ROS in an environment around tumor. It has been reported that at least some tumor cell lines can produce significant levels of $H_2O_2$, without exogenous stimulation, perhaps accounting for the elevated levels of oxidative DNA damage seen. As a result of elevated ROS, transcription factors and their corresponding genes are permanently activated, which is coupled with increased DNA damage, thus creates a selection pressure for a malignant phenotype seen in cancer.

Oxidative mechanisms have been demonstrated to possess a potential role in the initiation, promotion, and malignant conversion (progression) stages of carcinogenesis. ROS induce several kinds of DNA damage, including strand breakage, base modification and DNA-protein cross-linkage. Supportive of this proposal are the findings that G:C to T:A transversion potentially derived from 8-Hydroxy-2'-deoxyguanosine (8-OH-dG) have been observed in vivo in the ras oncogene and the p53 tumor suppressor gene in lung and liver cancer. G:C to T:A transversion is not unique to 8-OH-dG, whereas C:C to T:T substitutions in the absence of UV in internal tumors has been identified as signature mutations for ROS. (Cooke, M. S.. "Oxidative DNA Damage: Mechanisms, Mutation, And Disease". The FASEB Journal 2003, 17, 10, 1195-1214.)

## Neurodegeneration and DNA damage

Neurodegeneration is attributed to many nervous system and aging diseases, such as Ataxia Telangiectasia (AT), Nijmegen breakage syndrome (NBS), Huntington's disease (HD), Parkinson's disease (PD) and Alzheimer's disease (AD). Furthermore, deficiency in repair of nuclear and mitochondrial DNA damage has been linked to several neurodegenerative disorders. Many recent experimental results indicate that the post-mitotic neurons are particularly prone to accumulation of unrepaired DNA lesions and potentially lead to progressive neurodegeneration. One reason for this may be that neurons generally exhibit high mitochondrial respiration and associated ROS production that can damage mitochondrial and nuclear DNA. Another reason why the nervous system is particularly vulnerable to DNA damage is its limited capacity for cell replacement in adulthood, potentially leading to accumulation of damaged but irreplaceable terminally differentiated neurons. Neurons are dependent on transcription level and oxidative DNA damage could block this. Thus, accumulation of DNA lesions in repair-defective patients and possibly in ageing normal individuals progressively deprive neurons of vital transcripts, leading to cell dysfunction or apoptosis. (Jackson, Stephen P., and Jiri Bartek. "The DNA-damage Response In Human Biology And Disease." Nature: 1071-078.)(Souza-Pinto, Nadja C. De, David M. Wilson, Tinna V. Stevnsner, and Vilhelm A. Bohr."Mitochondrial DNA, Base Excision Repair and Neurodegeneration." DNA Repair 7.7 2008, 1098-109.)

Chronic ethanol exposure increases NMDA receptor binding in brain cells, which increases the susceptibility of cerebellar granule cells to glutamate toxicity. Ethanol withdrawal increased neuronal excitability and neurodegenration even for low doses of NMDA. Thus NMDA plays key role in neurotoxic effects of ethanol withdrawal. The excitotoxic effects of NMDA receptor activation involves the generation of ROS. This suggests a possible role of DNA damage as consequence of ROS and lipid peroxidation due to elevated levels of CYP2E1 and /or NMDA receptor activation during chronic ethanol exposure and withdrawal.

## Ageing, stem-cell dysfunction, cardiovascular disease and metabolic syndrome

Ageing is in part caused by accumulated DNA damage. Various endogenously arising DNA lesions accumulate with age in the nuclear and mitochondrial genomes of healthy mammals, including humans. This reflects not only ongoing DNA-damage induction but also declining DNA-repair capacity over time. Patients with inherited DDR defects display features of premature ageing. Work in various organisms has implicated growth hormone and insulin-like growth factor 1 signaling in regulating longevity, and notably, such signaling is down regulated in response to DNA damage.

Cell senescence and apoptosis are suspected causes of ageing under conditions where attempted tissue regeneration causes stem-cell exhaustion. Indicative of DNA damage contributing to such episodes, impaired stem-cell function is exhibited in mice with defects in the Fanconi anaemia, nucleotide excision repair (NER), mismatch repair or Non homologous end joining (NHEJ) pathways. Furthermore, whereas p53-induced cell death protects against tumorigenesis, pro-apoptotic p53 activity is harmful in settings such as stroke or heart attack. Induction of p53 by oxidative stress and other sources of DNA damage can also affect the development of atherosclerosis, thus providing a link between the DDR and cardiovascular disease. Indeed, growing evidence points to human atherosclerosis being characterized by enhanced DNA damage and DDR signaling, leading to senescence of vascular smooth muscle cells and death of other cells to yield atherosclerotic lesions. Modulating ROS production and the DDR therefore represent potential therapeutic opportunities for atherosclerosis.

Metabolic syndrome is a condition characterized by aberrant glucose metabolism, insulin resistance and atherosclerosis. Ataxia-telangiectasia mutated (ATM)-defective patients commonly exhibit insulin resistance and glucose intolerance, whereas mice heterozygous or homozygous for ATM mutations display features associated with metabolic syndrome and atherosclerosis. DDR-regulated kinases target multiple substrates involved in glucose metabolism. Although some linkages between the DDR and metabolic syndrome might be indirect, it is possible that the DDR directly modulates certain aspects of energy metabolism and vascular physiology of relevance to metabolic syndrome. (Jackson, Stephen P., and Jiri Bartek. "The DNA-damage Response In Human Biology And Disease." Nature: 1071-078.)

**Inflammation/infection**

The association between inflammation and oxidative stress is well documented, with studies of inflammatory conditions or infections reporting elevated levels of 8-OH-dG: hepatitis, hepatitis C infection and atopic dermatitis. Bactericidal species ($O^{2\bullet-}$ and $H_2O_2$), generated from the respiratory burst of invading neutrophils, macrophages, and eosinophils damage surrounding tissue, further initiating radical reactions leading to oxidative stress. Chronic inflammation and oxidative stress are closely linked to the pathogenesis of such autoimmune diseases as rheumatoid arthritis and systemic lupus erythematosus (SLE), causing radical species production, resulting into connective tissue damage and postulated to be the antigen driving autoantibody production. Mechanistically, chronic inflammation is closely linked to carcinogenesis. Tumor promoters report to recruit inflammatory cells that, with their potential to generate ROS, may provide the appropriate stimuli to lead to promotion. (Cooke, M. S.. "Oxidative DNA Damage: Mechanisms, Mutation, And Disease". The FASEB Journal 2003, 17, 10, 1195-1214.)

**Immune deficiencies**

Genome rearrangements involving DDR factors occur during immune-system development, and these DDR defects cause immune deficiency. Mutations in Non homologous end joining (NHEJ) factors yield B- and T-cell immune deficiency, while ataxia telangiectasia (AT) and Nijmegen breakage syndrome (NBS) patients (defective in ATM and NBS1, respectively) are prone to sometimes fatal infections, partly due to impaired immunity (class-switch recombination is particularly affected in AT patients). (Jackson, Stephen P., and Jiri Bartek. "The DNA-damage Response In Human Biology And Disease." Nature: 1071-078.)

B- and T-cells are the main components of the adaptive immune system and responsible for the generation of B cell receptors (BCRs, also known as immunoglobulins) and T cell receptors (TCRs), which together recognize a large repertoire of antigens. During the development of antigen receptor genes, a large number of variable (V), diversity (D), joining (J) and constant (C) segments undergo rearrangement by processes termed as V(D)J recombination and class switch recombination (CSR). Many cancers arising in such conditions are lymphomas and leukemia of B- and T-cell origin that result from impaired V(D)J recombination.

**Alcohol induced DNA damage**

Chronic alcohol consumption and metabolism result in the generation of several classes of DNA-damaging molecules, including reactive oxygen species (ROS), lipid peroxidation products, and acetaldehyde. Ethanol induction of CYP2E1 increases generation of ROS. DNA strand breaks occurs by means of hydroxyl radical, as it is very sensitive to hydroxyl radical, and which produces over 20 different types of DNA base damage, with diverse biological properties. The hydroxy radical do not diffuse the nucleus but it is generated via $H_2O_2$ with metal (fenton reaction) as $H_2O_2$ are highly diffusable in nucleus and produce DNA damage. Apart from that chronic alcoholism causes vitamin and other micro-nutrient deficient state that further aggravates the condition.

Malondialdehyde is the major product generated due to lipid peroxidation on ethanol consumption. Malondialdehyde reacts with guanosine residues in DNA to form malondialdehyde-deoxyguanosine (dG) adduct which is mutagenic and carcinogenic. Another mutagenic lipid peroxidation product due to chronic ethanol consumption is hydroxynonenal (HNE) DNA adducts. Etheno base adducts derived from lipid peroxidation are known carcinogens and mutagens. The mechanism of the mutagenicity of acetaldehyde involves direct attack of acetaldehyde on DNA to give $N^2$-ethyl-deoxyguanosine ($N^2$-Et-dG) even on moderate consumption of ethanol.

**Alcohol induced Liver disease**

Alcoholism is a common substance-abuse disorder that leads to significant medical complications. Alcohol affects virtually every organ system, and alcoholics are at increased risk for alcoholic liver diseases, gastrointestinal bleeding, pancreatitis, cardiomyopathy, trauma, mental health disorders, and a wide variety of cancers. Alcoholism is a major health problem throughout the world, with approximately 10% of the adult population in the United States suffering from alcoholism or its complications. (Schoppet, M., and Maisch, B. Alcohol and the heart. Hertz 2001, 26, 345-352.)

The forms of alcoholic liver diseases (ALD) are simple fatty liver (steatosis), fatty liver accompanied by inflammation (steatohepatitis) leading to scar tissue formation (fibrosis), and the destruction of the normal liver structure (liver cirrhosis), which may or may not improve with abstinence and subsequently might lead to liver cancer (hepatocellular carcinoma).(Kumar, Cotran, Robbins. Robbins Basic Pathology 7th ed. Philadephlia: Saunders; 2003.) Hepatic metabolism of ethanol accounts for nearly 90-95% of absorbed ethanol. Only 2-10% of absorbed ethanol is eliminated through the kidneys and lungs and extra hepatic metabolism is limited to the other organs including stomach and brain.

The mechanisms involved by which alcohol causes cell injury are complex and combination of several interrelated pathways. A major mechanism involves the role of lipid peroxidation and oxidative stress in alcohol toxicity. (Nordmann, R.; Ribiere, C.; Rouach, H..Implication of free radical mechanisms in ethanol-induced cellular injury. Free Radic. Biol. Med. 1992, 12, 219-240.) ROS generation is linked to the metabolism of alcohol. Three metabolic pathways of alcohol have been identified, which involve the following enzymes: alcohol dehydrogenase, microsomal ethanol oxidation system (MEOS) which is dependent on Cytochrome P450 2E1 (CYP2E1) and catalase. Each of these pathways could produce free radicals that affect the antioxidant system. These free radicals oxidize cellular biomolecules, such as proteins, DNA and initiate membrane peroxidative degradation (Mutlu-Turkoglu U, Dogru-Abbasoglu S, Aykac-Toker G, Mirsal H, Beyazyurek M, Uysal M. Increased lipid and protein oxidation and DNA damage in patients with chronic alcoholism. J Lab Clin Med, 2000, 136, 287-291.) The increase in transaminase enzymes (ALT and AST) indicate cellular leakage and loss of functional integrity of cell membrane.(Yue M, Yu CH, Ren K, Chen W, Li Y. Transient elevation of hepatic enzymes in volunteers after intake of alcohol. Hepatobiliary and Pancreatic Diseases Interna-tional 2006, 5, 1, 52-55.).

Alcohol, when consumed in low to moderate amount, is mainly catalysed by alcohol dehydrogenase to form acetaldehyde which results in the formation of free radicals with concomitant changes in the NADH levels and NADH/NAD$^+$ redox ratios.( Mantle, D., Preedy, V.R.. Free radicals as mediators of alcohol toxicity. Adverse Drug Reactions and Toxicological Reviews 1999, 18, 4, 235-252.) Acetaldehyde produced through MEOS initially represents a minor pathway of ethanol oxidation, probably accounting for less than 10% of the liver capacity to oxidize ethanol. (Lieber, C. S. and DeCarli, L. M.The role of the hepatic microsomal ethanol oxidizing system (MEOS) for ethanol metabolism in vivo. J. Pharmacol. Exp. Ther. 1972, 181, 279-287.) However, the activity of MEOS is enhanced after chronic alcohol consumption, due to induction of CYP2E1. (Lieber, C. S. Cytochrome P4502E1: its physiological and pathological role. Physiol. Rev. 1997, 77, 517-544.)Thus, CYP2E1 is a major contributor to ethanol-induced oxidant stress and to ethanol-induced liver injury.

Variety of enzymatic and nonenzymatic mechanisms are present to protect cells against ROS, including the superoxide dismutases (SOD), which remove $O_2^{-}$; Catalase (CAT) and the glutathione peroxidise (GPX) system, which remove $H_2O_2$ and non-enzymatic low-molecular-weight antioxidants such as GSH, vitamin E, vitamin C, vitamin A, ubiquinone, uric acid, and bilirubin.( Halliwell, B. Antioxidant defence mechanisms: from the beginning to the end. Free Radic. Res. 1999, 31:261-272.) Apart from that, ROS generated by endotoxin-activated Kupffer cells affects mitochondria leading to hepatocytes necrosis and apoptosis. ( Conde de la Rosa et al. Superoxide anions and hydrogen peroxide induce hepatocytes death by different mechanisms: Invovement of JNK and ERK MAP kinase. J Hepatol, 2006, 44, 5, 918-929.) The other plausible pathway of alcohol induced hepatotoxicity includes excess production of proinflammatory cytokines by gut-endotoxin stimulated Kupffer cells. (Arai M, Nakano S, Okuno F et al. Endotoxin-induced hypercoagulability: a possitive aggravating factor of alcoholic liver disease. Hepatology 1989, 9, 6, 846-851.)

**Alcohol induced Immunology Factors**

Glycyrrhizin inhibits high mobility group protein B1 (HMGB1) and also acts as an toll like receptor 4 (TLR4) antagonist as well as an inhibitor of microglial activation. Ethanol induced expression is provided for pro-inflammatory cytokines like TNF- $\alpha$ and IL-1b.

(Crews et al.)

Alcohol consumption increases significantly the values of cytokines like Interleukin (IL), IL-10, IL-12 and IFN-$\alpha$ , 13 hours after alcohol consumption. An increase is seen in production of IL-10, as a response to pro-inflammatory cytokine production. This indicates that there is impaired cellular immunity during veisalgia. (Kim et al. "Effects of Alcohol Hangover on Cytokine Production in Healthy Subjects." Alcohol, 31, 3, 2003, 167-170).

Natural killer (NK) cells are lymphoid cells poised and ready to assist in the destruction of virally infected cells and tumor cells from the body and can mediate their effect in an antigen independent manner. NK cells become activated upon stimulation by the cytokines IL-15, IL-18, IL-12, IL-17, IL-22, IFN$\gamma$ and TNF$\alpha$.

Terunuma, H et al., (2008). Potential role of NK cells in the induction of immune responses: implications for NK cell-based immunotherapy for cancers and viral infections. International Reviews of immunology, 27 (3): 93-110.

Many components have been reported so far in the composition of food or beverages to provide for DNA protection and/or DNA repair, hepatic stress and protection from pancreatic damage.

Different components like sugar alcohol, saponin glycoside, hydrolysable tannin, and Amino-Acid-Derived-Molecules are used in combination with alcohol to provide for DNA protection, protection from pancreatic stress and for hepato protection.

Appropriate concentration of above said compounds is useful for protection from DNA damage, hepatic and pancreatic stress. The exact amounts of compounds to be used in the composition to exert optimum DNA protection and repair and for protection from pancreatic stress and for hepato protection have not been reported. Therefore a composition comprising more than one compounds having DNA repair activity, hepatic and pancreatic protection wherein the appropriate amount of each compounds in the combination showing synergistic DNA protection and DNA repair activity is still to be found out. Similarly a combination of products which would provide protection from pancreatic stress and for hepato protection is also to be found out.

Following mentioned patent literature mentions combinations of different compounds which are reported for having DNA protection and/or DNA repair:

EP2218449 discloses use of mannitol in treatment of genome disorders and for DNA damage repair. It is used in treatment of the consequences of ageing and in genome maintenance disorders.

WO2007133076 discloses a combination of mannitol and proline in treatment of DNA damage. The combination is used to treat subjects suffering from genetic defects and ageing.

US8911774 discloses a composition which comprises glycyrrhizin in a concentration range from 0.005 % to 0.3 % (w/w). This composition provides DNA repair in the nucleus and in the mitochondria.

US8158608B2 discloses a fraction from Zingiber officinale which exhibits DNA protection activity. The fraction of Zingiber officinale comprises 42%-46% of gallic acid or 42%-46% of tannic acid.

US 4987123 discloses L-alanine and L-glutamine which is available in molar ratio of 1:0.1 to 1:10 or oligopeptide for hepatic disorders.

US 20100234308 discloses oligopeptide Alanylglutamine, a dipeptide containing two amino acids, alanine and glutamine. Each may be L- or D-forms, and the L-forms are preferred for wake up remedy

US 4596825 molar ratio of ornithine to alanine in said mixture is about 1:0.001 to 10, amount effective to prevent or alleviate said alcoholic liver disturbance.

The article titled 'Effects of tannic acid and its related compounds on food mutagens or hydrogen peroxide-induced DNA strands breaks in human lymphocytes' (Mutation Research/Fundamental and Molecular Mechanisms of Mutagenesis, Volume 556, Issues 1-2, 22 November 2004, Pages 75-82) discloses that tannic acid and its related components decreased the DNA strand breaks at a particular concentration.

The article titled 'Suppressing effect of tannic acid on the frequencies of mutagen-induced sister-chromatid exchanges in mammalian cells' (Mutation Research/Fundamental and Molecular Mechanisms of Mutagenesis, Volume 213, Issue 2, August 1989, Pages 195-203) discloses that tannic acid modifies DNA excision repair.

The article titled 'Effect of tannic acid, resveratrol and its derivatives, on oxidative damage and apoptosis in human neutrophils' (Food and Chemical Toxicology, Volume 84, October 2015, Pages 37-46) discloses the effect of tannic acid on DNA protective activity.

The article titled 'The efficacy of protective effects of tannic acid, gallic acid, ellagic acid, and propyl gallate against hydrogen peroxide-induced oxidative stress and DNA damages in IMR-90 cells' (Molecular Nutrition & Food Research, Volume 51, Issue 8, pages 962-968, August 2007) disclose the effect of the four components tannic acid, gallic acid, ellagic acid and propyl gallate in protection against DNA damage.

The article titled 'Ellagic acid induces apoptosis through inhibition of nuclear factor κB in pancreatic cancer cells' (World J Gastroenterol. Jun 21, 2008; 14(23): 3672-3680, Published online Jun 21, 2008. doi: 10.3748/WJG.14.3672) discloses the effect of ellagic acid on pancreatic cancer cells.

The article titled 'Oxyradicals and DNA damage' (Carcinogenesis, Volume 21, Issue 3, Pp. 361-370) disclose the effect of DNA damage on the pancreas.

It is observed that in prior art different components individually are reported, but it is observed that a combination of components are not reported in combination.

As mentioned in the above prior arts, DNA damage as well as pancreatic and hepatic stress is increasing due to changing and challenging environmental factors leading to variety of health problems and it is a major risk factor. Further people in the modern life style are in the habit of moderate to heavy alcohol consumption. Since alcohol is an exogenous factor for inducing DNA damage and pancreatic and hepatic stress, leading to disease such as Alcohol Induced Liver Disease and/or Alcohol Induced Pancreatic Disease, there it is necessary to develop such beverage composition with compounds having DNA repair and protection activities synergistically to reduce and avoid such diseases and complications arises due to its consumption.

Beverage, especially alcoholic drink inducing DNA damage should be reduced to reduce such health risk. One method to achieve this target and provide DNA protection and repair is to add a composition of suitable compounds having DNA protection and repair activities in an appropriate amount of each compound so that, optimum DNA protection and DNA repair can be achieved providing maximum health benefit, which also provides protection against pancreatic and hepatic stress.

In the above cited prior arts though the compounds having DNA protection and DNA repair have been mentioned, specific concentration range in terms of quantity of compound to be used to provide maximum health benefit either individually or in combination with synergetic activity is not mentioned. The ranges of the compounds are not clearly defined. The purity in case of extracts is not defined. Certain combinations are not reported specifically for DNA damage and for pancreatic and hepatic stress.

The present invention provides a novel composition of compounds having DNA protection and/or repair activities along with hepatic and pancreatic repair, wherein the composition is to be added in the alcoholic beverage and wherein each compound in the composition comprises in an appropriate quantity/amount so that, addition of the combination of compounds in such appropriate quantity in specific ratios in the composition exerts synergistic DNA protection and/or DNA repair activity, along with hepatic and pancreatic protection. Thus the present invention provides a useful synergistic alcoholic beverage composition to reduce the harmful effect of alcohol in human body.

## OBJECTS OF THE INVENTION

One object of the invention is to provide a composition to reduce DNA damage due of varied etiology including alcohol consumption.

Another object of the invention is to provide a composition to reduce hepatic (liver) damage due to varied etiology including alcohol consumption.

Another object of the invention is to provide a composition for reduction of DNA damage in the body due to endogenous or exogenous factors.

Another object of the invention is to provide a composition for reduction of hepatic (liver) damage in the body due to endogenous or exogenous factors.

Another object of the invention is to provide a composition which repairs and/or enhances repair of the DNA damage.

Yet another object of the invention is to provide a composition which repairs and/or enhances repair of the hepatic (liver) damage.

A further object of the invention is to provide a composition which reduces vesalgia/hangover.

A further object of the invention is to provide a composition which modulates immunological parameters

A further object of the invention is to provide a composition which reduces CNS stress.

Yet another object of the invention is to provide a composition which repairs and/or enhances repair of the immune system and CNS damage.

A further object of the invention is to provide a method for preparation of such composition.

## SUMMARY OF THE INVENTION

Accordingly the invention provides synergistic composition for reducing DNA and Hepatic damage, reducing/relieving hangover/vesalgia, enhancing DNA and Hepatic repair and modulating/alleviating immunology and CNS parameters, which can be used as a beverage additive for drink such as alcoholic beverage or can be used in a beverage composition such as alcoholic drink composition directly at the time of preparation. The preparation can be prepared in many forms such as liquid, semi solid, powder, solid etc.

Broadly the above described additive compositions and/or beverage composition comprising said additive composition, comprises following all or some of the active ingredients, optionally with or without one or more of non-active ingredients in amount ranging as below:

(a) hydrolyzable tannin or pyrogallol-type tannin compounds may be used in mass concentration range of 0.0001 to 2.0%;
(b) the amino acid derived molecule may be used in mass concentration range of 0.01 to 5.0%;
(c) sugar or sugar alcohol may be used in mass concentration range of 0.01 to 5.0 %; and
(d) the saponin glycoside which comprises Glycyrrhizin or its isomers or its derivatives may be used in a mass concentration range of 0.01 to 2.0 %;

- Optionally, quantum sufficient of water, and/or quantum sufficient of alcohol or quantum sufficient mixture of water and alcohol;
- Optionally, one or more of pH adjusting agents may be used in mass concentration range of about 0.01 to 0.2%;
- Optionally, one or more of flavouring agent may be used in mass concentration range of about 0.01 to 0.2%;
- Optionally, any other non-active ingredients at appropriate amount as may be required based on the composition.

All the above concentrations are in mass concentration or weight by volume (wt./v%).

In one aspect the invention provides a synergistic composition for reducing DNA and Hepatic damage, reducing/relieving hangover/vesalgia, enhancing DNA and Hepatic repair and modulating/alleviating immunology and CNS parameters comprising of:

(a) hydrolyzable tannin or pyrogallol-type tannin compounds in mass concentration range of 0.00035% to 0.025% and at least two active ingredients selected from:
(b) an amino acid derived molecule in mass concentration range of 0.1% to 2.0%;
(c) sugar alcohol in mass concentration range of 0.5% to 2.5 %; and
(d) saponin glycoside in mass concentration range of 0.01 % to 0.3 %.

In one embodiment the above composition is selected from:

(a) a composition comprising hydrolyzable tannin or pyrogallol-type tannin compounds, amino acid derived molecule and sugar alcohol;
(b) a composition comprising hydrolyzable tannin or pyrogallol-type tannin compounds, amino acid derived molecule and saponin glycoside;
(c) a composition comprising hydrolyzable tannin or pyrogallol-type tannin compounds, sugar alcohol and saponin glycoside; and
(d) a composition comprising hydrolyzable tannin or pyrogallol-type tannin compounds, amino acid derived molecule, sugar alcohol and saponin glycoside.

In above composition the hydrolyzable tannin or pyrogallol-type tannin compounds is selected from the group consisting of tannic acid, epigallitannins, gallotannins, ellagitannins, condensed tannin, a complex tannin and hydrolyzable tannin or a combination thereof.

In one embodiment tannic acid is selected from group consisting of Tanal 01, Tanal 02 and Tanal B.

Amino-acid derived molecule is selected from the group consisting of an amino-acid monomer, a dipeptide, a tripeptide, an oligopeptide, a polypeptide and a protein hydrolysate or a combination thereof.In one embodiment the amino-acid monomer is selected from the group consisting of alanine, glutamine, arginine, ornithine, arginine pryoglutamate, asparaginine, L-Aspartic acid, D-Asparatic acid, L-Carnitine, citruline, cysteine, gamma-aminobutyric acid (GABA), glutathione, glycine, histidine, L-isoleucine, L-leucine, L-lysine, methionine, phenylalanine, L-proline, pyroglutamate, serine, threonine, tyrptophan, tyrosine, L-valine, and L-Theanine or a combination thereof. In one embodiment the dipeptide (DP) is selected from the group consisting of L-alanyl-L-glutamine (L-Ala-L-Gln), glycyl-glycine (Gly-Gly) and L-glutamyl-L-alanine (Glu-Ala) or a combination thereof.In one embodiment the oligopeptide is selected from the group consisting of Oxidised L-Glutathione (GSSG), Reduced L-Glutathione and Glutathione or a combination thereof.In one embodiment the protein hydrolysate is selected from hydrolysed casein protein (CP) and hydrolysed whey protein (WP) or a combination thereof.In one embodiment the sugar alcohol is selected from the group consisting of D-Glycerol, L-Glycerol, D-Mannitol, L-Mannitol, D-erythritol, L-erythritol, D-xylitol, or L-xylitol, L-Maltitol, D-Maltitol, L-Sorbitol, D-Sorbitol, L-Lactitol, D-Lactitol, L-Isomalt and D-Isomalt or a combination thereof.In one embodiment preferably the sugar alcohol is selected from the group consisting from D-Mannitol, L-Mannitol, D-Sorbitol and L-Sorbitol.

The saponin glycoside comprises Glycyrrhizin (GA) or Glycyrrhizin (GA) salt, or a combination thereof.The Glycyrrhizin (GA) is selected from 18β-Glycyrrhizin and 18α-Glycyrrhizin, or a combination thereof. Glycyrrhizin(GA) salt is selected from 18α-Glycyrrhizin mono ammonium salt and 18β-Glycyrrhizin mono ammonium salt or a combination thereof. In one embodiment the Glycyrrhizin (GA) comprises equal parts of 18β-Glycyrrhizin and 18-α-Glycyrrhizin. In another aspect the composition further (optionally) comprises pH adjusting agent selected from the group consisting of potassium sorbate (KS), monobasic sodium phosphate, dibasic sodium phosphate and tribasic sodium phosphate in a mass concentration range of 0.01 to 0.2%.

In one embodiment the composition further comprises flavouring agent selected from the group consisting of extracts of herbs, spices, fruit, and artificial flavour, in mass concentration range of 0.01 to 0.2%.In another aspect, the composition further comprises quantum sufficient of water or alcohol or mixture of water and alcohol.

In one embodiment the above composition is selected from combinations of ingredients as presented in TABLE-1 and TABLE-2.In one embodiment the above composition is selected from below combinations (Sr. No. 1 to 98) of ingredients:

| Sr. No. | TA-01 | L-Ala-L-Gla | Man | 18β-GA | KS |
|---|---|---|---|---|---|
| 1 | 0.0075 | 0.5 | 0.5 | 0.1 | |

(continued)

| Sr. No. | TA-01 | L-Ala-L-Gla | Man | 18β-GA | KS |
|---|---|---|---|---|---|
| 2 | 0.00125 | 1 | 0.5 | 0.1 | |
| 3 | 0.00125 | 1 | 2.5 | 0.1 | |
| 4 | 0.00125 | 2 | 0.75 | 0.1 | |
| 5 | 0.025 | 0.5 | 0.5 | 0.1 | |
| 6 | 0.00125 | 1 | 0.5 | 0.04 | |
| 7 | 0.00125 | 0.5 | 0.5 | 0.04 | |
| 8 | 0.00125 | 1 | 0 | 0.1 | |
| 9 | 0.00125 | 1 | 0.5 | 0 | |
| 10 | 0.00125 | 0 | 0.5 | 0.1 | |
| 11 | 0.000625 | 1 | 0.75 | 0.3 | |
| 12 | 0.00125 | 1 | 0.5 | 0.02 | |
| 13 | 0.00035 | 0.5 | 1.5 | 0.3 | |
| 14 | 0.00035 | 0.5 | 2.5 | 0.3 | |
| 15 | 0.00035 | 1 | 1 | 0.3 | |
| 16 | 0.00035 | 1 | 0.75 | 0.3 | |
| | TA-01 | L-Ala-L-Gla | Man | 18α-GA | KS |
| 17 | 0.00125 | 2 | 0.75 | 0.1 | |
| 18 | 0.00125 | 1 | 0.5 | 0.04 | |
| 19 | 0.00125 | 1 | 2.5 | 0.3 | |
| 20 | 0.0075 | 0.5 | 0.5 | 0.3 | |
| 21 | 0.0005 | 1 | 0.75 | 0.3 | |
| 22 | 0.00035 | 0.5 | 1.5 | 0.3 | |
| 23 | 0.00035 | 0.5 | 2.5 | 0.3 | |
| 24 | 0.00035 | 1 | 0.75 | 0.3 | |
| | TA-01 | L-Ala-L-Gla | Man | 18βGA | KS |
| 25 | 0.00125 | 0 | 0.5 | 0.1 | 0.1 |
| 26 | 0.00125 | 1 | 0.5 | 0.1 | 0.1 |
| | TA-01 | GSSG | Man | 18βGA | KS |
| 27 | 0.00125 | 1 | 0.5 | 0.04 | |
| 28 | 0.0005 | 0.5 | 2.5 | 0.3 | |
| 29 | 0.00125 | 2 | 0.75 | 0.1 | |
| 30 | 0.0005 | 1 | 0.75 | 0.3 | |
| 31 | 0.0075 | 0.5 | 0.5 | 0.3 | |
| 32 | 0.0005 | 0.5 | 1.5 | 0.1 | |
| 33 | 0.0005 | 1.5 | 1 | 0.1 | |
| 34 | 0.0075 | 1 | 0.75 | 0.3 | |
| 35 | 0.00125 | 0.2 | 0.5 | 0.3 | |
| 36 | 0.00035 | 0.2 | 0.75 | 0.3 | |

(continued)

| | | TA-01 | L-Ala-L-Gla | Man | 18βGA | KS |
|---|---|---|---|---|---|---|
| | 37 | 0.00035 | 0.2 | 0.5 | 0.1 | |
| | 38 | 0.000625 | 0.1 | 0.5 | 0.1 | |
| | 39 | 0.0005 | 0.1 | 0.5 | 0.1 | |
| | | TA-01 | L-Ala-L-Gln | Sor | 18βGA | KS |
| | 40 | 0.0075 | 0.5 | 0.5 | 0.3 | |
| | 41 | 0.00125 | 1 | 0.5 | 0.04 | |
| | 42 | 0.0005 | 1 | 0.75 | 0.3 | |
| | 43 | 0.00125 | 2 | 0.75 | 0.1 | |
| | 44 | 0.00035 | 0.5 | 1.5 | 0.3 | |
| | | TA-2 | L-Ala-L-Gln | Man | 18βGA | KS |
| | 45 | 0.0075 | 0.5 | 0.5 | 0.3 | |
| | 46 | 0.00125 | 2 | 0.75 | 0.1 | |
| | 47 | 0.00035 | 0.5 | 1.5 | 0.3 | |
| | 48 | 0.00125 | 1 | 0.5 | 0.04 | |
| | 49 | 0.00035 | 1 | 0.75 | 0.3 | |
| | | TA-B | L-Ala-L-Gln | Man | 18βGA | KS |
| | 50 | 0.0075 | 0.5 | 0.5 | 0.3 | |
| | 51 | 0.00125 | 2 | 0.75 | 0.1 | |
| | 52 | 0.00035 | 0.5 | 1.5 | 0.3 | |
| | 53 | 0.00125 | 1 | 0.5 | 0.04 | |
| | 54 | 0.00035 | 1 | 0.75 | 0.3 | |
| | | TA-01 | CP | Man | 18βGA | KS |
| | 55 | 0.00125 | 0.2 | 0.5 | 0.3 | |
| | 56 | 0.00035 | 0.1 | 0.75 | 0.3 | |
| | 57 | 0.00035 | 0.1 | 0.5 | 0.04 | |
| | | TA-01 | L-Ala-L-Gln % | Man % | 18β-GA % | |
| | 58 | 0.0025 | 1 | 0.5 | 0.1 | |
| | | TA-01 % | L-Ala-L-Gln % | Man % | 18β-GA % | |
| | 59 | 0.00125 | 1 | 0.5 | 0.04 | |
| | 60 | 0.000625 | 0.5 | 0.5 | 0.04 | |
| | | TA-01% | L-Ala-L-Gln % | Man | 18β-GA % | |
| | 61 | 0.000625 | 1 | 0.5 | 0.02 | |
| | 62 | 0.000625 | 1 | 0.5 | 0.01 | |
| | 63 | 0.005 | 0.5 | 0.5 | 0.04 | |
| | 64 | 0.000625 | 0.5 | 2.5 | 0.04 | |
| | 65 | 0.000625 | 2 | 0.5 | 0.04 | |
| | 66 | 0.000625 | 0.5 | 0.5 | 0.3 | |
| | 67 | 0.0005 | 1 | 0.5 | 0.04 | |

(continued)

|  | | TA-01% | L-Ala-L-Gln % | Man | 18β-GA % | |
|---|---|---|---|---|---|---|
|  | 68 | 0.00035 | 0.5 | 0.5 | 0.04 | |
|  | 69 | 0.0025 | 0.5 | 0.5 | 0.04 | |
|  | | TA-01 | L-Ala-L-Gln | Man | 18αGA | |
|  | 70 | 0.000625 | 0.5 | 0.5 | 0.04 | |
|  | 71 | 0.000625 | 0.5 | 0.5 | 0.3 | |
|  | 72 | 0.00035 | 0.5 | 0.5 | 0.04 | |
|  | 73 | 0.0025 | 0.5 | 0.5 | 0.04 | |
|  | 74 | 0.000625 | 1 | 1 | 0.04 | |
|  | 75 | 0.000625 | 1.5 | 1.5 | 0.04 | |
|  | | TA-01 | GSSG | Man | 18βGA | |
|  | 76 | 0.000625 | 0.5 | 0.5 | 0.04 | |
|  | 77 | 0.00035 | 0.5 | 0.5 | 0.04 | |
|  | 78 | 0.000625 | 0.5 | 1 | 0.04 | |
|  | 79 | 0.000625 | 1 | 0.5 | 0.04 | |
|  | 80 | 0.00035 | 0.2 | 0.5 | 0.1 | |
|  | 81 | 0.0025 | 0.5 | 0.5 | 0.04 | |
|  | | TA-01 | L-Ala-L-Gln | Sor | 18βGA | |
|  | 82 | 0.000625 | 0.5 | 0.5 | 0.04 | |
|  | 83 | 0.0025 | 0.5 | 0.5 | 0.04 | |
|  | 84 | 0.000625 | 1 | **1** | 0.04 | |
|  | 85 | 0.000625 | 0.5 | 0.5 | 0.3 | |
|  | 86 | 0.000625 | 1.5 | **1.5** | 0.04 | |
|  | 87 | 0.00035 | 0.5 | 0.5 | 0.04 | |
|  | | TA-02 | L-Ala-L-Gln | Man | 18βGA | |
|  | 88 | 0.000625 | 0.5 | 0.5 | 0.04 | |
|  | 89 | 0.0025 | 0.5 | 0.5 | 0.04 | |
|  | 90 | 0.000625 | 0.5 | 0.5 | 0.3 | |
|  | 91 | 0.000625 | 1.5 | 1.5 | 0.04 | |
|  | 92 | 0.00035 | 0.5 | 0.5 | 0.04 | |
|  | 93 | 0.000625 | 1 | 0.75 | 0.1 | |
|  | | TA-B | L-Ala-L-Gln | Man | 18βGA | |
|  | 94 | 0.0025 | 0.5 | 0.5 | 0.04 | |
|  | 95 | 0.000625 | 0.5 | 0.5 | 0.3 | |
|  | 96 | 0.000625 | 1.5 | 1.5 | 0.04 | |
|  | 97 | 0.00035 | 0.5 | 0.5 | 0.04 | |
|  | 98 | 0.000625 | 1 | 0.75 | 0.1 | |

In one aspect the invention provides a synergistic composition for reducing DNA damage, reducing/relieving hangover, enhancing DNA repair and modulating immunology parameters comprising:

(a) hydrolyzable tannin or pyrogallol-type tannin compounds in mass a concentration range of 0.00035 to 0.025%; and at least two active ingredients selected from :
(b) amino acid derived molecule in mass concentration range of 0.1 to 2.0%;
(c) sugar alcohol in mass concentration range of 0.5 to 2.5 %; and
(d) saponin glycoside in mass concentration range of 0.01 to 0.3 %.

In one embodiment the above composition comprises:

(a) Tanal 01 or Tanal 02 or Tanal B in mass a concentration range of 0.00035 to 0.025%; and at least two active ingredients selected from the group comprising:
(b) amino acid derived molecule selected from L-alanyl-L-glutamine (L-Ala-L-Gln),oxidised L-glutathione (GSSG) and hydrolysed casein protein (CP) in mass concentration range of 0.1 to 2.0%;
(c) sugar alcohol selected from D-Mannitol, L-Mannitol, D-Sorbitol and L-Sorbitol in mass concentration range of 0.5 to 2.5 %; and
(d) saponin glycoside selected from 18-$\alpha$-Glycyrrhizin and 18-$\beta$-Glycyrrhizin or mixture of 18-$\alpha$-Glycyrrhizin and 18-$\beta$-Glycyrrhizin in mass concentration range of 0.01 to 0.3 %.

In one aspect the invention provides a synergistic composition for reducing DNA damage and enhancing DNA repair wherein composition is selected from:

(i)a composition comprising Tanal 01in mass concentration range of 0.00035% to 0.025%, L-Ala-L-Gln in mass concentration range of 0.5% to 2.0%, D-Mannitol or L-Mannitol in mass concentration range of 0.5% to 2.5%, and 18$\beta$-Glycyrrhizin in mass concentration range of 0.02% to 0.3%;

(ii)a composition comprising Tanal 02 in mass concentration range of 0.00035% to 0.0075%, L-Ala-L-Glnin mass concentration range of 0.5% to 2.0%, D-Mannitol or L-Mannitol in mass concentration range of 0.5% to 0.75%, and 18$\beta$-Glycyrrhizin in mass concentration range of 0.04% to 0.3%;

(iii)a composition comprising Tanal Bin mass concentration range of 0.00035% to 0.0075%, L-Ala-L-Gln in mass concentration range of 0.5% to 2.0%, D-Mannitol or L-Mannitol in mass concentration range of 0.5% to 0.75%, and 18$\beta$-Glycyrrhizin in mass concentration range of 0.04% to 0.3%;

(iv)a composition comprising Tanal 01in mass concentration range of 0.00035% to 0.0075%, L-Ala-L-Gln in mass concentration range of 0.5% to 2.0%, D-Mannitolor L-Mannitol in mass concentration range of 0.5% to 2.5%, and 18$\alpha$-Glycyrrhizin in mass concentration range of 0.04% to 0.3%;

(v)a composition comprising Tanal 01 in mass concentration range of 0.00035% to 0.000625%, Oxidised L-Glutathione (GSSG) in mass concentration range of 0.1% to 0.2%, D-Mannitol or L-Mannitol in mass concentration range of 0.5% to 0.75%, and 18$\beta$-Glycyrrhizin in mass concentration range of 0.1% to 0.3%;

(vi)a composition comprising Tanal 01 in mass concentration range of 0.00035% to 0.00125%, hydrolysed casein protein (CP) in mass concentration range of 0.1% to 0.2%, D-Mannitol or L-Mannitol in mass concentration range of 0.5% to 0.75%, and 18$\beta$-Glycyrrhizin in mass concentration range of 0.04% to 0.3%; and

(vii)a composition comprising Tanal 01 in mass concentration range of 0.00035% to 0.0075%, L-Ala-L-Gln in mass concentration range of 0.5% to 2.0%, D-Sorbitol L-Sorbitol in mass concentration range of 0.5% to 1.5%, and 18$\beta$-Glycyrrhizin in mass concentration range of 0.04% to 0.3 %.

In one aspect the invention provides a synergistic composition for reducing Hepatic damage, enhancing Hepatic repair and alleviating CNS parameters comprising:

(a) hydrolyzable tannin or pyrogallol-type tannin compounds in mass a concentration range of 0.00035 to 0.005%; and at least two active ingredients selected from group comprising:
(b) amino acid derived molecule in mass concentration range of 0.5 to 2.0%;
(c) sugar alcohol in mass concentration range of 0.5 to 2.5 %; and
(d) saponin glycoside in mass concentration range of 0.01 to 0.3 %.

In one embodiment the composition comprises:

(a) Tanal 01 or Tanal 02 or Tanal B in mass a concentration range of 0.00035 to 0.005%; and at least two other active ingredients selected from group comprising:

(b) amino acid derived molecule selected from L-alanyl-L-glutamine (L-Ala-L-Gln) or oxidised L-glutathione (GSSG) in mass concentration range of 0.5 to 2.0%;

(c) sugar alcohol selected from D-Mannitol or L-Mannitol or D-Sorbitol or L-Sorbitol in mass concentration range of 0.5 to 2.5 %; and

(d) saponin glycoside selected from 18-$\alpha$-Glycyrrhizin or 18-$\beta$-Glycyrrhizin or mixture of 18-$\alpha$-Glycyrrhizin and 18-$\beta$-Glycyrrhizin in mass concentration range of 0.01 to 0.3 %.

In one aspect the invention provides a synergistic composition for reducing hangover and alleviating hangover parameters wherein composition is selected from:

(i) a composition comprising Tanal 01 in mass concentration range of 0.00035% to 0.025%, L-Ala-L-Gln in mass concentration range of 0.5% to 2.0%, D-Mannitol or L-Mannitol in mass concentration range of 0.5% to 2.5%, and 18$\beta$-Glycyrrhizin in mass concentration range of 0.02% to 0.3%;

(ii) a composition comprising Tanal 02 in mass concentration range of 0.00035% to 0.00125%, L-Ala-L-Gln in mass concentration range of0.5% to 2.0%, D-Mannitol or L-Mannitol in mass concentration range of0.5% to 1.5%, and 18$\beta$-Glycyrrhizin in mass concentration range of0.04% to 0.3%;

(iii) a composition comprising Tanal B in mass concentration range of 0.00035% to 0.0075%, L-Ala-L-Gln in mass concentration range of 0.5% to 2.0%, D-Mannitol or L-Mannitol in mass concentration range of 0.5% to 1.5%, and 18$\beta$-Glycyrrhizin in mass concentration range of 0.04% to 0.3%;

(iv) a composition comprising Tanal 01 in mass concentration range of 0.0005% to 0.0075%, L-Ala-L-Gln in mass concentration range of0.5% to 1.0%, D-Mannitol or L-Mannitol in mass concentration range of 0.5% to 2.5%, and 18$\alpha$-Glycyrrhizin in mass concentration range of 0.04% to 0.3%;

(v) a composition comprising Tanal 01 in mass concentration range of 0.00035% to 0.0075%, Oxidised L-Glutathione (GSSG) in mass concentration range of0.1% to 1.5%, D-Mannitol or L-Mannitol in mass concentration range of 0.5% to 2.5%, and 18$\beta$-Glycyrrhizin in mass concentration range of 0.04% to 0.3%;

(vi) a composition comprising Tanal 01 in mass concentration range of 0.00035% to 0.00125%, hydrolysed casein protein (CP) in mass concentration range of 0.1% to 0.2%, D-Mannitol or L-Mannitol in mass concentration range of 0.5% to 0.75%, and 18$\beta$-Glycyrrhizin in mass concentration range of 0.04% to 0.3%; and

(vii) a composition comprising Tanal 01 in mass concentration range of 0.00125% to 0.0075%, L-Ala-L-Gln in mass concentration range of 0.5% to 2.0%, D-Sorbitol or L-Sorbitol in mass concentration range of 0.5% to 1.5%, and 18$\beta$-Glycyrrhizin in mass concentration range of 0.04% to 0.3%.

In one aspect the invention provides a synergistic composition for alleviating immunology parameters, wherein composition is selected from :

(i) a composition comprising Tanal 01 in mass concentration range of 0.00035% to 0.025%, L-Ala-L-Gln in mass concentration range of 0.5% to 2.0%, D-Mannitol or L-Mannitol in mass concentration range of 0.5% to 2.5%, and 18$\beta$-Glycyrrhizin in mass concentration range of 0.01% to 0.3%;

(ii) a composition comprising Tanal 02 in mass concentration range of 0.00035% to 0.0075%, L-Ala-L-Gln in mass concentration range of 0.5% to 2.0%, D-Mannitol or L-Mannitol in mass concentration range of 0.5% to 1.5%, and 18$\beta$-Glycyrrhizin in mass concentration range of 0.04% to 0.3%;

(iii) a composition comprising Tanal B in mass concentration range of 0.00035% to 0.0075%, L-Ala-L-Gln in mass concentration range of 0.5% to 2.0%, D-Mannitol or L-Mannitol in mass concentration range of 0.5% to 1.5%, and 18$\beta$-Glycyrrhizin in mass concentration range of 0.04% to 0.3%;

(iv) a composition comprising Tanal 01 in mass concentration range of 0.00035% to 0.0075%, L-Ala-L-Gln in mass concentration range of 0.5% to 2.0%, D-Mannitol or L-Mannitol in mass concentration range of 0.5% to 2.5%, and 18α-Glycyrrhizin in mass concentration range of 0.04% to 0.3%;

(v) a composition comprising Tanal 01 in mass concentration range of 0.00035% to 0.0075%, Oxidised L-Glutathione (GSSG) in mass concentration range of 0.1% to 2.0%, D-Mannitol or L-Mannitol in mass concentration range of 0.5% to 2.5%, and 18β-Glycyrrhizin in mass concentration range of 0.04% to 0.3%;

(vi) a composition comprising Tanal 01 in mass concentration range of 0.00035% to 0.00125%, hydrolysed casein protein (CP) in mass concentration range of 0.1% to 0.2%, D-Mannitol or L-Mannitol in mass concentration range of 0.5% to 0.75%, and 18β-Glycyrrhizin in mass concentration range of 0.04% to 0.3%; and

(vii) a composition comprising Tanal 01 in mass concentration range of 0.00035% to 0.0075%, L-Ala-L-Gln in mass concentration range of 0.5% to 2.0%, D-Sorbitol or L-Sorbitol in mass concentration range of 0.5% to 1.5%, and 18β-Glycyrrhizin in mass concentration range of 0.04% to 0.3%.

In one aspect the invention provides a synergistic composition for reducing hepatic damage and enhancing hepatic repair wherein composition is selected from:

(i) a composition comprising Tanal 01 in mass concentration range of 0.00035% to 0.0025%, L-Ala-L-Gln in mass concentration range of 0.5% to 2.0%, D-Mannitol or L-Mannitol in mass concentration range of 0.5% to 2.5%, and 18β-Glycyrrhizin in mass concentration range of 0.01% to 0.3%;

(ii) a composition comprising Tanal 01 in mass concentration range of 0.00035% to 0.0025%, L-Ala-L-Gln in mass concentration range of 0.5% to 1.5%, D-Mannitol or L-Mannitol in mass concentration range of 0.5% to 1.5%, and 18α-Glycyrrhizin in mass concentration range of 0.04% to 0.3%;

(iii) a composition comprising Tanal 01 in mass concentration range of 0.00035% to 0.000625%, Oxidised L-Glutathione (GSSG) in mass concentration range of 0.5% to 1.0%, D-Mannitol or L-Mannitol in mass concentration range of 0.5% to 1.0%, and 18β-Glycyrrhizin in mass concentration range of 0.04% to 0.1%;

(iv) a composition comprising Tanal 01 in mass concentration range of 0.00035% to 0.0025%, L-Ala-L-Gln in mass concentration range of 0.5% to 1.5%, D-Sorbitol or L- Sorbitol in mass concentration range of 0.5% to 1.5%, and 18β-Glycyrrhizin in mass concentration range of 0.04% to 0.3%;

(v) a composition comprising Tanal 02 in mass concentration range of 0.00035% to 0.0025%, L-Ala-L-Gln in mass concentration range of 0.5% to 1.0%, D-Mannitol or L-Mannitol in mass concentration range of 0.5% to 1.5%, and 18β-Glycyrrhizin in mass concentration range of 0.04% to 0.3%; and

(vi) a composition comprising Tanal B in mass concentration range of 0.00035% to 0.0025%, L-Ala-L-Gln in mass concentration range of 0.5% to 1.5%, D-Mannitol or L-Mannitol in mass concentration range of 0.5% to 1.5%, and 18β-Glycyrrhizin in mass concentration range of 0.04% to 0.3%.

In one aspect the invention provides a synergistic composition for reducing CNS stress and modulating and/or alleviating CNS parameters, wherein composition is selected from:

(i) a composition comprising Tanal 01 in mass concentration range of 0.00035% to 0.005%, L-Ala-L-Gln in mass concentration range of 0.5% to 2.0%, D-Mannitol or L-Mannitol in mass concentration range of 0.5% to 2.5%, and 18β-Glycyrrhizin in mass concentration range of 0.01% to 0.3%;

(ii) a composition comprising Tanal 01 in mass concentration range of 0.00035% to 0.0025%, L-Ala-L-Gln in mass concentration range of 0.5% to 1.5%, D-Mannitol or L-Mannitol in mass concentration range of 0.5% to 1.5%, and 18α-Glycyrrhizin in mass concentration range of 0.04% to 0.3%;

(iii) a composition comprising Tanal 01 in mass concentration range of 0.00035% to 0.000625%, Oxidised L-Glutathione (GSSG) in mass concentration range of 0.5% to 1.0%, D-Mannitol or L-Mannitol in mass concentration range of 0.5% to 1.0%, and 18β-Glycyrrhizin in mass concentration range of 0.04% to 0.1%;

(iv) a composition comprising Tanal 01 in mass concentration range of 0.00035% to 0.0025%, L-Ala-L-Gln in mass concentration range of 0.5% to 1.5%, D-Sorbitol or L- Sorbitol in mass concentration range of 0.5% to 1.5%, and 18β-Glycyrrhizin in mass concentration range of 0.04% to 0.3%;

(v) a composition comprising Tanal 02 in mass concentration range of 0.00035% to 0.0025%, L-Ala-L-Gln in mass concentration range of 0.5% to 1.0%, D-Mannitol or L-Mannitol in mass concentration range of 0.5% to 0.75%, and 18β-Glycyrrhizin in mass concentration range of 0.04% to 0.3%;

(vi) a composition comprising Tanal B in mass concentration range of 0.00035% to 0.0025%, L-Ala-L-Gln in mass concentration range of 0.5% to 1.0%, D-Mannitol or L-Mannitol in mass concentration range of 0.5% to 0.75%, and 18β-Glycyrrhizin in mass concentration range of 0.04% to 0.3%.

In one aspect the invention provides a process for the preparation of above synergistic beverage composition comprising the steps of:

(a) obtaining water or alcohol or alcohol-water mixture;
(b) mixing two or more ingredients selected from group comprising sugar alcohol, hydrolysable tannin and amino acid derived molecules in a reactor portion wise in small quantities containing water or alcohol or alcohol-water mixture of step (a);
(c) after mixing above ingredients saponin glycoside is added portion wise;
(d) adjusting the pH of the resulting solution of step (c) between 4.0 - 10; and
(e) obtaining the required beverage composition, filtering and bottling.

In any of the above the compositions, the composition is formulated as a beverage additive which is added to a beverage at the time of drinking or formulated as a ready to drink beverage wherein the beverage additive is mixed with the beverage at the time of preparation of the beverage. Without limiting, the synergistic composition of the present invention is formulated as beverage formulation. In one embodiment the beverage is an alcoholic beverage. Any other beverage can be used.

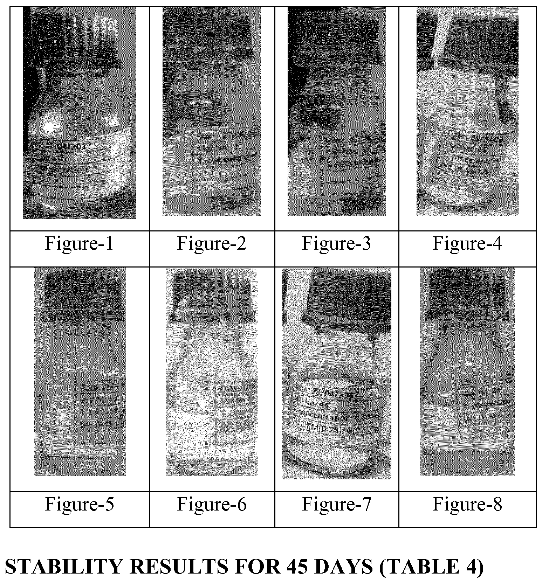

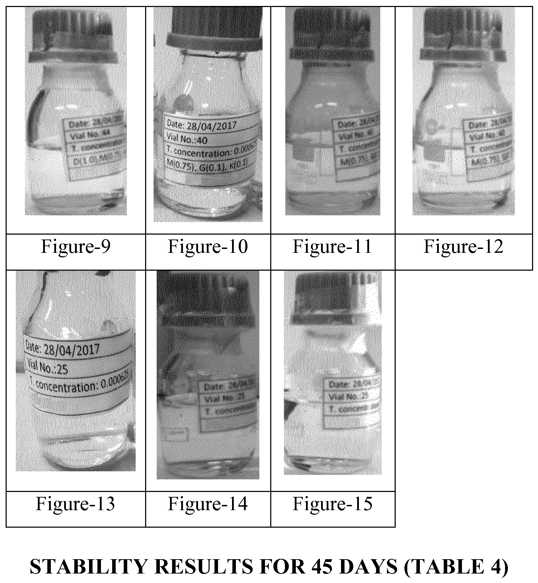

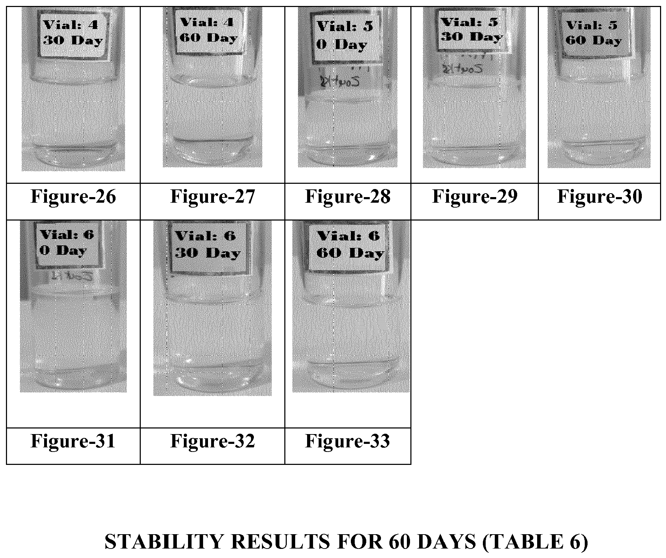

The compositions of the inventions when formulated are stable. Stability data are presented in Tables 4-6, and Figures 1-33. Further, the present invention is described in below detailed description, examples and experimental data presented in Table-1, Table-2, Table-3, Table-4, Table 5 and Table 6.

## BRIEF DESCRIPTION OF DRAWINGS AND FIGURES

**Figure 1 to Figure 15: 45 days stability report for different formulations prepared as per composition of the present invention**

**Figure 16 to Figure 33: 60 days stability report for different formulations prepared as per composition of the present invention**

## DETAILED DESCRIPTION OF THE INVENTION

The invention relates to a composition to reduce DNA and hepatic (liver) damage and to enhance repair thereof. More particularly the invention relates to a synergistic composition comprising combination of active ingredients which can be used an additive in a beverage composition and also relates to a beverage composition comprising said synergistic composition of active ingredients, wherein each active ingredient in the combination composition and/or beverage composition in appropriate concentration synergistically reduces the DNA damage as well as hepatic damage due to alcohol consumption and/or due to other reasons. The composition also enhances repair of the DNA and liver which have already been damaged. The composition also synergistically reduces hangover, modulates and/or alleviates immunology parameters and CNS parameters due to alcohol consumption and/or due to other reasons. Further a beverage composition comprising above synergistic combination composition and method of preparation thereof is provided.

The composition and the method of preparation of the composition of the present invention is described below, which do not restrict the scope and ambit of the present invention.

The above compositions of the present invention:

- comprise active-ingredients;
- may or may not comprise non-active ingredients; and

- may or may not comprise water or alcohol, or both water and alcohol as required in formulating and preparing the composition.

The composition may broadly comprise all or some of the active ingredients selected from (a) hydrolyzable tannin or pyrogallol-type tannin compounds, (b) amino acid derived molecule, (c) sugar or sugar alcohol and (d) saponin glycosideIn one aspect the synergistic composition of the invention comprises **two active ingredients.** In one aspect the synergistic composition of the invention comprises **three active ingredients**. In another aspect the synergistic composition of the invention comprises all the **four active ingredients**. Preferably the composition comprises three or four active ingredients, one active ingredient being tannin compound among the active ingredients of the composition.

In one aspect any of the above composition comprises tannin with one or more of other active ingredients. In one embodiment, any of the above two or three or four active ingredient(s) combination composition comprises hydrolyzable tannin or pyrogallol-type tannin compounds as one active ingredient and the other one or more active ingredients is/are selected from amino acid derived molecule, saponin glycoside, sugar or sugar alcohol.

In one aspect the composition comprises combination of two active ingredients such as (a) hydrolyzable tannin or pyrogallol-type tannin compounds and (b) amino acid derived molecule.

In one aspect the composition comprises combination of two active ingredients such as (a) hydrolyzable tannin or pyrogallol-type tannin compounds and (c) sugar or sugar alcohol.

In one aspect the composition comprises combination of two active ingredients such as (a) hydrolyzable tannin or pyrogallol-type tannin compounds and (d) saponin glycoside. In one aspect the composition comprises combination of three active ingredients such as (a) hydrolyzable tannin or pyrogallol-type tannin compounds, (b) amino acid derived molecule and (c) sugar or sugar alcohol.

In one aspect the composition comprises combination of three active ingredients such as (a) hydrolyzable tannin or pyrogallol-type tannin compounds, (b) amino acid derived molecule and (d) saponin glycoside.

In one aspect the composition comprises combination of three active ingredients such as (a) hydrolyzable tannin or pyrogallol-type tannin compounds, (c) sugar or sugar alcohol and (d) saponin glycoside.

In one aspect the composition comprises combination of four active ingredients such as (a) hydrolyzable tannin or pyrogallol-type tannin compounds, (b) amino acid derived molecule, (c) sugar or sugar alcohol and (d) saponin glycoside.

Optionally, any of the above composition may comprise one or more of excipent or additional non-active ingredients as may require for formulating and for better physical form, appearance and taste of the composition. In one embodiment, any of the above composition comprising at least two or at least three or all four active ingredients optionally further comprises one or more of pH adjusting agents,one or more of flavouring agent, water or alcohol or mixture of water and alcohol.

Broadly the above described additive compositions and/or beverage composition comprising said additive composition, comprises following all or some of the active ingredients, optionally with or without one or more of non-active ingredients in amount ranging as below:

(a) hydrolyzable tannin or pyrogallol-type tannin compounds may be used in mass concentration range of 0.0001 to 2.0%;
(b) the amino acid derived molecule may be used in mass concentration range of 0.01 to 5.0%;
(c) sugar or sugar alcohol may be used in mass concentration range of 0.01 to 5.0 %; and
(d) the saponin glycoside which comprises Glycyrrhizin or its isomers or its derivatives may be used in a mass concentration range of 0.01 to 2.0 %;

- Optionally, quantum sufficient of water, and/or quantum sufficient of alcohol or quantum sufficient mixture of water and alcohol;
- Optionally, one or more of pH adjusting agents may be used in mass concentration range of about 0.01 to 0.2%;
- Optionally, one or more of flavouring agent may be used in mass concentration range of about 0.01 to 0.2%;
- Optionally, any other non-active ingredients at appropriate amount as may be required based on the composition.

All the above concentrations are in mass concentration or weight by volume (wt./v%).

**Description of the ingredients used in the composition**

**(a) Hydrolysable tannin**

**Tannins:** Tannins are polyphenolic secondary metabolites of higher plants, and are either galloyl esters and their derivatives, in which galloyl moieties or their derivatives are bound to a variety of Polyol carbohydrate such as D-glucose, catechin and triterpenoid cores (gallotannins, ellagitannins and complex tannins), or they are oligomeric and polymeric proanthocyanidins that can possess different interflavanyl coupling and substitution patterns (condensed tannins).

Hydrolysable tannins like Tannic acid are obtained from various sources like Rhus semialata and Quercus infectoria (Building block Gallic acid, glucose), Caesalpina spinosa (Building block: Gallic acid, quinic acid). Hydrolysable gallotannins are obtained from Tara pods, Aleppo or Chinese gallnuts, or Sumac leaves.

In general, Hydrolyzable tannin or pyrogallol-type tannin compounds comprise a Tannin such as Tannic acid, epigallitannins, gallotannins, Ellagitannins, condensed tannin, a complex tannin, hydrolyzable tannin or a combination thereof. In particular, tannic acid from different source such as Tanal 01, Tanal 02, Brewtan (Tanal B) with different chemical composition.

**Tannic acid:** Tannic acid refers to the small group of polyphenols calledhydrolysable (gallo)tannins obtained from Aleppo or Chinese gallnuts, or Sumac leaves. The main building block glucose is combined with gallic acid and hydrolysable (gallo)tannins obtained from Tara pods building block quinic acidis combined with gallic acid. The botanical source & method of process results in various number of gallic acid combined to glucose molecule. Thus, this results in mixture of various1,2,3,4,6-tetragalloyl glucose 1,2,3,4,6-Pentagalloyl glucose,1,2,3,4,6-Hexagalloyl glucose, 1,2,3,4,6-Heptagalloyl glucose, 1,2,3,4,6-Octagalloyl glucose, 1,2,3,4,6-nonagalloyl glucose 1,2,3,4,6-decagalloyl glucose,1,2,3,4,6-undecagalloyl glucose,1,2,3,4,6-dodecagalloyl glucose.

Commercially available sources of tannic acid provide a nominal molecular weight for (1294 g/mol), the manufacturing process are heterogenous and variable mixtures of galloyl esters. Tannic acid is not an appropriate standard for any tannin analysis because of its poorly defined composition. Similarly, the literature information over tannic acid could be considered only when the composition is clearly defined.

**Epigallitannins:** When a hydrolyzable tannin molecule has carbohydrate (usually D-glucose but also cyclitols like quinic or shikimic acids). The hydroxyl groups of the carbohydrate are partially or totally esterified with phenolic groups with ellagic acid in ellagitannins.

**Gallotannins** are all those hydrolysable tannins (polymers) in which galloyl units or their meta-depsidic derivatives are bound to diverse Polyol carbohydrate such as glucose, catechin, or triterpenoid units.

**Ellagitannins** are those hydrolysable tannins in which at least two galloyl units are oxidative C-C coupled (*meta* or *para*depside bonds) to each other, and do not contain a glycosidically linked catechin unit.

**Complex tannins** are tannins in which a catechin unit is bound glycosidically to a gallotannin or an ellagitannin unit.

**Condensed tannins** are oligomeric and polymeric proanthocyanidins formed by linkage of C-4 of one catechin with C-8 or C-6 of the next monomeric catechin.

**Hydrolyzable tannin:** Tannins can be fractionated hydrolytically into their components, for example by the treatment with hot water or with tannases.

**Tanal 01:** Tanal 01 refers to Tannic acid, which are small group of polyphenols called hydrolysable (gallo) tannins obtained from Rhussemialata. The main building block glucose is combined with gallic acid. It is a mixture of various 1,2,3,4,6-tetragalloyl glucose 1,2,3,4,6-Pentagalloyl glucose, 1,2,3,4,6-Hexagalloyl glucose, 1,2,3,4,6-Heptagalloyl glucose, 1,2,3,4,6-Octagalloyl glucose, 1,2,3,4,6-nonagalloyl glucose 1,2,3,4,6-decagalloyl glucose, 1,2,3,4,6-undecagalloyl glucose, 1,2,3,4,6-dodecagalloyl glucose.

**Tanal 02:** Tanal 02 refers to Tannic acid, which are small group of polyphenols called hydrolysable (gallo) tannins obtained from Quercusinfectoria. The main building block glucose is combined with gallic acid. It is a mixture of various 1,2,3,4,6-tetragalloyl glucose 1,2,3,4,6-Pentagalloyl glucose, 1,2,3,4,6-Hexagalloyl glucose, 1,2,3,4,6-Heptagalloyl glucose, 1,2,3,4,6-Octagalloyl glucose, 1,2,3,4,6-nonagalloyl glucose 1,2,3,4,6-decagalloyl glucose, 1,2,3,4,6-undecagalloyl glucose, 1,2,3,4,6-dodecagalloyl glucose.

**Brewtan:** Brewtan (also known as **Tanal B**) refers to Tannic acid, which are small group of polyphenols called hydrolysable (gallo) tannins obtained from Aleppo or Chinese gallnuts, or Sumac leaves. The main building block glucose is combined with gallic acid. It is a mixture of various 1,2,3,4,6-tetragalloyl glucose 1,2,3,4,6-Pentagalloyl glucose, 1,2,3,4,6-Hexagalloyl glucose, 1,2,3,4,6-Heptagalloyl glucose, 1,2,3,4,6-Octagalloyl glucose, 1,2,3,4,6-nonagalloyl glucose 1,2,3,4,6-decagalloyl glucose, 1,2,3,4,6-undecagalloyl glucose, 1,2,3,4,6-dodecagalloyl glucose.

In one embodiment the hydrolyzable tannin or pyrogallol-type tannin compounds comprise a Tannin selected from group comprising Tannic acid, epigallitannins, gallotannins, Ellagitannins, condensed tannin, a complex tannin, hydrolyzable tannin or a combination thereof, wherein said groups are as defined above and as defined herein under definition.

Preferably, the hydrolyzable tannin or pyrogallol-type tannin compounds are selected from Tanal 01, Tanal 02 and Brewtan.

In one embodiment, the hydrolyzable tannin or pyrogallol-type tannin compound selected is Tanal 01. In one embodiment, the hydrolyzable tannin or pyrogallol-type tannin compound selected is Tanal 02. In one embodiment, the hydrolyzable tannin or pyrogallol-type tannin compound selected is Tanal B (Brewtan).

Broadly, the composition of the present invention which synergistically reduces DNA and liver damage and enhance repair thereof; reduces hangover, modulates and/or alleviates immunology parameters and CNS parameters; all due to alcohol consumption and/or due to other reasons comprises above defined hydrolyzable tannin or pyrogallol-type tannin compound in a mass concentration range of **0.0001% to 2.0 %.**

In one embodiment the hydrolyzable tannin or pyrogallol-type tannin compound, preferably selected from Tanal 01, Tanal 02 and Brewtan, may be present in a mass concentration range between 0.0001% to 1.0% or 0.0001% to 0.5% or 0.0001% to 0.25% or 0.0001% to 0.1% or 0.00035% to 0.025% or 0.00035% to 0.075% or 0.00035% to 0.0075% or 0.00035% to 0.00125% or 0.00035% to 0.000625% or 0.00125% to 0.0075%.

More specifically the amount of hydrolyzable tannin or pyrogallol-type tannin compound, preferably selected from Tanal 01, Tanal 02 and Brewtan may be present in a mass concentration range selected from 0.0001%, 0.00015%, 0.0002, 0.00025%, 0.0003%, 0.00035%, 0.0004%, 0.00045%, 0.0005%, 0.00055%, 0.0006%, 0.000625%, 0.00065%, 0.0007%, 0.000725%, 0.00075%, 0.0008%, 0.00085%, 0.0009%, 0.00095%, 0.001%, 0.00125%, 0.0015%, 0.002%, 0.0025%, 0.003%, 0.0035%, 0.004%, 0.0045%, 0.005%, 0.0055%, 0.006%, 0.0065%, 0.007%, 0.0075%, 0.008%, 0.009%, 0.01%, 0.02%, 0.025%, 0.03%, 0.04% and 0.05%.

In one embodiment, for reduction of damage of DNA and/or protection of DNA, hangover and modulates and/or alleviates Immunology parameters, the hydrolyzable tannin or pyrogallol-type tannin compound is present in a mass concentration range of 0.00035% to 0.025% or 0.00035% to 0.0075% or 0.00035% to 0.00125% or 0.00035% to 0.000625% or 0.00125% to 0.0075% or 0.0005% to 0.0075%.

In one preferred embodiment for the reduction of damage of DNA and/or protection of DNA, hangover and modulates and/or alleviates Immunology parameters the hydrolyzable tannin or pyrogallol-type tannin compound as defined above or preferably selected from Tanal 01, Tanal 02 and Brewtan is present in a mass concentration range of 0.00035%, 0.0005%, or 0.000625%, or 0.0009%, or 0.00125%, or 0.0025%, or 0.005%, or 0.0075%, or 0.025%.

In one embodiment, for reduction of hepatic and/or protection of liver, modulates and/or alleviates CNS and immunology parameters the hydrolyzable tannin or pyrogallol-type tannin compound is present in a mass concentration range of 0.00035% to 0.0025% or 0.00035% to 0.0075% or 0.00035% to 0.00125% or 0.00035% to 0.000625% or 0.00035% to 0.000625% or 0.00125% to 0.0075% or 0.00035% to 0.005%.

In one preferred embodiment for the reduction of hepatic and/or protection of liver, modulates and/or alleviates CNS and immunology parameters, the the hydrolyzable tannin or pyrogallol-type tannin compound as defined above or preferably selected from Tanal 01, Tanal 02 and Brewtan is present in a mass concentration range of 0.00035%, 0.0005%, or 0.000625%, or 0.0009%, or 0.00125%, or 0.0025%, or 0.005%, or 0.00625% or 0.0075%, or 0.025%.

All the above concentrations are in mass concentration or weight by volume (wt./v%).

**(b) Amino-Acid-Derived-Molecules**

The Amino-Acid derived Molecule of the invention may be selected from the group of an amino-acid monomer, a dipeptide, a tripeptide, a tetrapeptide, an oligopeptide, a protein and protein hydrolysate. A molecule consisting of one or more amino-acid molecules, such as an amino-acid monomer, dipeptide such as L-alanyl-L-glutamine, tripeptide such as L-Glutathione, oligopeptide such as oxidised-L-glutathione, polypeptide, protein, or a peptide hydrolysate such as casein protein hydrosylate or a peptide residue thereof.

The Amino-Acid-Derived-Molecule may comprise an amino acid monomer, such as alanine, glutamine, arginine, or ornithine. Under appropriate circumstances, as will be understood by one of ordinary skill in the art, in order to help achieve the above-mentioned benefits, it is desirable to include a different or additional amino acid monomer, such as arginine, arginine pryoglutamate, asparaginine, L-Aspartic acid, D-Asparatic acid, L-Carnitine, citruline, cysteine, cystine, gamma amino butyric acid (GABA), glutathione, glycine, histidine, L-isoleucine, L-leucine, L-lysine, methionine, phenylalanine, L-proline, pyroglutamate, serine, taurine, threonine, tyrptophan, tyrosine, L-valine, or L-Theanine, or a combination of any of these two or more, to achieve similar desirable effects.

The Amino-Acid-Derived-Molecule includes a peptide residue, such as a dipeptide residue, oligopeptide residue, or polypeptide residue. Under appropriate circumstances, as will be understood by one of ordinary skill in the art, in order to help achieve the above-mentioned benefits, it is desirable to include a different or additional amino acid monomer, such as L-Lysine HCl, L-IsoLeucine, L-Leucine, L-valine, L-Proline, L-Aspartic acid.

The Amino-Acid-Derived-Molecule also includes a dipeptide (DP), such as L-alanyl-L-glutamine. Under appropriate circumstances, as will be understood by one with ordinary skill in the art will understand, in order to help achieve the above-mentioned benefits, it is desirable to include a different dipeptide to achieve similar desirable effects, such as glycyl-glycine or L-glutamyl-L-alanine. In one embodiment the dipeptide (DP) is selected from L-alanyl-L-glutamine (L-Ala-L-Gln), glycyl-glycine (Gly-Gly) and L-glutamyl-L-alanine (Glu-Ala), or a combination of any of these two or more. Preferably the DP selected is L-alanyl-L-glutamine (L-Ala-L-Gln).

The above said oligopeptide comprises glutathione (L-Glutamyl-L-Cysteinyl-Glycine, Glu-Cys-Gly). The glutathione may be in its reduced form with a free thiol group (GSH) or in its oxidised form with a disulfide bond (GSSG).

In one preferred embodiment, the oligopeptide is oxidised glutathione (GSSG). Under appropriate circumstances, as will be understood by one with ordinary skill in the art, in order to help achieve the above-mentioned benefits, it is desirable to include a different oligopeptide such as glutathione oxidised or reduced or Peptide T. In one embodiment the oligopeptide is selected from Oxidised L-Glutathione (GSSG), Reduced L-Glutathione (GSH) and Glutathione or a combination of any of these two or more. In one preferred embodiment, the oligopeptide used is GSSG.

The Amino-Acid-Derived-Molecule also comprises protein, such as protein hydrosylate or its salts. The protein hydrosylate may be selected from casein protein hydrosylate (CP), Whey protein hydrosylate (WP). Under appropriate circumstances, as will be understood by one with ordinary skill in the art, in order to help achieve the above-mentioned benefits, it is desirable to include a different protein, such as casein hydrolysate, whey hydrolysate. In one preferred embodiment the protein used is casein protein hydrosylate (CP).

Broadly, the composition of the present invention which synergistically reduces DNA and liver damage and enhance repair thereof; reduces hangover, modulates and/or alleviates immunology parameters and CNS parameters; all due to alcohol consumption and/or due to other reasons comprises above defined Amino-Acid derived molecule in a mass concentration range of 0.01 to 5.0%.

The Amino-Acid derivative is present in the composition in a mass concentration range of 0.1 to 3.0%. The Amino-Acid derivative is present in the composition in a mass concentration range of 0.1 to 2.0%.

In one embodiment, for reduction of damage of DNA and/or protection of DNA, reduction of hepatic damage and liver protection, hangover, modulates and/or alleviates CNS and Immunology parameters, the Amino Acid derived molecule is present in a mass concentration range of 0.5% to 2.0%, 0.5% to 1.0%, 0.1% to 0.2%, 0.1% to 1.5%, 0.1% to 2.0%, 0.5% to 1.5%.

In one preferred embodiment for the reduction of damage of DNA and/or protection of DNA, hangover and Immunology parameters the Amino Acid derived molecule as defined above or preferably selected from L-Ala-L-Gln, protein hydrolysate and oxidized glutathione is present in a mass concentration range of 0.1% to 2.0% or 0.5% to 2.0%, or 0.5% to 1.0%, or 0.1 % to 0.2%, 0.1% to 0.5%.

In one preferred embodiment for the reduction of hepatic and/or protection of liver, modulates and/or alleviates CNS and immunology parameters, the Amino Acid derived molecule as defined above or preferably selected from L-Ala-L-Gln, protein hydrolysate and oxidized glutathione is present in a mass concentration range of 0.5% to 2.0%, or 0.5% to 1.5%, 0.5% to 1.5%, 0.5% to 1.0%.

All the above concentrations are in mass concentration or weight by volume (wt./v%).

**(c) Sugar or Sugar alcohol**

A sugar alcohol is a kind of alcohol prepared from sugars. These organic compounds are a class of polyols, also called polyhydric alcohol, polyalcohol, or glycitol. They are white, water-soluble solids that occur naturally and are used widely in the food industry as thickeners and sweeteners. Sugar alcohols such as Mannitol, Erythritol, Sorbitol, Xylitol etc., which are chemically stable can be used as a radical scavengers (hydroxyl radicals). Compounds like Erythritol, Mannitol, Sorbitol, Xylitol etc. up-regulated different types of superoxide dismutase (SOD) like Cu/Zn-, Mn- and EC-SOD isozymes. In particular, the SOD activity of the erythritol-added group increased by 2-5 times. Diabetics have a low SOD activity due to the Maillard reaction, because the Maillard reaction remarkably causes a decrease in the SOD activity (US Patent Application 20100037353 A1). Mannitol containing hyperosmolar solution has been shown to protect ethanol-induced gastric mucosal damage (Gharzouli K, Exp. Toxic. Pathol., 2001; 53: 175). Both rats and humans absorb and metabolize partially the Mannitol ingested in gastro intestinal tract (GIT). However, intestinal microflora convert Mannitol in to more absorbable form. In rat, absorbed mannitol is converted in to hepatic glycogen probably via fructose (J. Nutr. 1985, 115: 890). The mechanism of protecting living cells by Mannitol is not fully understood.

In one embodiment of the present invention, the sugar may be selected from D-Maltodextrin, L-Maltodextrin, D-Maltose, LMaltose, D-Dextrose, L-Dextrose, D-Glucose, L-Glucose, D-Trehalose, L-Trehalose, D-Sucrose, L-Sucrose, D-Lactose, L-Lactose, Hydrogenated Starch Hydrolysates, D-Fructose Or D-Galactose, or a mixture thereof.

In one embodiment of the present invention, the sugar alcohol may be selected from D-Glycerol, L-Glycerol, D-Mannitol, L-Mannitol,D-Erythritol, L-Erythritol, D-Xylitol, L-Xylitol, L-Maltitol, D-Maltitol, L-Sorbitol, D-Sorbitol, L-Lactitol, D-Lactitol, L-Isomalt or D-Isomalt, or a mixture thereof.

Under appropriate circumstances, as will be understood by one with ordinary skill in the art, in order to help achieve the above-mentioned benefits, it is desirable to include a different combination of the aforementioned sugars or sugar alcohols. In one preferred embodiment, the composition comprises sugar alcohol.

Under appropriate circumstances, as will be understood by one with ordinary skill in the art, in order to help achieve the above-mentioned benefits, it is desirable to include a combination of the aforementioned sugars and/or sugar alcohols.

In one embodiment of the invention, any of the above compositions may comprise only one sugar alcohol or sugar. In another embodiment of the invention, any of the above compositions may comprise mixture of two or more sugar alcohol(s) and/or sugar(s). Preferably any of the above compositions may comprise sugar alcohols such as Mannitol (D or L) or Xylitol (D or L) or Erythritol ( D or L) or Sorbitol (D or L).

In one embodiment, the sugar alcohol selected is D-Mannitol or L-Mannitol, or a mixture of D-Mannitol and L-Mannitol. In another embodiment, the sugar alcohol selected is D-Erythritol or L-Erythritol, or mixture of D-Erythritol and L-Erythritol. In another embodiment, the sugar alcohol selected is D-Sorbitol or L-Sorbitol, or mixture of D-Sorbitol and L-Sorbitol.

The Sugar or Sugar Alcohol may be used in the composition in a mass concentration range of 0.01 to 5.0 %. The Sugar or Sugar Alcohol may be used in the composition in a mass concentration range of 0.01 to 3.0 %. The Sugar or Sugar Alcohol may be used in the composition in a mass concentration range of 0.05 to 2.5%.

In one preferred embodiment, the sugar alcohol present in any of the above compositions is in the range of 0.5% to 2.5%.

In one embodiment, for reduction of damage of DNA and/or protection of DNA, reduction of hepatic damage and liver protection, hangover, modulates and/or alleviates CNS and Immunology parameters, the sugar alcohol such as D or L Mannitol, or D or L Sorbitol is present in a mass concentration range of 0.5% to 2.5%, 0.5% to 2.0%, or 0.5% to 0.75% or 0.5% to 1.5% or 0.5% to 1.0%.

All the above concentrations are in mass concentration or weight by volume (wt./v%).

**(d) Saponin Glycoside:**

The saponin glycoside which comprises Glycyrrhizin (or Glycyrrhizic acid or Glycyrrhizinic acid: abbreviated as GA) is the chief sweet-tasting constituent of *Glycyrrhiza glabra* (liquorice) root. It has also been given intravenously in Japan as a treatment for hepatitis C and as an emulsifier and gel-forming agent in foodstuff and cosmetics. Glycyrrhizin(GA) is a triterpenoid saponin glycoside. It is available as in racemic or pure form of 2 isomers: 18β-Glycyrrhizin and 18α-Glycyrrhizin. Hepato-protective mechanism of GA is due to its aglycone, glycyrrhetic acid, which inhibits both free radical generation as well as lipid peroxidation. 18α-GA has anti-hepato fibrosis effect - it is frequently used as a hepato--protective agent. The sweetness of GA has a slower onset than sugar, and lingers in the mouth for some time. GA is partly absorbed as an intact drug. (W. Xu-yinga et. al.)Chemico-Biological Interactions 181 (2009) 15-19), (T, Zing et. al., Chinese Journal of Modern Applied Pharmacy 2006, 02, 15-19).

GA induces phase II enzymes involved in the detoxification and excretion of carcinogenic or toxic substances and other antioxidant enzymes responsible for maintaining a balanced state between free radicals/oxidants and the antioxidants within the cellular environment. Oxidative injury in AR mice (allergic rhinitist mice) is reduced by GA, by increasing GSH content and decreased MDA formation in a dose dependent manner. Concomitant decreases were observed in glutathione peroxidase (GPx), catalase (CAT), total antioxidant capacity (TAOC) and SOD activities in AR mice. IFN-α, or type II interferon, is a cytokine that is critical for innate and adaptive immunity against viral and intracellular bacterial infections and for tumour control. (Xiao-Lan Li et al. Int. J. Mol. Sci. 2011, 12, 905 and Xiao-Lan Li et al. Molecules 2012, 17, 716-727).

The active ingredient saponin glycoside of the any of the above mentioned composition may comprise Glycyrrhizin or its derivatives or its salt.

In one embodiment the active ingredient saponin glycoside of the any of the above mentioned composition may comprise Glycyrrhizin (GA) or Glycyrrhizin salt (GA salt), or a combination of Glycyrrhizin (GA) and Glycyrrhizin salt (GA Salt).

In one embodiment the saponin glycoside comprises a glycyrrhizin (GA), such as 18-beta glycyrrhizin (18-βGA), 18-alpha glycyrrhizin (18-αGA), or a combination of 18-beta glycyrrhizin and 18-alpha glycyrrhizin (18β-GA+18α-GA). In one embodiment, when a combination of 18β-GA and 18α-GA is used, without limiting sense such combination comprises equal parts of 18β-GA and 18α-GA, i.e. in a ratio of 1:1. In other embodiment any other ratio of 18β-GA and 18α-GA combination can be used.

Alternatively, the saponin glycoside comprises a glycyrrhizin salt (GA salt), such as mono ammonium 18-beta glycyrrhizin, mono ammonium 18-alpha glycyrrhizin, or a combination of mono ammonium 18-beta and mono ammonium 18-alpha glycyrrhizin. In one embodiment, when a combination of 18β-GA and 18α-GA is used, without limiting sense such combination comprises equal parts of mono ammonium 18β-GA and mono ammonium 18α-GA, i.e. in a ratio of 1:1. In other embodiment any other ratio of mono ammonium 18β-GA and mono ammonium 18α-GA combination can be used.

In another aspect, the saponin glycoside comprises a combination of glycyrrhizin (GA) and a mono ammonium glycyrrhizin salt (GA salt). In one embodiment, the saponin glycoside comprises equal parts of GA and GA salt, wherein GA and GA salts are as defined above.

In one preferred embodiment the saponin glycoside used is 18-beta glycyrrhizin (18-βGA).

In one preferred embodiment the saponin glycoside used is 18-alpha glycyrrhizin (18-αGA).

Under appropriate circumstances, as will be understood by one with ordinary skill in the art, it may be desirable to include a different phytoconstituent extracted from glycyrrhizaglabra instead of glycyrrhizin, such as liquiritin or another flavonoid, to achieve similar desirable effects.

Broadly, the composition of the present invention which synergistically reduces DNA and liver damage and enhance repair thereof; reduces hangover, modulates and/or alleviates immunology parameters and CNS parameters; all due to alcohol consumption and/or due to other reasons comprises above defined saponin glycoside in a mass concentration range of **0.01 to 2.0 %.**

In one embodiment the Saponin Glycoside such as Glycyrrhizin or its derivatives or its salt, may be present in a mass concentration range between 0.01% to 1.5% or 0.01% to 1.0% or 0.01% to 0.75% or 0.01% to 0.5% or 0.01% to 0.4% or 0.01% to 0.3% or 0.01% to 0.2% or 0.01% to 0.1% or 0.02% to 0.3% or 0.03% to 0.3% or 0.03% to 0.2% or 0.03% to 0.1% or 0.04% to 0.3% or 0.04% to 0.2% or 0.04% to 0.1% or 0.05% to 0.5% or 0.1 to 0.5% or 0.1 to 0.4% or 0.1% to 0.3%.

More specifically the amount of Saponin Glycoside such as Glycyrrhizin or its derivatives or its salt may be 0.01% or 0.02% or 0.03% or 0.04% or 0.05% or 0.06% or 0.07% or 0.08% or 0.09% or 0.1% or 0.11% or 0.12% or 0.13% or 0.14% or 0.15% or 0.16% or 0.17% or 0.18% or 0.19% or 0.2% or 0.25% or 0.3% or 0.35% or 0.4% or 0.45% or 0.5% or 0.6% or 0.7% or 0.8% or 0.9% or 1.0% or 1.1% or 1.2% or 1.3% or 1.4% or 1.5% or 1.6% or 1.7% or 1.8% or 1.9% or 2.0%.

In one embodiment, for reduction of damage of DNA and/or protection of DNA, the saponin glycoside is present in a mass concentration range of 0.02% to 0.3% or 0.04% to 0.3% or 0.1% to 0.3% or near about or exactly 0.3%.

In one preferred embodiment for the reduction of damage of DNA and/or protection of DNA, the saponin glycoside is present in a mass concentration range of 0.01% or 0.02% or 0.03% or 0.04% or 0.05 or 0.06% or 0.07% or 0.08% or 0.09% or 0.1% or 0.2% or 0.3% or 0.4% or 0.5%.

Most preferably, for reduction of damage of DNA and/or protection of DNA, the saponin glycoside such as 18β-GA or 18α-GA or combination of 18β-GA and 18α-GA is present in a mass concentration range of 0.02% or 0.04% or 0.1% or 0.3%.

In one embodiment, for reduction of damage of liver and/or protection of liver, the saponin glycoside is present in a mass concentration range of 0.02 to 0.3%.

In one preferred embodiment for the for reduction of damage of liver and/or protection of liver, the saponin glycoside such as 18β-GA or 18α-GA or combination of 18β-GA and 18α-GA is present in a mass concentration range of 0.01% or 0.02% or 0.03% or 0.04% or 0.05 or 0.06% or 0.07% or 0.08% or 0.09% or 0.1% or 0.2% or 0.3% or 0.4% or 0.5%.

Most preferably, for reduction of damage of liver and/or protection of liver, the saponin glycoside is present in a mass concentration range of 0.01 %, 0.02% or 0.04% or 0.1% or 0.3%.

All the above concentrations are in mass concentration or weight by volume (wt./v%).

Further definitions of active ingredients for the groups such as hydrolysable tannin, amino acid derive molecule, sugar or sugar alcohol and saponin glycoside are as defined below under the heading- Definitions.

Optionally, any of the above said compositions of the invention further comprises other non-active ingredients such as one or more pH adjusting agent(s) and/or one or more flavouring agent(s) each present in an amount ranging from in a mass concentration range of about 0.01 to 0.2%.

In one embodiment the composition of the present invention comprises combination of ingredients in the mass concentration range or wt/v% of each of ingredients selected from below groups of combination of ranges (%):

**Liver & DNA:**

**TA-01 (Tannal 01) + Man (D-Mannitol) +DP( L-Ala-L-Gln or L-Alanine-Glutamine) + GA(18β-GA or 18β--Glycyrrhizin)**

DNA: TA01 0.00035 to 0.025, DP: 0.5 to 2.0, Man 0.5 to 2.5, GA : 0.02 to 0.3
Liver: TA01 0.00035 to 0.0025, DP: 0.5 to 2.0, Man 0.5 to 2.5, GA : 0.01 to 0.3
Hangover: TA01 0.00035 to 0.025, DP: 0.5 to 2.0, Man 0.5 to 2.5, GA : 0.02 to 0.3
CNS: TA01 0.00035 to 0.005, DP: 0.5 to 2.0, Man 0.5 to 2.5, GA : 0.01 to 0.3
Immunology: Liver: TA01 0.00035 to 0.0025, DP: 0.5 to 2.0, Man 0.5 to 2.5, GA : 0.01 to 0.3
Immunology DNA: TA01 0.00035 to 0.025, DP: 0.5 to 2.0, Man 0.5 to 2.5, GA : 0.02 to 0.3

**Liver & DNA**

**TA-01 (Tannal 01) + Man (D-Mannitol) +DP( L-Ala-L-Gln or L-Alanine-Glutamine) +)+ 18α-GA (18αGlycyrrhizin)**

DNA: 0.00035 to 0.0075 DP: 0.5 to 2.0, Man 0.5 to 2.5, **18**-GA : 0.04 to 0.3
Hangover: 0.0005 to 0.0075 DP: 0.5 to 1.0, Man 0.5 to 2.5, **18**α-GA : 0.04 to 0.3
Liver: TA01 0.00035 to 0.0025, DP: 0.5 to 1.5, Man 0.5 to 1.5,**18**α-GA : 0.04 to 0.3
CNS: TA010.00035 to 0.0025, DP: 0.5 to 1.5, Man 0.5 to 1.5, **18**α-GA : 0.04 to 0.3
Immunology Liver: TA01 0.00035 to 0.0025, DP: 0.5 to 1.5, Man 0.5 to 1.5, **18**α-GA : 0.04 to 0.3
Immunology DNA: 0.00035 to 0.0075 DP: 0.5 to 2.0, Man 0.5 to 2.5, **18**α-GA : 0.04 to 0.3

**Liver and DNA**

**TA-01 (Tannal 01) + Man (D-Mannitol) + GSSG (Oxidised L-glutathione) + GA(18β-GA or 18β--Glycyrrhizin)**

DNA: 0.00035 to 0.000625 GSSG: 0.1 to 0.2, Man 0.5 to 0.75, GA : 0.1 to 0.3
Hangover: 0.00035 to 0.0075 GSSG: 0.1 to 1.5, Man 0.5 to 2.5, GA : 0.04 to 0.3
Liver: TA01 0.00035 to 0.000625, GSSG: 0.5 to 1.0, Man 0.5 to 1.0, GA : 0.04 to 0.1
CNS: TA01 0.00035 to 0.000625, GSSG: 0.5 to 1.0, Man 0.5 to 1.0, GA : 0.04 to 0.1
Immunology liver: TA01 0.00035 to 0.000625, GSSG: 0.5 to 1.0, Man 0.5 to 1.0, GA : 0.04 to 0.1
Immunology DNA : 0.00035 to 0.0075 GSSG: 0.1 to 2.0, Man 0.5 to 2.5, GA : 0.04 to 0.3

**Liver & DNA**

**TA-01 (Tannal 01) + Sor (D-Sorbitol) +DP( L-Ala-L-Gln or L-Alanine-Glutamine) + GA(18β-GA or 18β--Glycyrrhizin)**

DNA: TA01 0.00035 to 0.0075 DP: 0.5 to 2.0, Sor 0.5 to 1.5, GA : 0.04 to 0.3
Hangover: TA01 0.00125 to 0.0075 DP: 0.5 to 2.0, Sor 0.5 to 1.5, GA : 0.04 to 0.3
Liver: TA01 0.00035 to 0.0025, DP: 0.5 to 1.5, Sor 0.5 to 1.5, GA : 0.04 to 0.3
CNS: TA01 0.00035 to 0.0025, DP: 0.5 to 1.5, Sor 0.5 to 1.5, GA : 0.04 to 0.3
Immunology Liver: TA 0.00035 to 0.0025,DP: 0.5 to 1.5, Sor 0.5 to 1.5, GA : 0.04 to 0.3
Immunology DNA: TA01 0.00035 to 0.0075 DP: 0.5 to 2.0, Sor 0.5 to 1.5, GA : 0.04 to 0.3

**Liver & DNA:**

**TA-02 (Tannal 02) + Man (D-Mannitol) +DP( L-Ala-L-Gln or L-Alanine-Glutamine) + GA(18β-GA or 18β--Glycyrrhizin)**

Liver: TA02 0.00035 to 0.0025, DP: 0.5 to 1.0, Man 0.5 to 1.5, GA : 0.04 to 0.3
CNS: TA02 0.00035 to 0.0025, DP: 0.5 to 1.0, Man 0.5 to 0.75, GA : 0.04 to 0.3
DNA: 0.00035 to 0.0075 ,DP: 0.5 to 2.0, Man 0.5 to 0.75, GA : 0.04 to 0.3
Hangover: 0.00035 to 0.00125, DP: 0.5 to 2.0, Man 0.5 to 1.5, GA : 0.04 to 0.3
Immunology Liver: TA02 0.00035 to 0.0025, DP: 0.5 to 1.5, Man 0.5 to 1.5, GA : 0.04 to 0.3
Immunology DNA : 0.00035 to 0.0075,DP: 0.5 to 2.0, Man 0.5 to 1.5, GA : 0.04 to 0.3

**Liver & DNA:**

**TA-B (Tannal B) + Man (D-Mannitol) +DP( L-Ala-L-Gln or L-Alanine-Glutamine) + GA(18β-GA or 18β--Glycyrrhizin)**

Liver: TAB 0.00035 to 0.0025, DP: 0.5 to 1.5, Man 0.5 to 1.5, GA : 0.04 to 0.3
CNS: 0.00035 to 0.0025, DP: 0.5 to 1.0, Man 0.5 to 0.75, GA : 0.04 to 0.3
DNA: 0.00035 to 0.0075,DP: 0.5 to 2.0, Man 0.5 to 0.75, GA : 0.04 to 0.3
Hangover: 0.00035 to 0.0075, DP: 0.5 to 2.0, Man 0.5 to 1.5, GA : 0.04 to 0.3
Immunology Liver : 0.00035 to 0.0025, DP: 0.5 to 1.5, Man 0.5 to 1.5, GA : 0.04 to 0.3
Immunology DNA: 0.00035 to 0.0075, DP: 0.5 to 2.0, Man 0.5 to 1.5, GA : 0.04 to 0.3

**TA-01 (Tannal 01) + Man (D-Mannitol) +CP (Casein protein hydrosylate) + GA(18β-GA or 18β--Glycyrrhizin)**

DNA: 0.00035 to 0.00125 CP: 0.1 to 0.2, Man 0.5 to 0.75, GA : 0.04 to 0.3
Hangover: 0.00035 to 0.00125 CP: 0.1 to 0.2, Man 0.5 to 0.75, GA: 0.04 to 0.3
Immunology: 0.00035 to 0.00125 CP: 0.1 to 0.2, Man 0.5 to 0.75, GA: 0.04 to 0.3

[Ranges are in %. All the above ranges are in mass concentration or wt/v%]

Various combination of the formulation comprising of (a) hydrolysable tannin and one or more compounds from group of category such as (b) amino acid derived molecule, (c) a sugar alcohol or sugar, and (d) Saponin glycoside in calculated proportions in the alcohol exhibits a synergistic effect which alleviates hepatic stress, DNA damage parameter, and modulates/alleviates parameters of CNS, hangover and immunology, which leads to preventing alcohol-induced damage or impairment of organs, which could be temporary or permanent. It has unexpectedly been found that the combination of these components, in synergistically effective quantities, demonstrates statistically significant improvement in selected DNA damage recovery, liver damage recovery and hepatic stress parameters than either the performance of each one alone, or the performance which would be expected as an additive effect of the cumulative protecting effect of each individual component. In addition, a composition of amino acid derived molecule, hydrolysable tannin, a sugar alcohol or sugar, and Saponin glycoside in alcohol has also been found to reduce hepatic stress. Further, an unexpected synergy is observed when an alcoholic beverage composition including these class of compounds are consumed over the alcoholic beverage composition including these class of compounds incorporated separately.

The sequential consumption of the synergistic composition simultaneously with alcohol improves the physiological and psychological parameters, bringing them closer to normal values. The toxicity of the alcoholic beverage is significantly reduced.

While not necessary to an understanding of the composition and method for preparing or using of the composition, it is of illustrative benefit to first describe what appears to be the biochemical mechanism. The immune response is triggered by several pro-inflammatory cytokines such as Interleukin (IL), IL-10, IL-12, IL-15, IL-17, IL-18, IL-22 and IFN-γ, IFN-α, TNF-α, IL-1b; which are upregulated in the liver in response to alcohol exposure. These interleukins are useful markers of activated natural killer cells.

**EXPERIMENTAL STUDY AND EVALUATION:**

**Evaluation of DNA damage, Hepatic stress, Hangover, CNS and Immunology Parameters**

A design of experiments ("DOE") methodology, a standard methodology of experimentation used to obtain the maximum information from the minimum number of experiments, was employed to investigate the performance of the composition for modulating immunology parameters, CNS parameters, hangover, hepatic stress, DNA damage and liver damage.

To evaluate DNA damage, one or more of the following methodology can be used:

1. Comet assay studies from peripheral lymphocytes.

In particular, Comet assay studies have been used for the evaluation of DNA damage of the present invention.

**COMET ASSAY:**

The Single Cell Gel Electrophoresis assay (SCGE, also known as comet assay) is a straightforward and sensitive technique for the detection of DNA damage at the level of the individual eukaryotic cell. It is being used as a standard technique for evaluation of DNA damage/repair, biomonitoring and genotoxicity testing. It involves the encapsulation of cells in a low-melting-point agarose suspension, lysis of the cells in neutral or alkaline (pH>13) conditions, and electrophoresis of the suspended lysed cells. The term "comet" refers to the pattern of DNA migration through the electrophoresis gel, which often resembles a comet (Cheng-Hung Chuang, Miao-Lin Hu. Use of whole blood directly for single-cell gel electrophoresis (comet) assay in vivo and white blood cells for in vitro assay. Mutation Research; 564 (2004) 75-82.).

### ALT, AST & ALP

For the hepato protective study the alanine aminotransferase (ALT), aspartate aminotransferase (AST) and alkaline phosphatise (ALP) (from serum) were taken into consideration.

One or more parameters were selected to evaluate hepatic stress. To evaluate alterations in liver functions, if any, serum ALT, AST, ALP were estimated. The levels of alanine aminotransferase (ALT), aspartate aminotransferase (AST) and alkaline phosphatise (ALP) in serum were measured using commercial spectrophotometric kit bought from Span Diagnostic Ltd. (www.span.in) according to the manufacturer's protocols.

The levels of "ALT" in serum were measured using commercial spectrophotometric kit bought from Span Diagnostic Ltd. (www.span.in) according to the manufacturer's' protocols (Begmeyer HU and Bernt E, 1974, Methods of enzymatic analysis, VerlagChemie, Weinhelm, Academic press, London, New York, Vol 2, p735).

The levels of "AST" in serum were measured using commercial spectrophotometric kit bought from Span Diagnostic Ltd. (www.span.in) according to the manufacturer's' protocols (Tietz, Norbert W. Fundamentals of Clinical Chemistry. 3rd ed. Philadelphia: W.B. Saunders, 1970. 447. Print.).

The levels of alkaline phosphatise ("ALP") in serum were measured using commercial spectrophotometric kit bought from Span Diagnostic Ltd. (www.span.in) according to the manufacturer's' protocols (Varley, H., Gowenlock, A. H., and Bell, M. Practical Clinical Biochemistry. Vol I. Fifth edn. London: William Heinemann Medical Books Lts, 1980. 453. Print).

### ACETYLCHOLINE ESTERASE

Acetylcholine esterase activity from brain tissue was measured following the method of Worek F, et al., 2012, Determination of acetylcholinesterase activity by the Ellman assay: A versatile tool for in vitro research on medical countermeasures against organophosphate poisoning, Drug testing and analysis, 4:282-291.

### MCP-1 & IL-12

Immunology parameters were evaluated using the measurement of MCP-1 and IL-12. The levels of "MCP 1" in serum were measured using ELISA kit bought from E Labscience Ltd. *(www.elabscience.com)* following manufacturer's' protocols (Wang L, Li Y, Chen J, et al. "Ischemic Cerebral Tissue and MCP-1 Enhance Rat Bone Marrow Stromal Cell Migration in Interface Culture." Experimental Hematology, 30, 7, 2002, 831-836).The levels of "Il-12" in serum were measured using ELISA kit bought from E Labscience Ltd. (*www.elabscience.com*) following manufacturer's' protocols (Trinchieri G. "Interleukin-12: A Cytokine at the Interface of Inflammation and Immunity." Adv. Immunol., 70, 1998, 83-243).

### For evaluation of Synergism:

1) DNA (1 day) study comet assay was performed.
2) Hangover (1 day) study: One or more parameter from IL-12, ACHase were evaluated.
3) Immunology (1 day) study One or more parameter from IL-12, MCP-1 were evaluated
4) Immunology (5 day) One or more parameter from IL-12, MCP-1were evaluated
5) CNS (5 day) IL-12 & ACHase was evaluated.
6) Liver (5 day): One or more parameter from AST, ALT, ALP were evaluated.

### Methods of Preparation:

### a) Beverage Additive

An embodiment of the method of preparation includes the following steps.

The ingredients which comprise two or more active ingredients selected from group comprising sugar alcohol, saponin glycoside, hydrolysable tannin and amino acid derived molecules were mixed in a reactor. The components were added portion wise in small quantities. A food preservative like potassium sorbate was added (optional) and the pH was adjusted to be in the range from 4.0 to 10.0. A flavouring agent was optionally added. The dissolved formulation was filtered using various filtering techniques and then the clear solution was bottled (as beverage additive).

**b) Alcoholic Beverage**

The ingredients which comprise two or more active ingredients selected from group comprisingsugar alcohol, saponin glycoside, hydrolysable tannin and amino acid derived molecules were mixed in a reactor containing alcohol and water. The components were added portion wise in small quantities. A food preservative like potassium sorbate (optional) was added and the pH was adjusted to be in the range from 4.0 to 10.0. A flavouring agent was optionally added. The dissolved formulation was filtered using various filtering techniques and then the clear solution was bottled (alcoholic beverage/drink).

There could be other variations of preparing the composition and only one embodiment of preparation is disclosed here. The other form of beverage additive can be solid, semisolid, powder etc as described in paragraphs above.

In similar way beverage additive formulations can be prepared without potassium sorbate.

In another aspect the invention provides a process for preparation of above composition. The ingredients which comprise two or more active ingredients selected from group comprising sugar alcohol (0.5-2.5%), hydrolysable tannin (0.00035-0.025%) and amino acid derived molecules (0.1-2.0%) were mixed in a reactor portion wise in small quantities containing alcohol and water. After mixing above ingredient saponin glycoside (0.01-0.3%) were added portion wise due to bubbling tendency. A food preservative like potassium sorbate (optional) was added and the pH was adjusted to be in the range from 4.0 to 10.0. The adjustment of pH & addition of L-Ala-L-Gln are critical as observed during stability studies.A flavouring agent was optionally added. The dissolved formulation was filtered using various filtering techniques and then the clear solution was bottled (alcoholic beverage).

In one embodiment the process comprises below steps:

(a) obtaining water or alcohol or alcohol-water mixture;
(b) mixing two or more ingredients selected from group comprising sugar alcohol, hydrolysable tannin and amino acid derived molecules in a reactor portion wise in small quantities containing water or alcohol or alcohol-water mixture of step (a);
(c) after mixing above ingredients saponin glycoside is added portion wise;
(d) adjusting the pH of the resulting solution of step (c) between 4.0 - 10; and
(e) obtaining the required beverage composition, filtering and bottling.

## MATERIALS AND METHODS

**Animal studies were approved by animal ethics committee (Reference No-IAEC/MC-1/2017/1; Dated24/02/2017).**

### Reagents

Distilled ethanol was obtained from Bengal Chemicals, West Bengal, India. Biochemical kits like AST, ALT, ALP and total protein were obtained from Span Diagnostics Ltd. Surat, India. Rat IL-12 ELISA kit and Rat MCP-1 ELISA kit (Elabscience Biotechnology Co.Ltd, WuHan, P.R.C.). All the chemicals used in the present study were of analytical grade and obtained from the following companies: Sigma (St. Louis, MO, USA), Merck (Mumbai, India), S D fine chemicals (Mumbai, India) and Qualigen (Mumbai, India).

### Animals

Male Wistar albino rats weighing 150-200 g were procured from local registered traders (544/GO/Re/S/02/CPCSEA valid up to 22/12/2021), Kolkata, India and were acclimatized for 7 days at standard housing condition ($26°C \pm 2°C$, 60-70% RH with $12 \pm 1$ hours light and dark cycle). Animals were fed with commercially available diet (Lipton India Pvt. Ltd, India) and water *ad-libitum* during the experiment period.

### Experimental design

(a) **Animal study model 1:** Saline/Alcohol (4.0 gm/kg/day, p.o.) / Formulation with alcohol (4.0 gm/kg/day, p.o.) were administered (fed orally by orogastric cannula) for one day. 12-16 hours after the dose the animals were sacrificed and blood samples were collected for estimation of biochemical parameters. Whole brain without cerebellum were dissected out, 10% homogenate was prepared with 0.1 M PB (pH 7.4). The homogenate solution was centrifuged at 10000 rpm for 20 minutes at 4 °C and the supernatant was used for estimation of acetylcholinesterase activity, which was estimated by Ellman's method (Ellman GL et al. A new and rapid colorimetric determination of acetylcholinesterase activity. Biochem Pharmacol, 1961, 7:88.)

**(b)Animal study model 2:** Saline/Alcohol (4.0 gm/kg/day, p.o.) / Formulation with alcohol (4.0 gm/kg/day, p.o.) were administered (fed orally by orogastric cannula) for one day. Blood samples were collected before administration of the Saline/Alcohol/ Formulation with alcohol and designated as '0' hour; then four (4hours after the dose. For comet assay blood was collected in heparinised tube. Single cell gel electrophoresis (SCGE, the Comet assay) was carried out using whole blood [Cheng-Hung Chuang, Miao-Lin Hu. Use of whole blood directly for single-cell gel electrophoresis (comet) assay in vivo and white blood cells for in vitro assay. Mutation Research; 564 (2004) 75-82.]. In short, 15 $\mu$l of whole blood was mixed with low melting agarose and fixed on a microscope slide. Those are then lysed by buffer enriched with detergent and high salt. Concomitant electrophoresis at alkaline pH results in unwinding of DNA followed by structures close to comets, when observed by fluorescence microscopy. The intensity of the comet tail relative to the head presents the number of DNA breaks. The probable basis for this is that loops containing a break lose their super coiling and become free to extend toward the anode. This is followed by analysis of photographs using CASP_1.2.3.b1software with staining of DNA and calculating fluorescence to determine the extent of DNA damage [Collins, A. R. (2004). "The comet assay for DNA damage and repair: principles, applications, and limitations". Mol. Biotechnol. 26 (3): 249-261].

**% Protection calculated in above experiments:**

$$\text{\% Protection} = 1 - [(\text{T-NS}) / (\text{AL-NS})] \times 100$$

T = Mean value of drug treated, NS = Mean value of normal control, AL = Mean value of alcohol alone.

**EXAMPLES**

**Example 1: (Hepatoprotection Study)**

**a) Model for Biological Testing:**

Male Wistar albino rats weighing 150-200 g were procured and randomly divided into groups consisting of eight (8) animals in each group. Hepatoprotection was induced by alcohol in rats by oral administration of 30% alcohol (4 gm/kg/day, p.o.) for 5 days and this group served as the negative control and treated groups received different formulation.

**b) Preparation of drug solution:**

All drug solutions were prepared in 40% aqueous alcohol, adjusting the pH in the range of 4.0-10.0 for evaluation of alcohol induced Hepatoprotection. This solution was further diluted with distilled water to obtain 30% aqueous alcoholic solution and administered orally by gavage to different rats group of step (1a).

**c) Evaluation of Hepato-Protective Activity:**

On day 6, the animals were anaesthetized with ether and blood samples were collected by cardiac puncture and the serum was used for the assay of marker enzymes viz. serum alanine aminotransferase (ALT), aspartate aminotransferase (AST), alkaline phosphatase (ALP).

**Example 2: (Hangover and CNS Studies)**

**a) Model for Biological Testing:**

Male Wistar albino rats weighing 150-200 g were procured and randomly divided into groups consisting of eight (8) animals in each group. Alcohol induced hangover and CNS related biochemical changes in rats by oral administration of 30% alcohol (4 gm/kg/day, p.o.) for 1 and 5 days and this group served as the negative control and treated groups received different formulation.

**b) Preparation of drug solution:**

All drug solutions were prepared in 40% aqueous alcohol, adjusting the pH in the range of 4.0-10.0 for evaluation of alcohol induced hangover and CNS related biochemical parameters. This solution was further diluted with distilled water to obtain 30% aqueous alcoholic solution and administered orally by gavage to different rats group of step (2a).

**c) Evaluation of Hangover and CNS Parameters:**

On day 2 and day 6, blood was collected from rats by cardiac puncture under mild ether anaesthesia and immediately after that the animals were decapitated and cerebral cortex was removed and stored at -20° C for further use. Acetylcholine esterase from brain homogenate was estimated for 1 day for hangover and 5 days for CNS. Interleukin-12 (IL-12) was also estimated from serum was on 1 day for hangover and 5 days for CNS biochemical parameter.

**Example 3: (Immunological Study)**

**a) Model for Biological Testing:**

Male Wistar albino rats weighing 150-200 g were procured and randomly divided into groups consisting of eight (8) animals in each group. Alcohol triggered modulation of the immune response in rats by oral administration of 30% alcohol (4 gm/kg/day, p.o.) for 1 and 5 days and this group served as the negative control and treated groups received different formulation.

**b) Preparation of drug solution:**

All drug solutions were prepared in 40% aqueous alcohol, adjusting the pH in the range of 4.0-10.0 for evaluation of alcohol triggered modulation of the immune response. This solution was further diluted with distilled water to obtain 30% aqueous alcoholic solution and administered orally by gavage to different rats group of step (3a).

**c) Evaluation of immunological Parameters:**

On day 2 and day 6, the animals were anaesthetized with ether and blood samples were collected by cardiac puncture and the serum was used for the assay of marker enzymes viz. interleukin-12 (IL-12) and Monocyte chemoattractant protein -1 (MCP-1) for 1day and 5 days.

**Example 4: (DNA protection Study)**

**a) Model for Biological Testing:**

Male Wistar albino rats weighing 150-200 g were procured and randomly divided into groups consisting of six (6) animals in each group. DNA damage was induced by alcohol in rats by oral administration of 30% alcohol (4 gm/kg/p.o.) once (single dose) and this group served as the negative control and treated groups received different formulations.

**b) Preparation of drug solution:**

All drug solutions were prepared in 40% aqueous alcohol, adjusting the pH in the range of 4.0-10.0 for evaluation of alcohol induced DNA damage and protection against it. This solution was further diluted with distilled water to obtain 30% aqueous alcoholic solution and administered orally by gavage to different rats group of step (4a).

**c) Evaluation of DNA protecting Activity:**

Before administration of alcohol and different drug solutions (mentioned in 4b), blood was collected from tail vein of the rats and designated as '0' hr followed by blood collection at 4 hr after administration of the drug solutions. The whole blood (uncoagulated) were used for comet assay.

**Example 5: (General Preparations/Formulations)**

**Five ingredients**

Sugar Alcohol (D-Mannitol, D-Sorbitol) 0.5-2.5g, Saponin Glycoside (18-Beta-Glycyrrhizin or 18-Alpha-Glycyrrhizin) 0.01-0.3g Amino Acid Derivative (L-Ala-L-Gln, GSSG, CP) 0.1-2.0g, Tannic acid (TA-01, TA02, TA-B) 0.00035-0.025g, and pH adjusting agent (potassium sorbate) 0.0-0.1gwere dissolved in aqueous alcohol (100 ml) to provide a corresponding 0.61035-4.925% (Total of 5 ingredient) solution.

**Four Ingredients**

Sugar Alcohol (D-Mannitol, D-Sorbitol) 0.5-2.5g, Saponin Glycoside (18-Beta-Glycyrrhizin or 18-Alpha-Glycyrrhizin) 0.01-0.3g Amino Acid Derivative (L-Ala-L-Gln, GSSG, CP) 0.1-2.0g & Tannic acid (TA-01, TA02, TA-B) 0.00035-0.025g were dissolved in aqueous alcohol (100 ml) to provide a corresponding 0.61035-4.825% (Total of 4 ingredient) solution.

**Amino Acid /Derivative**

**(a)** Tannic acid Tanal 01 0.00035g to 0.025g was dissolved in aqueous alcohol (100 ml) to provide a corresponding 0.00035% to 0.025% ; solution Ref 1 and Ref. 6 for DNA; and Tanal 02 or Brewtan F 0.00035 g to 0.00125g was dissolved in aqueous alcohol (100 ml) to provide a corresponding 0.00035% to 0.00125% solution Ref. 83, 85, 93 and 96 respectively (for DNA) TA-01: 0.000625g (Liver Ref 3) was dissolved in aqueous alcohol (100 ml) to provide a corresponding 0.000625% solution.TA-02 0.00035g (Liver Ref 64) were dissolved in aqueous alcohol (100 ml) to provide a corresponding 0.00035%. TAB: 0.00035g (Liver Ref 73) were dissolved in aqueous alcohol (100 ml) to provide a corresponding 0.00035% solution. This solution was administered in several portions to one of the rats group of Example (1a) or (2a) or (3a) or (4a). The administration was carried out over as per example 1(a) or 2(a) or 3(a) or 4 (a). Evaluation of hepato-protective activities were carried out as per Example (1c), hangover and CNS activities were carried out as per Example (2c) and immunological activities were carried out as per Example (3c) and DNA studies as per Example (4c).
**(b)** Sugar Alcohol D-Mannitol0.5g to 2.5g was dissolved in aqueous alcohol (100 ml) to provide a corresponding 0.5% to 2.5% solution Ref 11 and Ref. 15 for DNA and Sorbitol0.5g to 1.5gwas dissolved in aqueous alcohol (100 ml) to provide a corresponding 0.5% to 1.5% solution Ref 75 and Ref. 77 for DNA. Sugar Alcohol (D-Mannitol): 0.75g (Liver Ref 12) was dissolved in aqueous alcohol (100 ml) to provide a corresponding 0.75% solution. Sugar Alcohol (D-Sorbitol):0.5g (Liver Ref 55) was dissolved in aqueous alcohol (100 ml) to provide a corresponding 0.5% solution. Experiment was carried out following the method mentioned in Example 5 a.
**(c)** Saponin Glycoside (18$\alpha$-GA or 18$\beta$-GA or $\alpha$-GA+$\beta$-GA or other) 0.02g to 0.3g was dissolved in aqueous alcohol (100 ml) to provide a corresponding 0.01% to 0.3% solution Ref. 16 and Ref. 19 for DNA. Saponin Glycoside (18-alfa-Glycyrrhizin):0.04g (DNA Ref 42 and 44) to 0.3 g was dissolved in aqueous alcohol (100 ml) to provide a corresponding 0.04% to 0.3 % (Total of 1 ingredient) solution. (18-Beta-Glycyrrhizin):0.1g (Liver Ref 16) was dissolved in aqueous alcohol (100 ml) to provide a corresponding 0.1% solution. Saponin Glycoside (18-alfa-Glycyrrhizin):0.04g (Liver Ref 38) was dissolved in aqueous alcohol (100 ml) to provide a corresponding 0.04% (Total of 1 ingredient) solution. Experiment was carried out following the method mentioned in Example 5 a.
**(d)** Amino Acid Derivative (L-Alanyl-L-Glutamine or Oxidized L-Glutathione or hydrolyzed Casein Protein or other) 0.5g to 2.0g was dissolved in aqueous alcohol (100 ml) to provide a corresponding 0.5% to 2.0% solution. Ref 7 and Ref. 10 for DNA.Oxidized glutathione (GSSG):0.1g to 1 g (DNA Ref 56 and 59) was dissolved in aqueous alcohol (100 ml) to provide a corresponding 0.1 to 1% solution. Hydrolyzed Casein Protein 0.1g to 0.2 g was dissolved in aqueous alcohol (100 ml) to provide a corresponding 0.1% to 0.2% solution(DNA Ref 97 and 98).L-Ala-L-Gln:1.0 g (Liver Ref 8) was dissolved in aqueous alcohol (100 ml) to provide a corresponding 1% solution. Oxidized glutathione (GSSG):0.2g (Liver Ref 46) was dissolved in aqueous alcohol (100 ml) to provide a corresponding 0.2% solution. Experiment was carried out following the method mentioned in Example 5 a.

Following the similar procedures as described above for single ingredients, formulations for varying concentrations of each of TA-01, TA-02, TA-B, L-Ala-L-Gln, GSSG, CP, Man, Sor, 18$\alpha$-GA, 18$\beta$-GA, KS as presented in TABLE-1 and TABLE-2 and any other single ingredients from groups tannic acid, amino acid derivative, sugar alcohol and saponin glycoside were prepared/can be prepared.

**Combination of Two Active Ingredients:**

**(e)** Sugar Alcohol (D-Mannitol or D-Sorbitol or other) 0.5g and Saponin Glycoside (18α-GA or 18β-GA or α-GA+β-GA or other) 0.1g were dissolved in aqueous alcohol (100 ml) to provide a corresponding 0.6 % solution. Ref. 35 for DNA.Sugar Alcohol (D-Mannitol)0.5g and Tannic acid TA-01 0.000625g (Liver Ref 24) were dissolved in aqueous alcohol (100 ml) to provide a corresponding 0.500625% solution. Experiment was carried out following the method mentioned in Example 5 a.

**(f)** Amino Acid Derivative (Alanyl-Glutamine or Oxidized Glutathione or hydrolyzed Casein Protein or other) 1g and Saponin Glycoside (18α-GA or 18β-GA or α-GA+β-GA or other)0.1g were dissolved in aqueous alcohol (100 ml) to provide a corresponding 1.1% solution. Ref. 34 for DNA. SaponinGlycoside(18-Beta-Glycyrrhizin) 0.04g and Tannic acid TA-01 0.000625g (Liver Ref 25) were dissolved in aqueous alcohol (100 ml) to provide a corresponding 0.040625% solution. Experiment was carried out following the method mentioned in Example 5 a.

Following the similar procedures as described above for two ingredients, formulations of two ingredients for varying concentrations of each of TA-01, TA-02, TA-B, L-Ala-L-Gln, GSSG, CP, Man, Sor, 18α-GA, 18β-GA, KS as presented in TABLE-1 and TABLE-2 and any other two ingredients each selected from the groups tannic acid, amino acid derivative, sugar alcohol and saponin glycoside were prepared/can be prepared.

**Combination of Three Active Ingredients:**

**(g)** Sugar Alcohol (D-Mannitol or D-Sorbitol or other) 0.5g, Saponin Glycoside (18α-GA or 18β-GA or α-GA+β-GA or other) 0.1g and Amino Acid Derivative (Alanyl-Glutamine or Oxidized Glutathione or hydrolyzed Casein Protein or other) 1g were dissolved in aqueous alcohol (100 ml) to provide a corresponding 1.6% solution. Ref. 33 for DNA. Amino Acid Derivative L-Ala-L-Gln 0.5g and Sugar Alcohol (D-Mannitol)0.5g and Tannic acid TA-01 0.000625g (Liver Ref 26) were dissolved in aqueous alcohol (100 ml) to provide a corresponding 1.000625% solution. Experiment was carried out following the method mentioned in Example 5 a.

**(h)** Tannic acid 0.00125 g, Sugar alcohol (D-mannitol or D-sorbitol) 0.5 g and Saponin Glycoside (18α-GA or 18β-GA or α-GA+β-GA or other) 0.1gwere dissolved in aqueous alcohol (100 ml) to provide a corresponding 0.60125% solution. Ref. 32 for DNA. Sugar Alcohol (D-Mannitol) 0.5g and SaponinGlycoside(18-Beta-Glycyrrhizin) 0.04g and Tannic acid TA-01 0.000625g (Liver Ref 27) were dissolved in aqueous alcohol (100 ml) to provide a corresponding 0.540625% solution. Experiment was carried out following the method mentioned in Example 5 a.

Following the similar procedures as described above for two ingredients, formulations of three ingredients for varying concentrations of each of TA-01, TA-02, TA-B, L-Ala-L-Gln, GSSG, CP, Man, Sor, 18α-GA, 18β-GA, KS as presented in TABLE-1 and TABLE-2 and any other three ingredients each selected from the groups tannic acid, amino acid derivative, sugar alcohol and saponin glycoside were prepared/can be prepared.

**Combination of Four Active Ingredients**

**(i)** Sugar Alcohol (D-Mannitol or D-Sorbitol or other) 0.5g to 2.5g, Saponin Glycoside (18α-GA or 18β-GA or α-GA+β-GA or other) 0.04g to 0.3g, Amino Acid Derivative (Alanyl-Glutamine or Oxidized Glutathione or hydrolyzed Casein Protein or other) 0.5g to 2.0g and Tannic acid derivative 0.00035g to 0.025g were dissolved in aqueous alcohol (100 ml) to provide a corresponding 1.04035% to 4.825% solution. (Ref. 39, Ref. 23 and Ref. 24 producing 3.30035 %, 2.85125% and 1.125% respectively for DNA). Sugar Alcohol (D-Mannitol)0.5gSaponinGlycoside(18-Beta-Glycyrrhizin) 0.1g Amino Acid Derivative L-Ala-L-Gln 1.0g and Tannic acid TA-01 0.0025g (Liver Ref 21) were dissolved in aqueous alcohol (100 ml) to provide a corresponding 1.6025% solution. Sugar Alcohol (D-Mannitol)0.5gSaponinGlycoside(18-Beta-Glycyrrhizin) 0.04g Amino Acid Derivative L-Ala-L-Gln 1.0g and Tannic acid TA-01 0.00125g (Liver Ref 22) were dissolved in aqueous alcohol (100 ml) to provide a corresponding 1.54125% solution. Sugar Alcohol (D-Mannitol)0.5gSaponinGlycoside(18-Beta-Glycyrrhizin) 0.02g Amino Acid Derivative L-Ala-L-Gln 1.0g and Tannic acid TA-01 0.000625g (Liver Ref 29) were dissolved in aqueous alcohol (100 ml) to provide a corresponding 1.520625% solution. Sugar Alcohol (D-Mannitol)2.5gSaponinGlycoside(18-Beta-Glycyrrhizin) 0.04g Amino Acid Derivative L-Ala-L-Gln 0.5g and Tannic acid TA-01 0.000625g (Liver Ref 32) were dissolved in aqueous alcohol (100 ml) to provide a corresponding 3.040625% solution. Sugar Alcohol (D-Mannitol)0.5gSaponinGlycoside(18-Beta-Glycyrrhizin) 0.04g Amino Acid Derivative L-Ala-L-Gln 1.0g and Tannic acid TA-01 0.0005g (Liver Ref 35) were dissolved in aqueous alcohol (100 ml) to provide a corresponding 1.5405% solution. Sugar Alcohol (D-Mannitol)0.5gSaponinGlycoside(18-Beta-Glycyrrhizin) 0.04g Amino Acid Derivative L-Ala-L-Gln 0.5g and Tannic acid TA-01 0.0025g (Liver Ref 37) were dissolved in aqueous alcohol (100 ml) to provide a corresponding 1.0425% Experiment was carried out following the method mentioned in Example 5 a.

(j) Sugar Alcohol (D-Mannitol or D-Sorbitol or other) 0.5g, Saponin Glycoside (18α-GA or 18β-GA or α-GA+β-GA or other) 0.04g to 0.1g, Amino Acid Derivative (Oxidized Glutathione) 0.1g to 1.0g and Tannic acid derivative 0.00035g to 0.00125g were dissolved in aqueous alcohol (100 ml) to provide a corresponding 0.64035% to 1.60125% solution. (Ref. 73, Ref. 72 and Ref. 62 producing 0.700625 %, 0.80035% and 1.54125% respectively for DNA). Oxidized glutathione (GSSG) 0.5g and Sugar Alcohol (D-Mannitol)0.5gSaponinGlycoside(18-beta-Glycyrrhizin) 0.04g and Tannic acid TA-01 0.00035g (Liver Ref 50) were dissolved in aqueous alcohol (100 ml) to provide a corresponding 1.04035% solution. Oxidized glutathione (GSSG) 0.2g and Sugar Alcohol (D-Mannitol)0.5gSaponinGlycoside(18-beta-Glycyrrhizin) 0.1g and Tannic acid TA-01 0.00035g (Liver Ref 53) were dissolved in aqueous alcohol (100 ml) to provide a corresponding 0.80035% solution. Experiment was carried out following the method mentioned in Example 5 a.

(k) Sugar Alcohol (Sorbitol) 0.5g to 1.5g, Saponin Glycoside (18α-GA or 18β-GA or α-GA+β-GA or other) 0.04g to 0.3g, Amino Acid Derivative (Alanyl-Glutamine or Oxidized Glutathione or hydrolyzed Casein Protein or other) 0.5g to 1.0g and Tannic acid derivative 0.00035g were dissolved in aqueous alcohol (100 ml) to provide a corresponding 1.04035% to 2.80035% solution. (Ref. 82, Ref. 79 producing 2.30035 %, and 1.54125% respectively for DNA). Sugar Alcohol (D-Sorbitol)0.5gSaponinGlycoside(18-beta-Glycyrrhizin) 0.04g Amino Acid Derivative L-Ala-L-Gln 0.5g and Tannic acid TA-01 0.000625g (Liver Ref 58) were dissolved in aqueous alcohol (100 ml) to provide a corresponding 1.040625% solution. Experiment was carried out following the method mentioned in Example 5 a.

(l) Sugar Alcohol (D-Mannitol or D-Sorbitol or other) 0.5g to 0.75g, Saponin Glycoside (18α-GA or 18β-GA or α-GA+β-GA or other) 0.04g to 0.3g, Amino Acid Derivative (Alanyl-Glutamine or Oxidized Glutathione or hydrolyzed Casein Protein or other) 1.0g to 2.0g and Tannic acid derivative (Tanal 02) 0.00035g to 0.00125g were dissolved in aqueous alcohol (100 ml) to provide a corresponding 1.54035% to 3.05125% solution.. (Ref. 85, Ref. 87 and Ref.88 producing 2.85125 %, 1.54125% and 2.05035% respectively for DNA). Sugar Alcohol (D-Mannitol)0.5gSaponinGlycoside(18-beta-Glycyrrhizin) 0.04g Amino Acid Derivative L-Ala-L-Gln 0.5g and Tannic acid 2 TA-02 0.000625g (Liver Ref 67) were dissolved in aqueous alcohol (100 ml) to provide a corresponding 1.040625% solution. Sugar Alcohol (D-Mannitol)0.5gSaponinGlycoside(18-beta-Glycyrrhizin) 0.04g Amino Acid Derivative L-Ala-L-Gln 0.5g and Tannic acid 2 TA-02 0.00035g (Liver Ref 71) were dissolved in aqueous alcohol (100 ml) to provide a corresponding 1.04035% solution. Experiment was carried out following the method mentioned in Example 5 a.

(m)Sugar Alcohol (D-Mannitol or D-Sorbitol or other) 0.5g to 0.75g, Saponin Glycoside (18α-GA or 18β-GA or α-GA+β-GA or other) 0.04g to 0.3g, Amino Acid Derivative (Alanyl-Glutamine or Oxidized Glutathione or hydrolyzed Casein Protein or other) 0.5g to 2.0g and Tannic acid derivative (Brewtan F) 0.00035g to 0.00125g were dissolved in aqueous alcohol (100 ml) to provide a corresponding 1.04035% to 3.05125% solution. (Ref. 93, Ref. 92 and Ref.96 producing 2.85125 %, 1.54125% and 2.05035% respectively for DNA). Sugar Alcohol (D-Mannitol)0.5gSaponinGlycoside(18-beta-Glycyrrhizin) 0.04g Amino Acid Derivative L-Ala-L-Gln 0.5g and Tannic acid B TA-0B 0.0025g (Liver Ref 76) were dissolved in aqueous alcohol (100 ml) to provide a corresponding 1.0425% solution. Sugar Alcohol (D-Mannitol)0.5gSaponinGlycoside(18-beta-Glycyrrhizin) 0.04g Amino Acid Derivative L-Ala-L-Gln 0.5g and Tannic acid B TA-0B 0.00035g (Liver Ref 79) were dissolved in aqueous alcohol (100 ml) to provide a corresponding 1.04035% solution. Sugar Alcohol (D-Mannitol)0.75gSaponinGlycoside(18-beta-Glycyrrhizin) 0.1g Amino Acid Derivative L-Ala-L-Gln 1.0g and Tannic acid B TA-B 0.00065g (Liver Ref 80) were dissolved in aqueous alcohol (100 ml) to provide a corresponding 1.850625% solution. Experiment was carried out following the method mentioned in Example 5 a.

(n) Sugar Alcohol (D-Mannitol or D-Sorbitol or other) 0.5g, Saponin Glycoside (18α-GA or 18β-GA or α-GA+β-GA or other) 0.04g to 0.3g, Amino Acid Derivative (hydrolyzed Casein Protein or other) 0.1g to 0.2g and Tannic acid derivative 0.00035g to 0.00125g were dissolved in aqueous alcohol (100 ml) to provide a corresponding 0.64035% to 1.00125% solution. (Ref. 103, Ref. 101 producing 0.64035% and 1.00125 respectively for DNA).Experiment was carried out following the method mentioned in Example 5 a.

Following the similar procedures as described above for two ingredients, formulations of four ingredients for varying concentrations of each of TA-01, TA-02, TA-B, L-Ala-L-Gln, GSSG, CP, Man, Sor, 18-GA, 18β-GA, KS as presented in TABLE-1 and TABLE-2 and any other four ingredients each selected from the groups tannic acid, amino acid derivative, sugar alcohol and saponin glycoside were prepared/can be prepared.

Following the similar procedures as described above for four ingredients, formulations of two ingredients for varying concentrations of each of TA-01, TA-02, TA-B, L-Ala-L-Gln, GSSG, CP, Man, Sor, 18-GA, 18β-GA, KS as presented in TABLE-1 and TABLE-2 above and any other four ingredients each selected from the groups tannic acid, amino acid derivative, sugar alcohol and saponin glycoside were prepared and/or can be prepared.

Preparations/formulations comprising a single active ingredient, two active ingredients, three active ingredients and four active ingredients as mentioned in the example 5 above and also additionally optionally comprising a pH adjusting agent and/or flavouring agent can be prepared and biological evaluation can carried out by the way as described in above examples 1-4 and also as per method of preparation as described above. Further non-limiting specific examples are as given below. The examples below are illustrative and in similar way other combinations and/or beverage additive or beverage comprising combinations of active ingredients can be prepared by taking suitable amounts of active ingredients and optionally suitable amounts of pH adjusting agents and/or flavouring agents.

In the similar way as exemplified above other preparations comprising a single active ingredient, two active ingredients, three active ingredients and four active ingredients as mentioned in the example 5 above and also additionally optionally comprising a pH adjusting agent and/or flavouring agent can be prepared by taking appropriate required concentration/amount of each ingredient.

**Specific Examples**

Below are some non-limiting examples with specific concentration(s) of ingredients(s) in the composition which were prepared and evaluated for different activities as described in examples 1-4. In similar manner and as described in general example 5 above, other combination compositions with different concentration of ingredients as presented in Table-1 and Table-2 were prepared and evaluated for activities.

**EXAMPLE-6 (TANNIC ACID)**

(a) **Tanal 01 0.00035g** was dissolved in aqueous alcohol (100 ml) to provide a corresponding 0.00035% solution. This solution was administered in several portions to one of the rats group of Example (1a) or (2a) or (3a) or (4a). The administration was carried out over as per example 1(a) or 2(a) or 3(a) or 4 (a). Evaluation of hepato-protective activities were carried out as per Example (1c), hangover and CNS activities were carried out as per Example (2c) and immunological activities were carried out as per Example (3c) and DNA studies as per Example (4c).

(b) **Tanal 02 0.00035g** was dissolved in aqueous alcohol (100 ml) to provide a corresponding 0.00035% solution.

(c) **Tanal B 0.00035g** was dissolved in aqueous alcohol (100 ml) to provide a corresponding 0.00035% solution.

(d) **Tanal 01 0.025g** was dissolved in aqueous alcohol (100 ml) to provide a corresponding 0.025% solution.

(e) **Tanal 02 0.025g** was dissolved in aqueous alcohol (100 ml) to provide a corresponding 0.025% solution.

(f) **Tanal B 0.025g** was dissolved in aqueous alcohol (100 ml) to provide a corresponding 0.025% solution.

(g) Following similar procedures as described in example 6a, solutions of Tanal-01, Tanal-02 and Tanal B with different concentrations as presented in Table-1 and/or Table-2 viz. 0.0005%, 0.000625%, 0.00125%, 0.0075% and 0.005% were prepared taking 0.0005g, 0.000625g, 0.00125g, 0.0075g and 0.005g in aqueous alcohol (100 ml) respectively of Tanal-01 or Tanal-02 or Tanal B.

Administrations to rats and evaluation of activities such as hepato-protective, CNS, immunological and DNA for solutions prepared in Examples 6(b) to 6(g) were carried out as per procedures described in Example 6a (similar as in examples 1-4). The results i.e. % protection and/or % synergism observed are presented in Table-1 and/or Table-2.

**EXAMPLE-7 (Amino Acid : L-Ala-L-Gln, GSSG, CP)**

(a) **Amino acid: L-Ala-L-Gln: 0.5 g** was dissolved in aqueous alcohol (100 ml) to provide a corresponding 0.5% solution. This solution was administered in several portions to one of the rats group of Example (1a) or (2a) or (3a) or (4a). The administration was carried out over as per example 1(a) or 2(a) or 3(a) or 4 (a). Evaluation of hepato-protective activities were carried out as per Example (1c), hangover and CNS activities were carried out as per Example (2c) and immunological activities were carried out as per Example (3c) and DNA studies as per Example (4c).

(b) **Amino acid: L-Ala-L-Gln: 2 g** was dissolved in aqueous alcohol (100 ml) to provide a corresponding 2% solution.

(c) **Amino acid: GSSG: 0. g** was dissolved in aqueous alcohol (100 ml) to provide a corresponding 0.1% solution.

(d) **Amino acid: GSSG: 2 g** was dissolved in aqueous alcohol (100 ml) to provide a corresponding 2% solution.

(e) **Amino acid: Casein Protein Hydrolysate (CP): 0.1 g** was dissolved in aqueous alcohol (100 ml) to provide a corresponding 0.1% solution.

**(f) Amino acid: Casein Protein Hydrolysate (CP): 2 g** was dissolved in aqueous alcohol (100 ml) to provide a corresponding 2% solution.

**(g)** Following similar procedures as described in example 7a, solutions of L-Ala-L-Gln, GSSG and CP with different concentrations as presented in Table-1 and/or Table-2 viz. 0.5%, 1%, 1.5%, 2% and 0.1%, 0.2%, 0.5%, 1%, 1.5% and 2% were prepared taking 0.1%, 0.2% and 0.5% in aqueous alcohol (100 ml) respectively of L-Ala-L-Gln, GSSG and CP.

Administrations to rats and evaluation of activities such as hepato-protective, CNS, immunological and DNA for solutions prepared in Examples 7(b) to 7(g) were carried out as per procedures described in Example 7a (similar as in examples 1-4). The results i.e. % protection and/or % synergism observed are presented in Table-1 and/or Table-2.

**EXAMPLE-8 (Sugar alcohol: D-Mannitol, D-Sorbitol)**

**(a) Sugar alcohol: D-Mannitol: 0.5 g** was dissolved in aqueous alcohol (100 ml) to provide a corresponding 0.5% solution. This solution was administered in several portions to one of the rats group of Example (1a) or (2a) or (3a) or (4a). The administration was carried out over as per example 1(a) or 2(a) or 3(a) or 4 (a).Evaluation of hepato-protective activities were carried out as per Example (1c), hangover and CNS activities were carried out as per Example (2c) and immunological activities were carried out as per Example (3c) and DNA studies as per Example (4c).

**(b) Sugar alcohol: D-Mannitol: 2.5 g** was dissolved in aqueous alcohol (100 ml) to provide a corresponding 2.5% solution.

**(c) Sugar alcohol: D-Sorbitol: 0.5g** was dissolved in aqueous alcohol (100 ml) to provide a corresponding 0.5% solution.

**(d) Sugar alcohol: D-Sorbitol: 1.5g** was dissolved in aqueous alcohol (100 ml) to provide a corresponding 1.5% solution.

**(e)** Following similar procedures as described in example 8a, solutions of D-Mannitol and D-Sorbitol with different concentrations as presented in Table-1 and/or Table-2 viz. 0.5%, 0.75%, 1%, 1.5% and 2% and 0.5%, 0.75%, 1% and 1.5%, were prepared taking in aqueous alcohol (100 ml) respectively of D-Mannitol and D-Sorbitol.

Administrations to rats and evaluation of activities such as hepato-protective, CNS, immunological and DNA for solutions prepared in Examples 8(b) to 8(e) were carried out as per procedures described in Example 8a (similar as in examples 1-4). The results i.e. % protection and/or % synergism observed are presented in Table-1 and/or Table-2.

**EXAMPLE-9 (Saponin glycosides: 18-beta-GA and 18-alpha-GA)**

**(a) Saponin glycosides: 18-beta-GA 0.01 g** was dissolved in aqueous alcohol (100 ml) to provide a corresponding 0.01% solution. This solution was administered in several portions to one of the rats group of Example (1a) or (2a) or (3a) or (4a). The administration was carried out over as per example 1(a) or 2(a) or 3(a) or 4 (a). Evaluation of hepato-protective activities were carried out as per Example (1c), hangover and CNS activities were carried out as per Example (2c) and immunological activities were carried out as per Example (3c) and DNA studies as per Example (4c).

**(b) 18-beta-GA: 0.3 g** was dissolved in aqueous alcohol (100 ml) to provide a corresponding 0.3% solution.

**(c) 18-alpha-GA: 0.04 g** was dissolved in aqueous alcohol (100 ml) to provide a corresponding 0.04% solution.

**(d) 18-alpha-GA: 0.3 g** was dissolved in aqueous alcohol (100 ml) to provide a corresponding 0.3% solution.

**(e)** Following similar procedures as described in example 9a, solutions of 18-beta-GA and 18-alpha-GA with different concentrations as presented in Table-1 and/or Table-2 viz. 0.01%, 0.02%, 0.04%, 0.1% and 0.3% and 0.04%, 0.1% and 0.3%, were prepared taking in aqueous alcohol (100 ml) respectively of 18-beta-GA and 18-alpha-GA.

Administrations to rats and evaluation of activities such as hepato-protective, CNS, immunological and DNA for solutions prepared in Examples 9(b) to 9(e) were carried out as per procedures described in Example 9a (similar as in examples 1-4). The results i.e. % protection and/or % synergism observed are presented in Table-1 and/or Table-2.

**Example 10: Combination of two ingredients**

**a) Tannic acid TA-01 0.00125g and Amino acid: L-Ala-L-Gln** 1g were dissolved in aqueous alcohol (100 ml) to provide a corresponding 1.00125% solution (DNA REF 29). This solution was administered in several portions to one of the rats group of Example (1a) or (2a) or (3a) or (4a). The administration was carried out over as per example 1(a) or 2(a) or 3(a) or 4 (a). Evaluation of hepato-protective activities were carried out as per Example (1c), hangover and CNS activities were carried out as per Example (2c) and immunological activities were carried out as per Example (3c) and DNA studies as per Example (4c).

**b) Tannic acid TA-01 0.000625g and Amino acid: L-Ala-L-Gln** 0.5g were dissolved in aqueous alcohol (100 ml) to provide a corresponding 0.500625% solution (LIVER REF 23).

c) Following the similar procedures as described above for two ingredients in example 10a-10b, formulations of two ingredients for varying concentrations of each of TA-01, TA-02, TA-B, L-Aln-L-Gln, GSSG, CP, Man, Sor, 18α-GA, 18β-GA, KS as presented in TABLE-1 and TABLE-2 and any other two ingredients each selected from the groups tannic acid, amino acid derivative, sugar alcohol and saponin glycoside were prepared/can be prepared. Administrations to rats and evaluation of activities such as hepato-protective, CNS, immunological and DNA for solutions prepared in Examples 10(b-c) were carried out as per procedures described in Example 10a (similar as in examples 1-4). The results i.e. % protection and/or % synergism observed are presented in Table-1 and/or Table-2.

**Example 11. Combination of three ingredients**

**a) Tannic acid TA-01 0.00125g and Amino acid: L-Ala-L-Gln** 1g and **18-beta-GA** 0.1g were dissolved in aqueous alcohol (100 ml) to provide a corresponding 1.10125% solution (DNA REF 27). This solution was administered in several portions to one of the rats group of Example (1a) or (2a) or (3a) or (4a). The administration was carried out over as per example 1(a) or 2(a) or 3(a) or 4 (a). Evaluation of hepato-protective activities were carried out as per Example (1c), hangover and CNS activities were carried out as per Example (2c) and immunological activities were carried out as per Example (3c) and DNA studies as per Example (4c).
b) **Tannic acid TA-01 0.00125g and Amino acid: L-Ala-L-Gln** 1g and **D-mannitol** 0.5g were dissolved in aqueous alcohol (100 ml) to provide a corresponding 1.50125% solution (DNA REF 28).
c) **Tannic acid TA-01 0.00125g** and **18-beta-GA** 0.1g and D-**mannitol** 0.5g were dissolved in aqueous alcohol (100 ml) to provide a corresponding 0.60125% solution (DNA REF 32).
d) **Tannic acid TA-01 0.000625g** and **Amino acid: L-Ala-L-Gln** 0.5g and D-**mannitol** 0.5g were dissolved in aqueous alcohol (100 ml) to provide a corresponding 1.006225% solution (LIVER REF 26).
e) **Tannic acid TA-01 0.000625g** and **Amino acid: L-Ala-L-Gln** 0.5g and **18-beta-GA** 0.04g were dissolved in aqueous alcohol (100 ml) to provide a corresponding 0.540625% solution (LIVER REF 28).
f) Following the similar procedures as described above for three ingredients in example 11 (a) to (e), formulations of three ingredients for varying concentrations of each of TA-01, TA-02, TA-B, L-Aln-L-Gln, GSSG, CP, Man, Sor, 18α-GA, 18β-GA, KS as presented in TABLE-1 and TABLE 2 and any other three ingredients each selected from the groups tannic acid, amino acid derivative, sugar alcohol and saponin glycoside were prepared/can be prepared.

Administrations to rats and evaluation of activities such as hepato-protective, CNS, immunological and DNA for solutions prepared in Examples 11(b) to 11(f) were carried out as per procedures described in Example 11a (similar as in examples 1-4). The results i.e. % protection and/or % synergism observed are presented in Table-1 and/or Table-2.

**Example 12. Combination of four ingredients**

**A: Tannic acid derivatives**

**a) Tannic acid TA-01 0.00035g and Amino acid: L-Ala-L-Gln** 0.5g, D-**mannitol** 2.5g and **18-beta-GA** 0.3g were dissolved in aqueous alcohol (100 ml) to provide a corresponding 3.30035% solution (DNA REF 39). This solution was administered in several portions to one of the rats group of Example (1a) or (2a) or (3a) or (4a). The administration was carried out over as per example 1(a) or 2(a) or 3(a) or 4 (a). Evaluation of hepato-protective activities were carried out as per Example (1c), hangover and CNS activities were carried out as per Example (2c) and immunological activities were carried out as per Example (3c) and DNA studies as per Example (4c).

b) **Tannic acid TA-01 0.00125g and Amino acid: L-Ala-L-Gln** 1g, **D-mannitol** 0.5g and **18-beta-GA** 0.1g were dissolved in aqueous alcohol (100 ml) to provide a corresponding 1.60125% solution (DNA REF 21).

c) **Tannic acid TA-01 0.025g** and **Amino acid: L-Ala-L-Gln** 0.5g, D-**mannitol** 0.5g and **18-beta-GA** 0.1g were dissolved in aqueous alcohol (100 ml) to provide a corresponding 1.125% solution (DNA REF 20).

d) **Tannic acid TA-01 0.00035g** and **Amino acid: L-Ala-L-Gln** 0.5g, D-**mannitol** 0.5g and **18-beta-GA** 0.04g were dissolved in aqueous alcohol (100 ml) to provide a corresponding 0.54035% solution (LIVER REF 42).

e) **Tannic acid TA-01 0.000625g** and **Amino acid: L-Ala-L-Gln** 0.5g, D-**mannitol** 0.5g and **18-beta-GA** 0.04g were dissolved in aqueous alcohol (100 ml) to provide a corresponding 1.04625% solution (LIVER REF 22B).

f) **Tannic acid TA-01 0.0025g** and **Amino acid: L-Ala-L-Gln** 0.5g, D-**mannitol** 0.5g and **18-beta-GA** 0.04g were dissolved in aqueous alcohol (100 ml) to provide a corresponding 1.0425% solution (LIVER REF 21).

g) **Tannic acid TA-02 0.00035g** and **Amino acid: L-Ala-L-Gln** 1g, D-**mannitol** 0.75g and **18-beta-GA** 0.3g were dissolved in aqueous alcohol (100 ml) to provide a corresponding 2.05035% solution (DNA REF 90).

h) **Tannic acid TA-02 0.00125g** and **Amino acid: L-Ala-L-Gln** 1g, D-**mannitol** 0.5g and **18-beta-GA** 0.04g were dissolved in aqueous alcohol (100 ml) to provide a corresponding 1.54125% solution (DNA REF 89).

i) **Tannic acid TA-02 0.0075g** and **Amino acid: L-Ala-L-Gln** 0.5g, D-**mannitol** 0.5g and **18-beta-GA** 0.3g were dissolved in aqueous alcohol (100 ml) to provide a corresponding 1.3075% solution (DNA REF 86)

k) **Tannic acid TA-02 0.00035g** and **Amino acid: L-Ala-L-Gln** 0.5g, D-**mannitol** 0.5g and **18-beta-GA** 0.04g were dissolved in aqueous alcohol (100 ml) to provide a corresponding 1.04035% solution (LIVER REF 71)

l) **Tannic acid TA-02 0.000625g** and **Amino acid: L-Ala-L-Gln** 0.5g, D-**mannitol** 0.5g and **18-beta-GA** 0.04g were dissolved in aqueous alcohol (100 ml) to provide a corresponding 1.040625% solution (LIVER REF 67)

m) **Tannic acid TA-02 0.025g** and **Amino acid: L-Ala-L-Gln** 0.5g, D-**mannitol** 0.5g and **18-beta-GA** 0.04g were dissolved in aqueous alcohol (100 ml) to provide a corresponding 1.0425% solution (LIVER REF 76)

n) **Tannic acid TA-B 0.00035g** and **Amino acid: L-Ala-L-Gln** 1g, D-**mannitol** 0.75g and **18-beta-GA** 0.3g were dissolved in aqueous alcohol (100 ml) to provide a corresponding 2.05035% solution (DNA REF 98).

o) **Tannic acid TA-B 0.00125g** and **Amino acid: L-Ala-L-Gln** 2g, D-**mannitol** 0.75g and **18-beta-GA** 0.1g were dissolved in aqueous alcohol (100 ml) to provide a corresponding 2.85125% solution (DNA REF 95).

p) **Tannic acid TA-B 0.0075g** and **Amino acid: L-Ala-L-Gln** 0.5g, D-**mannitol** 0.5g and **18-beta-GA** 0.3g were dissolved in aqueous alcohol (100 ml) to provide a corresponding 1.3075% solution (DNA REF 94)

Q) **Tannic acid TA-B 0.00035g** and **Amino acid: L-Ala-L-Gln** 0.5g, D-**mannitol** 0.5g and **18-beta-GA** 0.04g were dissolved in aqueous alcohol (100 ml) to provide a corresponding 1.04035% solution (LIVER REF 79)

r) **Tannic acid TA-B 0.000625g** and **Amino acid: L-Ala-L-Gln** 1.5g, D-**mannitol** 1.5g and **18-beta-GA** 0.04g were dissolved in aqueous alcohol (100 ml) to provide a corresponding 3.040625% solution (LIVER REF 78)

s) **Tannic acid TA-B 0.025g** and **Amino acid: L-Ala-L-Gln** 0.5g, D-**mannitol** 0.5g and **18-beta-GA** 0.04g were dissolved in aqueous alcohol (100 ml) to provide a corresponding 1.0425% solution (LIVER REF 76)

t) Following the similar procedures as described above in example 12A(a) for tannic acid derivatives, formulations of four ingredients for varying concentrations of each of TA-01, TA-02, TA-B, L-Aln-L-Gln, GSSG, CP, Man, Sor, 18α-GA, 18β-GA, KS as presented in TABLE-1 and TABLE 2 and any other four ingredients each selected from the groups tannic acid, amino acid derivative, sugar alcohol and saponin glycoside were prepared/can be prepared.

**B: Amino Acid : L-Ala-L-Gln, GSSG, CP**

a) **Tannic acid TA-01 0.0.00125g and Amino acid: L-Ala-L-Gln** 0.5g, D-**mannitol** 0.5g and **18-beta-GA** 0.04g were dissolved in aqueous alcohol (100 ml) to provide a corresponding 1.04125% solution (DNA REF 26). This solution was administered in several portions to one of the rats group of Example (1a) or (2a) or (3a) or (4a). The administration was carried out over as per example 1(a) or 2(a) or 3(a) or 4 (a). Evaluation of hepato-protective activities were carried out as per Example (1c), hangover and CNS activities were carried out as per Example (2c) and immunological activities were carried out as per Example (3c) and DNA studies as per Example (4c).

b) **Tannic acid TA-01 0.0.00125g** and **Amino acid: L-Ala-L-Gln** 1g, D-**mannitol** 0.5g and **18-beta-GA** 0.1g were dissolved in aqueous alcohol (100 ml) to provide a corresponding 1.60125% solution (DNA REF 21).

c) **Tannic acid TA-01 0.0.00125g** and **Amino acid: L-Ala-L-Gln** 2g, D-**mannitol** 0.75g and **18-beta-GA** 0.1g were dissolved in aqueous alcohol (100 ml) to provide a corresponding 2.85125% solution (DNA REF 23).

d) **Tannic acid TA-01 0.000625g** and **Amino acid: L-Ala-L-Gln** 0.5g, D-**mannitol** 0.5g and **18-beta-GA** 0.04g were dissolved in aqueous alcohol (100 ml) to provide a corresponding 1.04625% solution (LIVER REF 22B)

e) **Tannic acid TA-01 0.0005g** and**Amino acid: L-Ala-L-Gln** 1g, D-**mannitol** 0.5g and **18-beta-GA** 0.04g were dissolved in aqueous alcohol (100 ml) to provide a corresponding 1.5405% solution (LIVER REF 35).

f) **Tannic acid TA-01 0.000625g** and**Amino acid: L-Ala-L-Gln** 2g, D-**mannitol** 0.5g and **18-beta-GA** 0.04g were dissolved in aqueous alcohol (100 ml) to provide a corresponding 2.540625% solution (LIVER REF 33).

g) **Tannic acid TA-01 0.0005g** and**Amino acid: GSSG** 0.1g, **D-mannitol** 0.5g and **18-beta-GA** 0.1g were dissolved in aqueous alcohol (100 ml) to provide a corresponding 0.7005% solution (DNA REF 74)

h) **Tannic acid TA-01 0.00035g** and**Amino acid: GSSG** 0.2g, D-**mannitol** 0.5g and **18-beta-GA** 0.1g were dissolved in aqueous alcohol (100 ml) to provide a corresponding 0.80035% solution (DNA REF 72)

i) **Tannic acid TA-01 0.00035g** and**Amino acid: GSSG** 0.2g, D-**mannitol** 0.5g and **18-beta-GA** 0.1g were dissolved in aqueous alcohol (100 ml) to provide a corresponding 0.80035% solution (LIVER REF 53)

j) **Tannic acid TA-01 0.00035g** and**Amino acid: GSSG** 0.5g, D-**mannitol** 0.5g and **18-beta-GA** 0.04g were dissolved in aqueous alcohol (100 ml) to provide a corresponding 1.04035% solution (LIVER REF 50)

k) **Tannic acid TA-01 0.00035g** and**Amino acid: CP** 0.1g, D-**mannitol** 0.5g and **18-beta-GA** 0.04g were dissolved in aqueous alcohol (100 ml) to provide a corresponding 0.64035% solution (DNA REF 105)

l) **Tannic acid TA-01 0.00125g** and**Amino acid: CP** 0.2g, D-**mannitol** 0.5g and **18-beta-GA** 0.3g were dissolved in aqueous alcohol (100 ml) to provide a corresponding 1.00125% solution (DNA REF 103)

m) Following the similar procedures as described above in example 12B(b) for amino acids, formulations of four ingredients for varying concentrations of each of TA-01, TA-02, TA-B, L-Aln-L-Gln, GSSG, CP, Man, Sor, 18α-GA, 18β-GA, KS as presented in TABLE-1 and TABLE-2 and any other four ingredients each selected from the groups tannic acid, amino acid derivative, sugar alcohol and saponin glycoside were prepared/can be prepared.

**C: Sugar alcohol: D-Mannitol, D-Sorbitol**

a) **Tannic acid TA-01 0.00125g** and**Amino acid: L-Ala-L-Gln** 1g, **D-mannitol** 0.5g and **18-beta-GA** 0.1g were dissolved in aqueous alcohol (100 ml) to provide a corresponding 1.60125% solution (DNA REF 21). This solution was administered in several portions to one of the rats group of Example (1a) or (2a) or (3a) or (4a). The administration was carried out over as per example 1(a) or 2(a) or 3(a) or 4 (a). Evaluation of hepato-protective activities were carried out as per Example (1c), hangover and CNS activities were carried out as per Example (2c) and immunological activities were carried out as per Example (3c) and DNA studies as per Example (4c).

b) **Tannic acid TA-01 0.00125g** and**Amino acid: L-Ala-L-Gln** 2g, D-**mannitol** 0.75g and **18-beta-GA** 0.1g were dissolved in aqueous alcohol (100 ml) to provide a corresponding 2.85125% solution (DNA REF 23).

c) **Tannic acid TA-01 0.000625g** and**Amino acid: L-Ala-L-Gln** 0.5g, D-**mannitol** 0.5g and **18-beta-GA** 0.04g were dissolved in aqueous alcohol (100 ml) to provide a corresponding 1.04625% solution (LIVER REF 22B)

d) **Tannic acid TA-01 0.000625g** and**Amino acid: L-Ala-L-Gln** 0.5g, D-**mannitol** 2.5g and **18-beta-GA** 0.04g were dissolved in aqueous alcohol (100 ml) to provide a corresponding 3.040625% solution (LIVER REF 32)

e) **Tannic acid TA-01 0.00125g** and**Amino acid: L-Ala-L-Gln** 1g, D-**sorbitol** 0.5g and **18-beta-GA** 0.04g were dissolved in aqueous alcohol (100 ml) to provide a corresponding 1.54125% solution (DNA REF 79).

f) **Tannic acid TA-01 0.00035g** and**Amino acid: L-Ala-L-Gln** 0.5g, D-**sorbitol** 1.5g and **18-beta-GA** 0.3g were dissolved in aqueous alcohol (100 ml) to provide a corresponding 2.30035% solution (DNA REF 82).

g) **Tannic acid TA-01 0.000625g** and**Amino acid: L-Ala-L-Gln** 0.5g, D-**sorbitol** 0.5g and **18-beta-GA** 0.04g were dissolved in aqueous alcohol (100 ml) to provide a corresponding 1.040625% solution (LIVER REF 58)

h) **Tannic acid TA-01 0.00035g** and**Amino acid: L-Ala-L-Gln** 1.5g, D-**sorbitol** 1.5g and **18-beta-GA** 0.04g were dissolved in aqueous alcohol (100 ml) to provide a corresponding 3.040625% solution (LIVER REF 62)

i) Following the similar procedures as described above in example 12C(a) for sugar alcohol, formulations of four ingredients for varying concentrations of each of TA-01, TA-02, TA-B, L-Aln-L-Gln, GSSG, CP, Man, Sor, 18α-GA, 18β-GA, KS as presented in TABLE-1 and TABLE-2 and any other four ingredients each selected from the groups tannic acid, amino acid derivative, sugar alcohol and saponin glycoside were prepared/can be prepared.

**D: Saponin glycoside: 18-beta-GA, 18-alpha-GA**

a) **Tannic acid TA-01 0.00125g** and**Amino acid: L-Ala-L-Gln** 1g, D-**mannitol** 0.5g and **18-beta-GA** 0.02g were dissolved in aqueous alcohol (100 ml) to provide a corresponding 1.520125% solution (DNA REF 37). This solution was administered in several portions to one of the rats group of Example (1a) or (2a) or (3a) or (4a). The administration was carried out over as per example 1(a) or 2(a) or 3(a) or 4 (a). Evaluation of hepato-protective activities were carried out as per Example (1c), hangover and CNS activities were carried out as per Example (2c) and immunological activities were carried out as per Example (3c) and DNA studies as per Example (4c).

b) **Tannic acid TA-01 0.00125g** and**Amino acid: L-Ala-L-Gln** 0.5g, D-**mannitol** 0.5g and **18-beta-GA** 0.04g were dissolved in aqueous alcohol (100 ml) to provide a corresponding 1.04125% solution (DNA REF 26).

c) **Tannic acid TA-01 0.00125g** and**Amino acid: L-Ala-L-Gln** 1g, D-**mannitol** 0.5g and **18-beta-GA** 0.1g were dissolved in aqueous alcohol (100 ml) to provide a corresponding 1.60125% solution (DNA REF 21).

d) **Tannic acid TA-01 0.000625g** and**Amino acid: L-Ala-L-Gln** 1g, D-**mannitol** 0.5g and **18-beta-GA** 0.01g were dissolved in aqueous alcohol (100 ml) to provide a corresponding 1.510625% solution (LIVER REF 30)

e) **Tannic acid TA-01 0.000625g** and**Amino acid: L-Ala-L-Gln** 0.5g, D-**mannitol** 0.5g and **18-beta-GA** 0.04g were dissolved in aqueous alcohol (100 ml) to provide a corresponding 1.04625% solution (LIVER REF 22B)

f) **Tannic acid TA-01 0.000625g** and**Amino acid: L-Ala-L-Gln** 0.5g, D-**mannitol** 0.5g and **18-beta-GA** 0.3g were dissolved in aqueous alcohol (100 ml) to provide a corresponding 1.30625% solution (LIVER REF 34)

g) **Tannic acid TA-01 0.00125g** and**Amino acid: L-Ala-L-Gln** 1g, D-**mannitol** 0.5g and **18-alpha-GA** 0.04g were dissolved in aqueous alcohol (100 ml) to provide a corresponding 1.54125% solution (DNA REF 46).

h) **Tannic acid TA-01 0.0075g** and**Amino acid: L-Ala-L-Gln** 0.5g, D-**mannitol** 0.5g and **18-alpha-GA** 0.3g were dissolved in aqueous alcohol (100 ml) to provide a corresponding 1.3075% solution (DNA REF 48).

i) **Tannic acid TA-01 0.000625g** and**Amino acid: L-Ala-L-Gln** 0.5g, D-**mannitol** 0.5g and **18-beta-GA** 0.04g were dissolved in aqueous alcohol (100 ml) to provide a corresponding 1.04625% solution (LIVER REF 22B)

j) **Tannic acid TA-01 0.000625g** and**Amino acid: L-Ala-L-Gln** 0.5g, D-**mannitol** 0.5g and **18-beta-GA** 0.04g were dissolved in aqueous alcohol (100 ml) to provide a corresponding 1.040625% solution (LIVER REF 40)

k) **Tannic acid TA-01 0.000625g** and**Amino acid: L-Ala-L-Gln** 0.5g, D-**mannitol** 0.5g and **18-alpha-GA** 0.3g were dissolved in aqueous alcohol (100 ml) to provide a corresponding 1.300625% solution (LIVER REF 41)

l) Following the similar procedures as described above in example 12D(a) for saponin glycosides, formulations of four ingredients for varying concentrations of each of TA-01, TA-02, TA-B, L-Aln-L-Gln, GSSG, CP, Man, Sor, 18$\alpha$-GA, 18$\beta$-GA, KS as presented in TABLE-1 above and any other four ingredients each selected from the groups tannic acid, amino acid derivative, sugar alcohol and saponin glycoside were prepared/can be prepared.

Administrations to rats and evaluation of activities such as hepato-protective, CNS, immunological and DNA for solutions prepared in Examples 12A(b) to 12A(t), 12B(b) to 12B(m), 12C(b) to 12C(i) and 12D(b) to 12D(1) were carried out as per procedures described in Example 12A(a) (similar as in examples 1-4). The results i.e. % protection and/or % synergism observed are presented in Table-1 and/or Table-2.

The comparative tests results of any single active ingredient, two active ingredients, three active ingredients and four active ingredients comprising different combinations at different amounts show increased protection (%) and synergism (%) of various test parameters in case of three and four active ingredients combination each at appropriate amount are depicted in below Table-1 and Table-2.

## RESULT AND DISCUSSIONS

The major pathways of alcohol metabolism responsible for the generation of reactive oxygen species (ROS) are via alcohol dehydrogenase in the cytosol, microsomal ethanol oxidizing system in the endoplasmic reticulum and aldehyde oxidase in the mitochondria (Lieber CS, 1997 & 1998; Mira L et al., 1995). Exposure to acute or chronic alcohol triggers production of ROS with concomitant drop in the levels or activity of antioxidants that can eliminate ROS. As a consequence, there is an imbalance between pro-oxidants and antioxidants that may lead to the oxidative damage of biomolecules, such as lipids, proteins and DNA, which could lead to genotoxicity, mutagenesis, carcinogenesis, membrane damage, lipid per-oxidation, protein oxidation and fragmentation(Knecht et al., 1990; Tsukamoto and Lu),

Interaction of ROS with DNA can alter normal repair mechanism of damaged DNA which raises the possibility of genetic and epigenetic mutations and potentially increase susceptibility to a number of diseases including cancer and neurodegeneration. Chronic alcohol consumption as well as binging has been shown to result in DNA damage (Wu and Cederbaum, 2003).

Lymphocytes have the similar capability to metabolize ethanol like the other somatic cells. In fact, alcohol and acetaldehyde dehydrogenase (ADH and ALDH) are present in the peripheral lymphocytes (Dyck, 1990). Thus, DNA damage may be induced by the oxidative products of ethanol metabolism and may cause dysfunction of peripheral lymphocytes and the immune system and lead to potential mutations and cancer development as a result of the error-prone repair process (Kido et al., 2006). Decreased rate of DNA damage, as observed in some of the formulated alcohol groups, will also diminish the chances of mutation and its adverse pathophysiology.

It was observed that a significant rise in the levels of different cytokines including IL-12, and MCP-1 during the hangover state and could be well correlated with hangover scale scores (Kim DJ, et al., 2003).Various formulations in Table 1 and Table 2 reported prevent the dysregulation in cytokine pathway and thereby could be able to subside the symptoms of a hangover.

It was also observed that acetylcholine esterase activity increases in hangover. Higher level of brain acetylcholine esterase activity, disrupts cognition and memory functions because it hydrolyzes Ach before it reaches the receptor (Rico EP et al, 2007, Toxicology Letters 174: 25-30; Vinod Tiwari et al, 2009, Behavioural Brain Research 203:296-303). Many formulationsdemonstrated it could prevent increase of acetylcholine esterase activity and thereby able to restore the deficit in cognition and memory functions, one of the important symptoms of a hangover.

In single dose study, significant DNA damage, as judged by the extent of comet tail (Olive Tail Moment score), was observed in only alcohol treated groups. Combination of four ingredients TA (Tanal 1) + DP (L-Alanyl-L-Glutamine)+ Man (D-Mannitol) + b-GA (18-Beta-Glycyrrhizin)with a range from 0.00125% + 0.5% + 0.5% + 0.04% showed highest synergism 122.4%. When DP was replaced with GSSG or G (Oxidized glutathione), the dose had to be lowered drastically as at higher dose of GSSG no synergism was observed, formulation of four ingredients, viz., TA (Tanal 1) + G + Man + b-GA with a range from 0.00035% + 0.1%+0.5%+0.1% to 0.0005%+0.20%+0.75%+0.3% i.e. at lower dose GSSG showed good synergism ranging from 28-40%.

Tannic acid-1(TA-1) 0.00125% when combined with DP (2%), Mannitol (0.75%) and b-GA (0.1%) showed good synergism in all immunological parameters (40.5% in MCP1 and 19.5 % in IL-12) and 10.7% in Achase activity and 17.2 % in DNA. However replacing TA-1 with Tannic acid-2 (TA-2) also showed moderate synergism in all immunological parameters (16.48% in MCP1 and 9.76 % in IL-12) and 13.6% in Achase activity while 11.0% in DNA. Again, replacing TA-1 with Brewtan F (TA-B) showed good synergism in all immunological parameters (38.5% in MCP1 and 18.49 % in IL-12) and 14.9% in Achase, 10.2% in DNA.TA-01 was almost equivalent to TA-B in this combination in MCP-1 (40.5 VS 38.58), IL-12 (11.5 VS 18.49), ACH (10.7 VS 14.97), DNA (17.2 VS10.17) parameter but TA-02 parameter MCP-1 (16.48), IL-12 (9.76), ACH (13.6) had lower values in comparison to TA-01 & TA-B except for DNA(20.6).

Similar trend was observed when Tannic acid-1(TA-1) 0.00125% was combined with DP (1%), Mannitol (0.5%) and b-GA (0.04%); showed good synergism in all immunological parameters (48.2% in MCP1 and 16.7 % in IL-12) and 61.1% in Achase activity and 38.4 % in DNA. Replacing TA-1 with Tannic acid-2 (TA-2) with same dose also showed good synergism in all immunological parameters in the above combination (33.98% in MCP1 and 23.32 % in IL-12) and 33.12% in Achase activity with 19.73% in DNA. Again, replacing TA-1 with Brewtan F (TA-B) with same dose showed good synergism in all immunological parameters (40.92% in MCP1 and 16.21 % in IL-12) and 23.22% in Achase, 30.64% in DNA. TA-01 was almost equivalent to TA-B in this combination in IL-12 (16.7 VS 16.21), slightly higher in MCP-1 (48.2 VS 40.92) and DNA (38.4 VS 30.64), superior in ACH (61.1 VS 23.22) parameters but TA-02 parameter in MCP-1 (33.98), IL-12 (23.32), ACH (33.12), DNA (19.73) had lower values in comparison to TA-01 & TA-B.

Another combination of Tannic acid-1(TA-1) 0.00035% with DP (1%), Mannitol (0.75%) and b-GA (0.3%) showed significant level of synergism in all immunological parameters (31.04% in MCP1 and 29.15 % in IL-12) and 11.61% in Achase activity and 17.7 % in DNA. Replacing TA-1 with Tannic acid-2 (TA-2) in above combination with same dose, relatively lower levels of synergism was observed in immunological parameters (5.93% in MCP1 and 17.05% in IL-12),14.84% in Achase activity, and 6.91% in DNA. However, replacing TA-1 with Brewtan F (TA-B) in above combination with same dose showed significant synergism in all immunological parameters (41.95% in MCP1 and 41.28 % in IL-12) and 9.14% in Achase, 31.37% in DNA. TA-B was better than TA-1 in this combination in IL-12 (29.15 VS 41.28), in MCP-1 (31.04 VS 41.95) and in DNA (17.7 VS 31.3) parameter almost similarvalue in ACH (11.61 VS 9.14), but TA-02 in MCP-1 (5.93), IL-12 (17.5), ACH (14.84), DNA (6.91) parameters had lower values in comparison to TA-01 & TA-B.

In another example; when Tannic acid-1(TA-1) 0.00035% was combined with DP (0.5%), Mannitol (1.5%) and b-GA (0.3%) showed lower level of synergism in all immunological parameters (10.2% in MCP-1 and 19.1 % in IL-12) and 10.8% in Achase activity and 11.1 % in DNA. Replacing b-GA with a-GA, increased synergism drastically in all immunological parameters as 41.03% in MCP-1 and 36.59 in IL-12, similar value in DNA (11.8%) was observed, but no synergism was observed in Achase. A-GA was found to be better than B-GA in this combinations as evident from the values of IL-12 (36.59 VS 19.1), MCP-1 (41.03 VS 10.2) and in ACH ( < 1 VS 10.8) but was similar in DNA (11.1 VS 11.8) parameter.

In another combination when Tannic acid-1(TA-1) 0.00035% was combined with DP (0.5%), Mannitol (1.5%) and b-GA (0.3%); and Mannitol (1.5%) was replaced with Sorbitol (1.5%), synergism level of sorbitol was relatively higher in immunological parameters IL-12 (19.1 VS 28.29), MCP-1 (10.2 VS 25.9) and in DNA parameter (11.1 VS 20.1) in comparison to mannitol, However no synergism was found in Achase in sorbitol, whereas mannitol (Achase 10.8) was not equivalent to that of combination having sorbitol as evident from the values of IL-12 (19.1 VS 28.29), MCP-1 (10.2 VS 25.9) and in DNA (11.1 VS 20.1) parameter but in ACH (10.8 VS < 1).Thus, sorbitol was overall superior to mannitol in this combination.

After 5 alcohol doses of 4 gm/kg, per oral, mild hepatotoxicity was observed as indicated by a rise of serum ALT, AST and ALP. In liver study, moderate degree of protection was observed in formulated alcohol treated groups. Combination of four ingredients Tannic acid-1(TA-1) 0.00035% when combined with DP (0.5%), Mannitol (0.5%) and b-GA (0.04%) showed significant level of synergism in all hepatoprotective parameters (14.89% in ALT, 11.82% in AST and 12.86% in ALP), immunological parameters (58.59% in MCP-1 and 37.51% in IL-12) and CNS parameter (28.57% in Achase activity). However, replacing TA-1 with Tannic acid-2 (TA-2), also showed good synergism in all hepatoprotective parameters (20.77% in ALT, 29.19% in AST and 21.16% in ALP), immunological parameters (17.65% in MCP-1 and 24.08% in IL-12) and CNS parameter (45.10% in Achase activity). Again, replacing TA-1 with Brewtan F (TA-B) showed significant synergism in all hepatoprotective parameters (22.49% in ALT, 28.03% in AST and 27.35% in ALP), immunological parameters (16.22% in MCP-1 and 16.42% in IL-12) while CNS parameter was 51.4% in Achase activity.

When Tannic acid-1(TA-1) 0.000625% was combined with DP (0.5%), Mannitol (0.5%) and b-GA (0.3%); showed good synergism in all hepatoprotective parameters (3.75% in ALT, 40.90% in AST and 19.40% in ALP), immunological parameters (17.14% in MCP-1 and 45.05% in IL-12) and CNS parameter (19.2% in Achase activity). However, replacing TA-1 with Tannic acid-2 (TA-2), also showed synergism in all hepatoprotective parameters (4.72% in ALT, 14.88% in AST and 18.59% in ALP), immunological parameters (9.83% in MCP-1 and 12.4% in IL-12) and CNS parameter (7.3% in Achase activity). Again, replacing TA-1 with Brewtan F (TA-B) showed significant synergism in all hepatoprotective parameters (5.98% in ALT, 26.68% in AST and 34.02% in ALP), immunological parameters (15% in MCP-1 and 19.72% in IL-12) and CNS parameter (10.2% in Achase activity).

Another combination of Tannic acid-1(TA-1) 0.0025% was combined with DP (0.5%), Mannitol (0.5%) and b-GA (0.04%) showed synergism in all hepatoprotective parameters (10.46% in AST and 2.7% in ALP) except in ALT (less than 1%), immunological parameters (33.68% in MCP-1 and 18.27% in IL-12) and CNS parameter (30.17% in Achase activity). Replacing TA-1 with Tannic acid-2 (TA-2) showed good synergism in all hepatoprotective parameters (5.13% in ALT, 17.87% in AST and 4.31% in ALP), immunological parameters (9.54% in MCP-1 and 23.32% in IL-12) while in CNS parameter it was 29.6% (in Achase). Again, replacing TA-1 with Brewtan F (TA-B) showed significant synergism in all hepatoprotective parameters (5.06% in ALT, 10.32% in AST and 16.64% in ALP), immunological parameters (8.69% in MCP-1 and 124.13% in IL-12) and CNS parameter (44.4% in Achase activity).

In another example, when Tannic acid-1(TA-1) 0.000625% was combined with DP (0.5%), Mannitol (0.5%) and b-GA (0.04%) showed significant level of synergism in all hepatoprotective parameters (88.50% in AST, 66.49% in ALP and 91.68 % in ALT), immunological parameters (61.62% in MCP-1 and 62.57% in IL-12) and CNS parameter (13.5% in Achase activity). Replacing b-GA with a-GA, also good synergism was found in all hepatoprotective parameters (18.44% in AST, 23.26% in ALP and 13.87 % in ALT), immunological parameters (12.48% in MCP-1 and 20.17% in IL-12) and CNS parameter (13.2% in Achase activity).

Combination of Tannic acid-1 (TA-1) 0.00035% with DP (0.5%), Mannitol (0.5%) and b-GA (0.04%) showed significant level of synergism in all hepatoprotective parameters, immunological parameters and in CNS parameter as stated earlier but replacing Mannitol (0.5%) with Sorbitol (0.5%) showed good synergism in all hepatoprotective parameters (27.54% in AST, 10.85% in ALP and 8.14 % in ALT), immunological parameters (37.15% in MCP-1 and 27.60% in IL-12) and CNS parameter (53.70% in Achase activity).

From the present study it could be distinctly concluded that, in single dose DNA study groups, combination of TA 1 (0.00125%) with DP (1%), Mannitol (0.5%) and B-GA (0.1 %) was found to be best compared to all other groups in terms of protection as well as synergism *viz*. in immunological parameters, protection and synergism observed for MCP-1 were 42.2 and 72.3% and for IL-12, these were observed as 51.7 and 78.4% respectively. This was also observed for Achase activity (58.1% protection and 47.4 % synergism) and in DNA (106% protection and 122.4 %synergism) too. The second most active group (in terms of protection as well as synergism) was found to be the combination of TA 1 (0.0075%) with DP (0.5%), Mannitol (0.5%) and B-GA (0.1%). the observed protection and synergism for immunological parameters for MCP-1 were 51.5% and 35% and for IL-12, it was 46% and 42%. In case of Achase activity (49.8% protection and 21 % synergism) and in DNA (94.5% protection and 54 %) were noted.

In 5 dose liver study groups, combination of TA 1 (0.000625%) with DP (0.5%), Mannitol (0.5%) and B-GA (0.04%) was found to be the best compared to all other groups in terms of protection as well as synergism viz. in hepatoprotection, ALT (63.86% and 88.50%), AST (20.61% and 66.49%), ALP (40.06% and 91.68%), in immunological parameters, protection and synergism observed for MCP-1 were 29.60% and 61.62% and for IL-12, it was 31.59% and 62.57%. In CNS parameter, for Achase activity protection was 13.5% and synergism 13.5 %. The second most active group (in terms of both protection as well as synergism) was found to be the combination TA 1 (0.00125%) with DP (1%), Mannitol (0.5%) and B-GA (0.04%). In hepato-protection, ALT (67.53% and 32.16%), AST (44.32 % and 76.36%), ALP (70.19% and 28.55%), in immunological parameters, protection and synergism observed for MCP-1 were 45.30% and 27.64% and for IL-12 it was 47.31% and 17.63%. In CNS parameter, for Achase activity protection was 29.7% while observed synergism was 13.9 %.

Thus, it may be concluded that above mentioned formulations, when consumed along with alcohol, may reduce hangover related symptoms compared to drinking alcohol alone and in long run it may able to reduce the chance of alcohol related disorders including hepatotoxicity and mutagenesis.

**STABILITY STUDY**

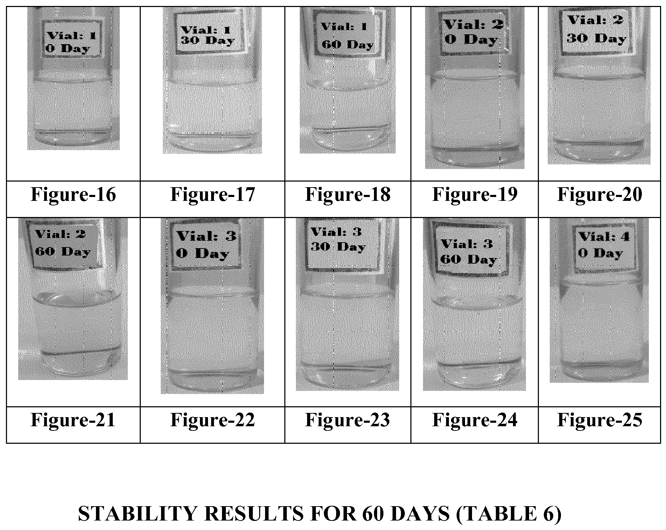

The stability of the composition of the present invention are studied and evaluated. The stability study data obtained after evaluation are presented in below Table 3, Table 4 and Figure 1 to Figure 15 for 45 days stability data and in Table-5, Table-6 and Figure 16 to Figure 33 for 60 days data. Further the stability study protocols, conditions and observations are described below.

Grading:

| +++++++++ | VY | Very strong yellow |
|---|---|---|
| ++++++++ | VY | Very strong yellow |
| +++++++ | StY | Strong yellow |
| ++++++ | StY | Strong yellow |
| ++++++ | MY | Moderate yellow |
| +++++ | MY | Moderate yellow |
| ++++ | FY | Faint yellow |
| +++ | FY | Faint yellow |
| ++ | SY | Slight yellow |
| + | SY | Slight yellow |
| Nil | C | Colourless |

Equipment Name: Thermolab Stability Chamber
Condition: 40 °C / 75% humidity

**STABILITY DATA [45 DAYS, TABLE-3, TABLE-4, FIGURES 1 TO 15]**

**Stability Testing Protocol-**

**Blank :** Take 20 ml aqueous alcohol, Prepare vial (Vial No: **15**)
**0.000625%:**

    A. Take 10ml aq. alcohol add 5 mg TA-01 stock solution (0.5mg/ml)
    B. Take 79ml aq. Alcohol add sol. A 1ml
    C. Take 20ml sol. B prepare vial (Vial no: 25)
    D. Sol. B 60 ml add 450mg MAN and add 24mg 18β-GA
    E. Sol. D 20 ml add 20mg KS prepare vial (vial no: 40)
    F. Sol. D 40 ml add 400mg (L-Ala-L-Gln)
    G. Sol. F 20 ml prepare vial (vial no: 45)
    H. Sol. F 20ml add 20mg KS prepare vial (vial no: 44)

**\*Note (for below table) :**

TA = Tannic Acid /Tannal 01(TA-01),
MAN = Mannitol (Man),
GA = Glycyrrhizic Acid (18β-GA),
KS = Potassium Sorbate (KS),
DP = Dipeptide (L-Ala-L-Gln)

**Table-3: Formulations evaluated for 45 days stability study & Stability Report**

| Vial No. | TA-01 | (L-Ala-L-Gln) | MAN | 18β-GA | KS | Aq Alcohol | 0 days | | 30 days | | 45 days | |
|---|---|---|---|---|---|---|---|---|---|---|---|---|
| 15 | | | | | | 20 Ml | Nil | C | Nil | C | Nil | C |
| 45 | 0.000625 | 1.0 | 0.75 | 0.04 | | 20 Ml | Nil | C | Nil | C | + | SY |
| 44 | 0.000625 | 1.0 | 0.75 | 0.04 | 0.1 | 20 Ml | Nil | C | Nil | C | +<br>+ | SY |
| 40 | 0.000625 | | 0.75 | 0.04 | 0.1 | 20 Ml | Nil | C | Nil | C | +<br>+<br>+ | FY |
| 25 | 0.000625 | | | | | 20 Ml | Nil | C | Nil | C | +<br>+<br>+<br>+ | FY |

**Table-4: 45 days Stability Report**

| STABILITY REPORT - 45 DAYS (Condition : 40°C/75% H) | | | | |
|---|---|---|---|---|
| Vial No. | T (Concentration) | Comparative Intermittency - Yellow Colour | | |
| | | 0 Day | 30 Day | 45 Day |
| 15 | Blank | **Nil** | **Nil** | **Nil** |
| | | Figure-1 | Figure-2 | Figure-3 |
| 45 | | **Nil** | Nil | + |
| | | Figure-4 | Figure-5 | Figure-6 |
| 44 | | **Nil** | Nil | ++ |
| | | Figure-7 | Figure-8 | Figure-9 |
| 40 | | **Nil** | Nil | +++ |
| | | Figure-10 | Figure-11 | Figure-12 |
| 25 | | **Nil** | Nil | ++++ |
| | | Figure-13 | Figure-14 | Figure-15 |

**STABILITY DATA [60 DAYS, TABLE-5, TABLE-6, FIGURES 16 TO 33] Stability Testing Protocol-**

**Required Glassware:** Volumetric flask (100ml-2 50ml-2), Glass Beaker (100ml-1, 50ml-2), Measuring cyclinder (10 mL - 4, 25 mL -2, 50 mL - 2), Micropipette (10 mL-1, 1 mL - 1), Parafilm - 1, Glass vial (25ml) - 6

**Blank :** Take 10 ml aqueous alcohol, Prepare vial (Vial: BLANK)

**0.015%:**

A. Take 50 ml aqueous alcohol add 7.5 mg Tannic acid (TA-01)

B. 10 ml of Sol. A, prepare vial (Vial: 0.015%)

C. Take remaining 40 ml sol. A, add 480 mg Mannitol and 40 mg Glycyrrhizin (18β-GA)

D. Sol. C 10ml prepare vial (Vial: 0.015% (TA-01)+1.2% (D-Mannitol) + 0.1% (18β Glycyrrhizin without KS)

E. Sol. C 10 ml add 10 mg of Potassium sorbate prepare vial (Vial: 0.015% (TA-01) +1.2% (D-Mannitol + 0.1% (18β-Glycyrrhizin) + 0.1% (Potassium sorbate)

F. Sol. C 10 ml add Dipeptide (L-Ala-L-Gln) 100 mg prepare vial (Vial: 0.015% (TA-01)+ 1.0 % (L-Ala-L-Gln) + 1.2% (D-Mannitol) + 0.1% (18β-Glycyrrhizin Without KS)

G. Sol. C 10 ml add 100 mg Dipeptide (L-Ala-L-Gln) and add 10 mg Potassium sorbate prepare vial (Vial: 0.015% (TA-01) + 1.2% (D-Mannitol) + 0.1% (18β-Glycyrrhizin) + 1.0% (L-Ala-L-Gln)

**Table-5: Formulations evaluated for 60 days stability study & Stability Report**

| Vial No. | Formulation composition | RESULT | | |
|---|---|---|---|---|
| | | **0 Day** | **30 Day** | **60 Day** |
| 1 | Blank | Colourless | Colourless | Colourless |
| 2 | 0.015 (TA-01) | Colourless | Yellow | Yellow |
| 3 | 0.015 (TA-01) + 1.2(MAN) + 0.1 (18β-GA) + 0.1 (KS) | Colourless | Colourless | Slight yellow |
| 4 | 0.015 (TA-01) +1.0 (L-Ala-L-Gln) + 1.2(MAN) + 0.1 (18β-GA) + 0.1 (KS) | Colourless | Colourless | Very slight yellow |
| 5 | 0.015 (TA-01) + 1.2(MAN) + 0.1 (18β-GA) | Colourless | Colourless | Very slight yellow |
| 6 | 0.015 (TA-01) + 1.0 (L-Ala-L-Gln) + 1.2(MAN) + 0.1 (18β-GA) | Colourless | Colourless | Very slight yellow |

STABILITY REPORT (Condition : 32°C ± 5°C)

**Table-6: 60 days stability report**

| Vial No. | Formulation composition | Comparative Intermittency - Yellow Colour (1-10) | | |
|---|---|---|---|---|
| | | **0 Day** | **30 Day** | **60 Day** |
| 1 | Blank | **Nil** | **Nil** | **Nil** |
| | | Figure-16 | Figure-17 | Figure-18 |
| 2 | 0.015 (TA-01) | **Nil** | +++++++ | +++++++ +++ |
| | | Figure-19 | Figure-20 | Figure-21 |
| 3 | 0.015 (TA-01) + 1.2(MAN) + 0.1 (18β-GA) + 0.1 (KS) | **Nil** | **Nil** | ++++ |
| | | Figure-22 | Figure-23 | Figure-24 |
| 4 | 0.015 (TA-01) + 1.0 (L-Ala-L-Gln) + 1.2(MAN) + 0.1 (18β-GA) + 0.1 (KS) | **Nil** | **Nil** | +++ |
| | | Figure-25 | Figure-26 | Figure-27 |

STABILITY REPORT (Condition : 32°C ± 5°C)

(continued)

| STABILITY REPORT (Condition : 32°C $\pm$ 5°C) | | | | |
|---|---|---|---|---|
| Vial No. | Formulation composition | Comparative Intermittency - Yellow Colour (1-10) | | |
| | | 0 Day | 30 Day | 60 Day |
| 5 | 0.015 (TA-01) + 1.2(MAN) + 0.1 (18β-GA) | Nil | Nil | ++ |
| | | Figure-28 | Figure-29 | Figure-30 |
| 6 | 0.015 (TA-01) + 1.0 (L-Ala-L-Gln) + 1.2(MAN) + 0.1 (18β-GA) | Nil | Nil | + |
| | | Figure-31 | Figure-32 | Figure-33 |

**Stability Observations**

**60 day:** When various formulations were stored at 32°C $\pm$ 5°C for 0 day, 30 day and 60 days. Certain conclusion could be drawn from vials 1-5 with concentration of TA-01 (0.015%), Mannitol (1.2%), L-Ala-L-Gln (1.0%), 18β-GA (0.1%) and potassium sorbate (0.1 %) respectively. The absence of potassium sorbate improves the stability of formulation as TA-01 tends to degrade in more in neutral or basic condition in comparison to acidic condition and L-Ala-L-Gln has some positive role towards stability. At higher concentration of TA-01, the stability could be ranked in increasing order i.e. 6>5>4>3>2.

**45 day:** When various formulations were stored at accelerated condition 40°C / 75% (humidity) for 0 day, 30 day and 45 days. Certain conclusion could be drawn from vials 15, 45, 44, 40 and 25 with concentration of TA-01 (0.000625%), Mannitol (0.75 %), L-Ala-L-Gln (1.0%), 18β-GA(0.04%) and potassium sorbate (0.1%) respectively. Similar trend was observed as in 60 day stability studies with lower concentration of TA-01 *i.e.* the absence of potassium sorbate improves the stability of formulation and L-Ala-L-Glnhas positive impact on stability of formulation. At lower concentration of TA-01, the stability could be ranked in increasing order i.e. 45>44> 40> 25.

**Technical Advancements and Economic Significance:**

The present invention provides the following technical advancements over prior art: An alcoholic beverage is provided which provides DNA protection. The alcoholic beverage also provides protection against pancreatic damage. The alcoholic beverage also provides protection against hepatic stress.

**Definitions:**

As used in the present invention, the following words and phrases are generally intended to have the meaning as set forth below, except to the extent that the context in which they are used to indicate otherwise.

**Active ingredient:** The term active ingredient means a composite mixture derived from saponin glycoside, amino acid derived molecule and sugar or sugar alcohol alleviating hepatic stress, oxidative stress, modulating immunology parameters, DNA damage, and pancreas damage induced by xenobiotic such as alcohol.

**Stress:** Stress is a condition, which is triggered by generation of reactive oxygen species ("ROS") or reactive nitrogen species ("RNS") due to induced injury caused by xenobiotic like alcohol. This ultimately leads to imbalance and could cause hepatic stress or oxidative stress or CNS stress or all of them.

**Amino-Acid-Derived-Molecules:** A molecule consisting of one or more amino-acid molecules, such as an amino-acid monomer, dipeptide, tripeptide, oligopeptide, polypeptide, protein, or a peptide hydrolysate or a peptide residue thereof.

**Saponin Glycoside**:areclass of compound which are obtained from the plant glycosidespossessing a distinct property of forming soapy lather in water.Saponins on hydrolysis give sugars (glucose, galactose, rhamnose or xylose, etc.) and aglycones (sapogenin).It also Includes components such as Glycyrrhizin, including a derivative or its isomer, such as 18α-Glycyrrhizin, 18β-Glycyrrhizin, 18α-mono ammonium glycyrrhizinate, 18β-mono ammonium glycyrrhizinate, or a combination thereof.

**Sugar:** Compounds such as D-Maltodextrin, L-Maltodextrin, D-Maltose, L-Maltose, D-Dextrose, L-Dextrose, D-Glucose, L-Glucose, D-Trehalose, L-Trehalose, D-Sucrose, L-Sucrose, D-Lactose, L-Lactose, Hydrogenated Starch Hydrolysates, D-Fructose, D-Galactose, or a mixture thereof.

**Sugar alcohol:** Compounds such as, D-Glycerol, L-Glycerol, D-Mannitol, L-Mannitol, D-erythritol, L-erythritol, D-xylitol, or L-xylitol, L-Maltitol, D-Maltitol, L-Sorbitol, D-Sorbitol, L-Lactitol, D-Lactitol, L-Isomalt, D-Isomalt or a mixture thereof. **Hydrolyzable tannin or pyrogallol-type tannin compounds:** Includes compounds such as Tannins, Tannic acid, epigallitannins, gallotannins, Ellagitannins, condensed tannin, complex tannin product or a mixture thereof.

**Alcoholism:** A chronic and often progressive disease that includes problems controlling drinking, being preoccupied with alcohol, continuing to use alcohol even when it causes problems, having to drink more to get the same effect (physical dependence), or having withdrawal symptoms when one rapidly decreases or stops drinking. (Definition adapted from Mayo Clinic Staff Definition. ("Diseases and Conditions: Alcoholism (Definition)." Mayo Clinic. (2014, December 5). Retrieved May 11, 2015, from http://www.mayoclinic.org/diseases-conditions/alcoholism/basics/definition/con-20020866).

**Alcoholic:** An individual afflicted with alcoholism.

**Moderate drink:** Consumption of at least one but less than three alcoholic beverages a day for men, and at least one but less than two alcoholic beverages a day for women.

**Binge drinking:** Consumption by an individual of sufficient alcohol to raise the individual's blood alcohol content above 0.08%, which, for most adults, would be reached by consuming five drinks for men, or four drinks for women, during a two-hour period.

**Hepato protection:** The ability to reduce stress and prevent damage to the liver

**Synergistic Composition:** An interaction of active ingredients which, when administered simultaneously, produce an overall biological effect greater than when administered individually, i.e., having biological effects greater than the sum of individual biological effects of any of them

**DNA protection:** The ability of alcohol to provide protection to the DNA from being damaged after consumption of the same.

**Veisalgia (Hangover):** the medical term for a hangover, usually caused by Binge Drinking of alcohol. Symptoms often include headache, irritability, nausea, fatigue, and lethargy.

**CNS Protection:** the ability to reduce stress and prevent damage to the central nervous system, including the brain and the spinal cord.

Peptide or polypeptide residue like Dipeptide, Tripeptide, Oligopeptide Glycyrrhizin derivative Includes components such as Glycyrrhizin, including a derivative or its isomer, such as 18$\alpha$-Glycyrrhizin, 18$\beta$-Glycyrrhizin, 18$\alpha$-mono ammonium glycyrrhizinate, 18$\beta$-mono ammonium glycyrrhizinate, or a combination thereof.

**Tannic acid:** Tannic acid refers to the small group of polyphenols calledhydrolysable (gallo)tannins obtained from Aleppo orChinese gallnuts, or Sumac leaves. The main building block glucoseis combined with gallic acid and hydrolysable (gallo)tannins obtained from Tara pods building block quinic acidis combined with gallic acid. The botanical source & method of process results in various number of gallic acid combined to glucose molecule. Thus, this results in mixture of various1,2,3,4,6-tetragalloyl glucose 1,2,3,4,6-Pentagalloyl glucose,1,2,3,4,6-Hexagalloyl glucose, 1,2,3,4,6-Heptagalloyl glucose, 1,2,3,4,6-Octagalloyl glucose, 1,2,3,4,6-nonagalloyl glucose 1,2,3,4,6-decagalloyl glucose,1,2,3,4,6-undecagalloyl glucose,1,2,3,4,6-dodecagalloyl glucose. As used in the present invention, the following words and phrases are generally intended to have the meaning as set forth below, except to the extent that the context in which they are used to indicate otherwise.

**Active sugar alcohol ingredient:** A sugar alcohol such as D-Mannitol, D-Erythritol or L-Erythritol.

**Active saponin glycoside ingredient:** A saponin glycoside such as 18$\beta$-GA, 18$\alpha$-GA, or a mixture with equal parts 18$\beta$-GA and 18$\alpha$-GA.

**Epigallitannins:** When a hydrolyzable tannin molecule has carbohydrate (usually D-glucose but also cyclitols like quinic or shikimic acids). The hydroxyl groups of the carbohydrate are partially or totally esterified with phenolic groups with ellagic acid in ellagitannins.

**Gallotannins** are all those hydrolysable tannins (polymers) in which galloyl units or their meta-depsidic derivatives are bound to diverse Polyol carbohydrate such as glucose, catechin, or triterpenoid units.

**Ellagitannins** are those hydrolysable tannins in which at least two galloyl units are oxidativeC-C coupled (*meta* or *para*depside bonds) to each other, and do not contain a glycosidically linked catechin unit.

**Complex tannins** are tannins in which a catechin unit is bound glycosidically to a gallotannin or an ellagitannin unit.

**Condensed tannins** are oligomeric and polymeric proanthocyanidins formed by linkage of C-4 of one catechin with C-8 or C-6 of the next monomeric catechin.

**Hydrolyzable tannin:** Tannins can be fractionated hydrolytically into their components, for example by the treatment with hot water or with tannases.

**Tannins:** Tannins are polyphenolic secondary metabolites of higherplants, and are either galloyl esters and their derivatives,in which galloyl moieties or their derivatives are bound toa variety of Polyol carbohydrate such as D-glucose, catechin and triterpenoid cores (gallotannins,ellagitannins and complex tannins), or they are oligomeric and polymeric proanthocyanidins that can possessdifferent interflavanyl coupling and substitution patterns(condensed tannins).

**Tanal 01:** Tanal 01 refers to Tannic acid, which are small group of polyphenols called hydrolysable (gallo) tannins obtained from Rhussemialata. The main building block glucose is combined with gallic acid. It is a mixture of various 1,2,3,4,6-tetragalloyl glucose 1,2,3,4,6-Pentagalloyl glucose, 1,2,3,4,6-Hexagalloyl glucose, 1,2,3,4,6-Heptagalloyl glucose, 1,2,3,4,6-Octagalloyl glucose, 1,2,3,4,6-nonagalloyl glucose 1,2,3,4,6-decagalloyl glucose, 1,2,3,4,6-undecagalloyl glucose, 1,2,3,4,6-dodecagalloyl glucose.

**Tanal 02:** Tanal 02 refers to Tannic acid, which are small group of polyphenols called hydrolysable (gallo) tannins obtained from Quercusinfectoria. The main building block glucose is combined with gallic acid. It is a mixture of various 1,2,3,4,6-tetragalloyl glucose 1,2,3,4,6-Pentagalloyl glucose, 1,2,3,4,6-Hexagalloyl glucose, 1,2,3,4,6-Heptagalloyl glucose, 1,2,3,4,6-Octagalloyl glucose, 1,2,3,4,6-nonagalloyl glucose 1,2,3,4,6-decagalloyl glucose, 1,2,3,4,6-undecagalloyl glucose, 1,2,3,4,6-dodecagalloyl glucose.

**Brewtan:** Brewtan refers to Tannic acid, which are small group of polyphenols called hydrolysable (gallo) tannins obtained from Aleppo or Chinese gallnuts, or Sumac leaves. The main building block glucose is combined with gallic acid. It is a mixture of various 1,2,3,4,6-tetragalloyl glucose 1,2,3,4,6-Pentagalloyl glucose, 1,2,3,4,6-Hexagalloyl glucose, 1,2,3,4,6-Heptagalloyl glucose, 1,2,3,4,6-Octagalloyl glucose, 1,2,3,4,6-nonagalloyl glucose 1,2,3,4,6-decagalloyl glucose, 1,2,3,4,6-undecagalloyl glucose, 1,2,3,4,6-dodecagalloyl glucose.

**Peptide:** Dipeptide,Tripeptide& Tetrapeptide

- X-X dipeptide (with X designating an amino acid and "-" indicates peptide bond).

This nomenclature is continued by tripeptide X-X-X (three amino acids, two peptide bonds,), tetrapeptide (X-X-X-X),

**Oligopeptide:** An oligopeptide, also called as peptide, a protein fragment or molecule that usually consists of two and less than 25 amino acid residues linked in a polypeptide chain. These amino acid monomers are linked by peptide (amide) bond and can include dipeptides, tripeptides, tetrapeptides, and pentapeptides.

Peptides are biologically occurring short chains of amino acid monomers linked by peptide (amide) bonds.
A polypeptide is a long, continuous, and unbranched peptide chain. Hence, peptides fall under the broad chemical classes of biological oligomers and polymers,
**[0287]** Peptide fragments refer to fragments of proteins that are used to identify or quantify the source protein. Often these are the products of enzymatic degradation performed in the laboratory on a controlled source protein.

| | Tannin | Amino Acid | Sug. Alco. | Sapo Gly | | MCP-1 | MCP-1 | IL12 | IL12 | Achase | Achase | DNA | DNA |
| Gr. | TA-01 | L-Ala-L-Gla | Man | 18β-GA | KS | Prot | SYN | Prot | SYN | Prot | SYN | Prot | SYN |
|---|---|---|---|---|---|---|---|---|---|---|---|---|---|
| 1 | 0.00035 | | | | | 1.3 | | 2.4 | | 1.1 | | 2.27 | |
| 2 | 0.0005 | | | | | 2.4 | | 2.8 | | 0.9 | | 3.1 | |
| 3 | 0.000625 | | | | | 4.1 | | 4.0 | | 3.2 | | 7.6 | |
| 4 | 0.00125 | | | | | 8.9 | | 11.7 | | 9.7 | | 17.2 | |
| 5 | 0.0075 | | | | | 28.3 | | 22.0 | | 22.1 | | 40.9 | |
| 6 | 0.025 | | | | | 47.9 | | 41.9 | | 36.9 | | 76.5 | |
| 7 | | 0.5 | | | | 3.5 | | 3.7 | | 3.2 | | 1.9 | |
| 8 | | 1 | | | | 9.3 | | 10.5 | | 13.8 | | 11.9 | |
| 9 | | 1.5 | | | | 15.5 | | 18.3 | | 22.1 | | 18.9 | |
| 10 | | 2 | | | | 22.5 | | 26.3 | | 32.7 | | 28.3 | |
| 11 | | | 0.5 | | | 2.5 | | 4.2 | | 4.1 | | 17.6 | |
| 12 | | | 0.75 | | | 4.3 | | 6.3 | | 4.3 | | 27.0 | |
| 13 | | | 1 | | | 8.5 | | 8.5 | | 12.8 | | 30.4 | |
| 14 | | | 1.5 | | | 13.2 | | 15.6 | | 33.2 | | 42.3 | |
| 15 | | | 2.5 | | | 22.7 | | 21.4 | | 45.1 | | 52.2 | |
| 16 | | | | 0.02 | | 0.5 | | 0.5 | | 0.5 | | 0.5 | |
| 17 | | | | 0.04 | | 1.0 | | 1.0 | | 1.0 | | 0.4 | |
| 18 | | | | 0.1 | | 3.8 | | 2.6 | | 11.8 | | 1.0 | |
| 19 | | | | 0.3 | | 14.0 | | 7.5 | | 17.2 | | 6.1 | |
| 20 | 0.0075 | 0.5 | 0.5 | 0.1 | | 51.5 | 35 | 46.0 | 42 | 49.8 | 21 | 94.5 | 54 |
| 21 | 0.00125 | 1 | 0.5 | 0.1 | | 42.2 | 72.3 | 51.7 | 78.4 | 58.1 | 47.4 | 106 | 122.4 |
| 22 | 0.00125 | 1 | 2.5 | 0.1 | | 51.0 | 14.2 | 50.8 | 9.9 | 55.8 | | 96.5 | 17.3 |
| 23 | 0.00125 | 2 | 0.75 | 0.1 | | 55.5 | 40.5 | 52.3 | 11.5 | 58.1 | 10.7 | 86.1 | 17.2 |
| 24 | 0.025 | 0.5 | 0.5 | 0.1 | | 67.7 | 17.4 | 58.2 | 11.0 | 49.8 | | 111.8 | 15.3 |

TABLE-1 (1 DAY DATA) - ALL VALUES ARE IN %

EP 3 257 513 A1

(continued)

EP 3 257 513 A1

| | Tannin | Amino Acid | Sug. Alco. | Sapo Gly | | MCP-1 | MCP-1 | IL12 | IL12 | Achase | Achase | DNA | DNA |
|---|---|---|---|---|---|---|---|---|---|---|---|---|---|
| | | | | | | | | | | | | | |
| | | | | | | TABLE-1 (1 DAY DATA) - ALL VALUES ARE IN % | | | | | | | |
| Gr. | TA-01 | L-Ala-L-Gla | Man | 18β-GA | KS | Prot | SYN | Prot | SYN | Prot | SYN | Prot | SYN |
| 25 | 0.00125 | 1 | 0.5 | 0.04 | | 32.2 | 48.2 | 55.0 | 16.7 | 46.1 | 61.1 | 65.2 | 38.4 |
| 26 | 0.00125 | 0.5 | 0.5 | 0.04 | | 25.2 | 58.3 | 28.0 | 36.1 | 26.3 | 45.9 | 56.1 | 51.2 |
| 27 | 0.00125 | 1 | 0 | 0.1 | | 32.6 | 48.2 | 30.3 | 22.2 | 45.2 | 27.9 | 49.7 | 65.2 |
| 28 | 0.00125 | 1 | 0.5 | 0 | | 23.5 | 13.3 | 20.6 | | 32.7 | 18.5 | 37.8 | |
| 29 | 0.00125 | 1 | 0 | 0 | | 21.6 | 18.8 | 18.7 | | 28.6 | 21.6 | 32.1 | 10.4 |
| 30 | 0.00125 | 0 | 0.5 | 0 | | 12.9 | 13.4 | 12.5 | | 18.0 | 30.2 | 27.4 | |
| 31 | 0.00125 | 0 | 0 | 0.1 | | 15.9 | 25.2 | 17.0 | 19.2 | 22.1 | 2.9 | 24.8 | 36.5 |
| 32 | 0.00125 | 0 | 0.5 | 0.1 | | 19.0 | 25.0 | 25.4 | 37.2 | 32.7 | 27.8 | 49 | 36.9 |
| 33 | 0 | 1 | 0.5 | 0.1 | | 20.4 | 30.6 | 23.5 | 35.8 | 37.5 | 26.2 | 39.45946 | 29.5 |
| 34 | 0 | 1 | 0 | 0.1 | | 14.2 | 8.3 | 15.2 | 15.7 | 28.4 | 10.9 | 14.7027 | 14.2 |
| 35 | 0 | 0 | 0.5 | 0.1 | | 8.5 | 34.4 | 8.4 | 23.8 | 15.3 | | 25.94595 | 39.7 |
| 36 | 0.000625 | 1 | 0.75 | 0.3 | | 33.5 | 5.8 | 35.4 | 25.2 | 45.2 | 17.3 | 63.7 | 20.4 |
| 37 | 0.00125 | 1 | 0.5 | 0.02 | | 28.3 | 33.3 | 32.0 | 19.0 | 35.0 | 24.6 | 55 | 15.6 |
| 38 | 0.00035 | 0.5 | 1.5 | 0.3 | | 35.3 | 10.2 | 34.8 | 19.1 | 60.6 | 10.8 | 58.38 | 11.1 |
| 39 | 0.00035 | 0.5 | 2.5 | 0.3 | | 47.5 | 14.6 | 42.5 | 21.4 | 74.1 | 11.3 | 68.97 | 10.5 |
| 40 | 0.00035 | 1 | 1 | 0.3 | | 35.70 | 7.85 | 33.59 | 16.24 | 45.13 | 0.51 | 56.76 | 12.08 |
| 41 | 0.00035 | 1 | 0.75 | 0.3 | | 37.87 | 31.04 | 34.48 | 29.15 | 40.63 | 11.61 | 55.60 | 17.70 |
| | TA-01 | L-Ala-L-Gla | Man | 18α-GA | KS | MCP-Prot | SYN | IL12-Prot | SYN | AcH-Prot | SYN | DNA-Prot | SYN |
| 42 | | | | 0.04 | | 1.5 | | 0.9 | | 0.9 | | 0.95 | |
| 43 | | | | 0.1 | | 3.7 | | 3.4 | | 11.5 | | 2.78 | |
| 44 | | | | 0.3 | | 15.1 | | 8.1 | | 19.4 | | 6.19 | |
| 45 | 0.00125 | 2 | 0.75 | 0.1 | | 46.2 | 17.3 | 45.5 | | 47.0 | | 90.8 | 20.6 |
| 46 | 0.00125 | 1 | 0.5 | 0.04 | | 27.8 | 25.2 | 28.0 | 2.7 | 35.0 | 22.9 | 68.3 | 43.2 |

(continued)

| | TA-01 | L-Ala-L-Gla | Man | 18α-GA | KS | MCP-Prot | SYN | IL12-Prot | SYN | AcH-Prot | SYN | DNA-Prot | SYN |
|---|---|---|---|---|---|---|---|---|---|---|---|---|---|
| 47 | 0.00125 | 1 | 2.5 | 0.3 | | 56.8 | 1.3 | 62.4 | 20.6 | 77.0 | | 79.3 | |
| 48 | 0.0075 | 0.5 | 0.5 | 0.3 | | 42.7 | | 46.0 | 21.2 | 58.1 | 19.0 | 73.3 | 10.1 |
| 49 | 0.0005 | 1 | 0.75 | 0.3 | | 27.3 | | 32.8 | 18.3 | 45.2 | 17.6 | 53.2 | 10.4 |
| 50 | 0.00035 | 0.5 | 1.5 | 0.3 | | 46.68 | 41.03 | 40.70 | 36.59 | 50.12 | | 58.9 | 11.8 |
| 51 | 0.00035 | 0.5 | 2.5 | 0.3 | | 50.23 | 17.91 | 42.18 | 18.50 | 57.93 | | 70.6 | 12.8 |
| 52 | 0.00035 | 1 | 0.75 | 0.3 | | 39.59 | 19.60 | 33.37 | 11.99 | 47.16 | | 54.08 | 14.19 |
| | TA-01 | L-Ala-L-Gla | Man | 18βGA | KS | MCP-Prot | SYN | IL12-Prot | SYN | AcH-Prot | SYN | DNA-Prot | SYN |
| 53 | | | | | 0.1 | 0 | | 0 | | 0.0 | | 0.24 | |
| 54 | 0.00125 | 0 | 0.5 | 0.1 | 0.1 | 21.2 | 39.3 | 26.1 | 41.1 | 33.2 | 29.7 | 52.4 | 45.5 |
| 55 | 0.00125 | 1 | 0.5 | 0.1 | 0.1 | 30.9 | 26.1 | 36.0 | 24.1 | 43.8 | 11.2 | 64.4 | 34.7 |
| 56 | .. | 0.1 | .. | .. | | 3.2 | | 2.5 | | 2.68 | | 2.38 | |
| 57 | | 0.2 | | | | 4.54 | | 3.97 | | 2.92 | | 5.2 | |
| 58 | | 0.50 | | | | 9.8 | | 5.5 | | 5.5 | | 22.3 | |
| 59 | | 1.00 | | | | 16.4 | | 8.7 | | 11.5 | | 25.9 | |
| 60 | | 1.50 | | | | 18.2 | | 9.3 | | 20.3 | | 35.1 | |
| 61 | | 2.00 | | | | 20.8 | | 13.8 | | 28.6 | | 33.9 | |
| 62 | 0.00125 | 1 | 0.5 | 0.04 | | 37.0 | 28.3 | 35.4 | 38.4 | 35.0 | 33.2 | 46.26 | |
| 63 | 0.0005 | 0.5 | 2.5 | 0.3 | | 44.1 | | 44.5 | 19.7 | 47.5 | | 50.14 | |
| 64 | 0.00125 | 2 | 0.75 | 0.1 | | 39.2 | 10.8 | 35.1 | 0.1 | 45.2 | | 39.87 | |
| 65 | 0.0005 | 1 | 0.75 | 0.3 | | 39.6 | 6.7 | 34.5 | 36.3 | 43.3 | 27.8 | 45.54 | |
| 66 | 0.0075 | 0.5 | 0.5 | 0.3 | | 38.8 | | 38.3 | | 43.4 | | 43.38 | |
| 67 | 0.0005 | 0.5 | 1.5 | 0.1 | | 37.4 | 28.1 | 34.9 | 31.6 | 58.1 | 13.0 | 54.82 | |
| 68 | 0.0005 | 1.5 | 1 | 0.1 | | 40.0 | 21.7 | 28.2 | 21.7 | 62.2 | 35.8 | 56.20 | |
| 69 | 0.0075 | 1 | 0.75 | 0.3 | | 45.3 | | 51.7 | 16.3 | 64.5 | 17.1 | 64.11 | |
| 70 | 0.00125 | 0.2 | 0.5 | 0.3 | | 31.35 | 4.71 | 30.04 | 9.74 | 32.73 | | 37.30 | |

(continued)

| | TA-01 | L-Ala-L-Gla | Man | 18βGA | KS | MCP-Prot | SYN | IL12-Prot | SYN | AcH-Prot | SYN | DNA-Prot | SYN |
|---|---|---|---|---|---|---|---|---|---|---|---|---|---|
| 71 | 0.00035 | 0.2 | 0.75 | 0.3 | | 28.26 | 17.07 | 25.82 | 27.98 | 29.57 | 15.85 | 51.88 | 27.96 |
| 72 | 0.00035 | 0.2 | 0.5 | 0.1 | | 17.39 | 43.25 | 18.78 | 42.59 | 22.30 | 11.95 | 35.68 | 37.03 |
| 73 | 0.000625 | 0.1 | 0.5 | 0.1 | | 21.17 | 55.36 | 19.38 | 45.77 | 26.40 | 21.20 | 40.07 | 40.37 |
| 74 | 0.0005 | 0.1 | 0.5 | 0.1 | | 15.10 | 26.63 | 16.78 | 38.80 | 21.87 | 12.24 | 33.67 | 40.00 |
| 75 | | | 0.5 | | | 6.3 | | 6.6 | | 3.2 | | 21.8 | |
| 76 | | | 0.75 | | | 8.0 | | 8.7 | | 5.5 | | 28.6 | |
| 77 | | | 1.5 | | | 15.1 | | 16.8 | | 35.6 | | 43.6 | |
| 78 | 0.0075 | 0.5 | 0.5 | 0.3 | | 49.3 | | 52.3 | 31.4 | 55.9 | 22.4 | 80.8 | 14.4 |
| 79 | 0.00125 | 1 | 0.5 | 0.04 | | 30.0 | 17.6 | 32.8 | 10.0 | 35.0 | 26.4 | 56.9 | 10.9 |
| 80 | 0.0005 | 1 | 0.75 | 0.3 | | 34.4 | 2.2 | 34.1 | 15.6 | 30.4 | | 46.7 | |
| 81 | 0.00125 | 2 | 0.75 | 0.1 | | 46.2 | 12.7 | 40.4 | | 64.5 | 8.1 | 82.8 | 10.3 |
| 82 | 0.00035 | 0.5 | 1.5 | 0.3 | | 42.68 | 25.90 | 39.00 | 28.29 | 49.83 | | 64.68 | 20.1 |
| | TA-2 | L-Ala-L-Gln | Man | 18βGA | KS | MCP-Prot | SYN | IL12-Prot | SYN | AcH-Prot | SYN | DNA-Prot | SYN |
| 83 | 0.00035 | | | | | 2.75 | | 2.60 | | 1.6 | | 2.38 | |
| 84 | 0.00125 | | | | | 7.61 | | 8.9 | | 7.9 | | 14.9 | |
| 85 | 0.0075 | | | | | 25.97 | | 20.5 | | 20.5 | | 38.1 | |
| 86 | 0.0075 | 0.5 | 0.5 | 0.3 | | 47.14 | 2.54 | 40.11 | 11.73 | 42.19 | | 79.38 | 11.50 |
| 87 | 0.00125 | 2 | 0.75 | 0.1 | | 44.51 | 16.48 | 48.41 | 9.76 | 64.41 | 13.59 | 70.65 | 11.00 |
| 88 | 0.00035 | 0.5 | 1.5 | 0.3 | | 36.16 | 8.09 | 32.56 | 10.87 | 45.77 | | 48.04 | |
| 89 | 0.00125 | 1 | 0.5 | 0.04 | | 27.35 | 33.98 | 30.34 | 23.32 | 32.73 | 22.12 | 53.63 | 19.73 |
| 90 | 0.00035 | 1 | 0.75 | 0.3 | | 32.15 | 5.93 | 31.45 | 17.05 | 42.38 | 14.84 | 39.45 | 6.91 |
| 91 | 0.00035 | | | | | 3.11 | | 3.2 | | 2.2 | | 4.11 | |
| 92 | 0.00125 | | | | | 9.69 | | 13.4 | | 10.2 | | 15.8 | |
| 93 | 0.0075 | | | | | 30.01 | | 23.2 | | 24.8 | | 42.9 | |
| 94 | 0.0075 | 0.5 | 0.5 | 0.3 | | 54.12 | 8.22 | 45.37 | 17.54 | 44.98 | | 76.20 | 11.29 |

(continued)

| No. | TA-2 | L-Ala-L-Gln | Man | 18βGA | KS | MCP-Prot | SYN | IL12-Prot | SYN | AcH-Prot | SYN | DNA-Prot | SYN |
|---|---|---|---|---|---|---|---|---|---|---|---|---|---|
| 95 | 0.00125 | 2 | 0.75 | 0.1 | | 55.84 | 38.58 | 57.59 | 18.49 | 67.83 | 14.97 | 79.40 | 10.17 |
| 96 | 0.00035 | 0.5 | 1.5 | 0.3 | | 34.32 | 1.52 | 32.26 | 7.54 | 44.59 | | 49.82 | |
| 97 | 0.00125 | 1 | 0.5 | 0.04 | | 31.69 | 40.92 | 33.82 | 16.21 | 35.86 | 23.22 | 59.69 | 30.64 |
| 98 | 0.00035 | 1 | 0.75 | 0.3 | | 43.59 | 41.95 | 38.85 | 41.28 | 40.93 | 9.14 | 64.20 | 31.37 |

| No. | TA-01 | CP | Man | 18βGA | KS | MCP-Prot | SYN | IL12-Prot | SYN | AcH-Prot | SYN | DNA-Prot | SYN |
|---|---|---|---|---|---|---|---|---|---|---|---|---|---|
| 99 | .. | 0.1 | .. | .. | | 4.35 | | 3.68 | | 4.3 | | 3.48 | |
| 100 | | 0.2 | | | | 6.75 | | 6.34 | | 8.3 | | 7.57 | |
| 101 | | 0.5 | | | | | | 17.6 | | 21.66 | | 32.87 | |
| 102 | | 2 | | | | | | 24.6 | | | | 33.13 | |
| 103 | 0.00125 | 0.2 | 0.5 | 0.3 | | 40.96 | 27.41 | 34.93 | 17.43 | 41.42 | 5.68 | 59.89 | 23.65 |
| 104 | 0.00035 | 0.1 | 0.75 | 0.3 | | 29.98 | 25.17 | 25.89 | 30.26 | 29.57 | 9.91 | 45.16 | 16.32 |
| 105 | 0.00035 | 0.1 | 0.5 | 0.04 | | 12.44 | 35.94 | 14.05 | 24.34 | 13.75 | 30.98 | 32.20 | 35.64 |

Man= D-Mannitol, Sor = D-Sorbitol, KS = Potassium Sorbate, Syn= Synergistic, Pro = Protection, GSSG= Oxidised-L- Glutathione, CP= Casein Protein Hydrolysate

| | Tannin | Amino Acid | SugA lco | Sap Gly | ALT | ALT | AST | AST | ALP | ALP | MCP-1 | MCP-1 | IL12 | IL12 | ACH ase | ACH ase |
|---|---|---|---|---|---|---|---|---|---|---|---|---|---|---|---|---|
| Gr. | TA-01 | L-Ala-L-Gln | Man | 18β GA | Prot | SYN | Prot | SYN | Prot | SYN | Prot | SYN | Prot | SYN | Prot | SYN |
| 1 | 0.00035 | | | | 10.85 | | 13.18 | | 13.81 | | 7.34 | | 5.64 | | 1.3 | |
| 2 | 0.0005 | | | | 21.62 | | 12.28 | | 17.49 | | 8.52 | | 6.88 | | 1.6 | |
| 3 | 0.000625 | | | | 20.29 | | 0 | | 6.12 | | 9.54 | | 8.63 | | 3.3 | |
| 4 | 0.00125 | | | | 33.21 | | 2.95 | | 23.22 | | 19.68 | | 15.62 | | 8.6 | |
| 5 | 0.0025 | | | | 49.36 | | 37.7 | | 39.59 | | 28.78 | | 24.35 | | 14.7 | |
| 6 | 0.0050 | | | | 42.90 | | 43.92 | | 41.73 | | 33.19 | | 30.34 | | 19.8 | |
| 7 | | 0.5 | | | 9.1 | | 5.9 | | 7.2 | | 0.10 | | 2.64 | | 2.9 | |
| 8 | | 1 | | | 13.4 | | 15.7 | | 23.8 | | 7.14 | | 16.44 | | 11.8 | |
| 9 | | 1.5 | | | 14.68 | | 20.25 | | 22.35 | | 11.01 | | 22.53 | | 21 | |
| 10 | | 2 | | | 38.8 | | 30.7 | | 28.7 | | 19.16 | | 29.54 | | 30.4 | |
| 11 | | | 0.5 | | 3.6 | | 4.3 | | 4.8 | | 5.29 | | 5.08 | | 4.3 | |
| 12 | | | 0.75 | | 13.4 | | 10.6 | | 10.40 | | 6.17 | | 8.13 | | 5.1 | |
| 13 | | | 1 | | 16.4 | | 13.8 | | 13.92 | | 7.34 | | 12.38 | | 10.9 | |
| 14 | | | 1.5 | | 26.86 | | 28.52 | | 26.74 | | 12.65 | | 20.60 | | 29.6 | |
| 15 | | | 2.5 | | 38.4 | | 42.6 | | 34.7 | | 35.88 | | 31.99 | | 40.2 | |
| 16 | | | | 0.01 | 0.5 | | 0 | | 0.25 | | 0 | | 0 | | 0.2 | |
| 17 | | | | 0.02 | 1 | | 0 | | 1 | | 0.27 | | 1.02 | | 0.4 | |
| 18 | | | | 0.04 | 0.89 | | 2.18 | | 2.78 | | 3.38 | | 3.08 | | 1.3 | |
| 19 | | | | 0.1 | 4.24 | | 8.25 | | 6.29 | | 2.2 | | 5.85 | | 9.7 | |
| 20 | | | | 0.3 | 25.62 | | 20.46 | | 11.7 | | 15.46 | | 10.07 | | 16.6 | |
| 21 | 0.0025 | 1 | 0.5 | 0.1 | 75.02 | 2.61 | 56.87 | | 81.44 | 9.34 | 45.20 | 4.12 | 50.30 | | 35.1 | |

TABLE-2 (5 DAY DATA) - ALL VALUES ARE IN %

|    | TA-01 % | L-Ala-L-Gln % | Man % | 18β-GA % | Observed | % Syn | Observed | % Syn | Observed | % Syn | Observed | % Syn | Observed | % Syn | Observed | % Syn |
|----|---------|---------------|-------|----------|----------|-------|----------|-------|----------|-------|----------|-------|----------|-------|----------|-------|
| 22 | 0.00125 | 1 | 0.5 | 0.04 | 67.53 | 32.16 | 44.32 | 76.36 | 70.19 | 28.55 | 45.30 | 27.64 | 47.31 | 17.63 | 29.7 | 13.9 |
| 22 B | 0.000625 | 0.5 | 0.5 | 0.04 | 63.86 | 88.50 | 20.61 | 66.49 | 40.06 | 91.68 | 29.60 | 61.62 | 31.59 | 62.57 | 13.5 | 13.5 |

|    | TA-01% | L-Ala-L-Gln % | Man | 18β-GA % | Observed | % Syn | Observed | % Syn | Observed | % Syn | Observed | % Syn | Observed | % Syn | Observed | % Syn |
|----|--------|---------------|-----|----------|----------|-------|----------|-------|----------|-------|----------|-------|----------|-------|----------|-------|
| 23 | 0.000625 | 0.5 | | | 31.11 | 5.86 | 6.57 | 11.32 | 15.71 | 17.92 | 11.60 | 20.27 | 13.37 | 18.64 | 8.8 | 41.9 |
| 24 | 0.000625 | | 0.5 | | 28.91 | 21.02 | 5.47 | 27.21 | 12.40 | 13.56 | 18.30 | 23.36 | 15.12 | 10.26 | 8.2 | 7.9 |
| 25 | 0.000625 | | | 0.04 | 25.58 | 20.78 | 4.37 | 100.46 | 10.76 | 20.85 | 15.20 | 17.60 | 14.12 | 20.57 | 7.2 | 56.5 |
| 26 | 0.000625 | 0.5 | 0.5 | | 41.11 | 24.60 | 12.02 | 17.87 | 23.14 | 27.71 | 18.60 | 24.54 | 20.36 | 24.51 | 12.5 | 19.0 |
| 27 | 0.000625 | | 0.5 | 0.04 | 28.91 | 16.68 | 6.62 | 11.34 | 18.19 | 32.77 | 19.50 | 7.06 | 18.36 | 14.12 | 10.6 | 19.1 |
| 28 | 0.000625 | 0.5 | | 0.04 | 36.71 | 21.22 | 11.48 | 42.10 | 22.32 | 38.63 | 20.30 | 55.86 | 19.36 | 34.90 | 10.3 | 37.3 |
| 29 | 0.000625 | 1 | 0.5 | 0.02 | 47.77 | 25.11 | 30.60 | 52.99 | 42.15 | 18.00 | 30.20 | 35.76 | 37.32 | 19.74 | 21.3 | 7.6 |

|    | TA-01 | L-Ala-L-Gln | Man | 18β-GA | Observed | % Syn | Observed | % Syn | Observed | % Syn | Observed | % Syn | Observed | % Syn | Observed | % Syn |
|----|-------|-------------|-----|--------|----------|-------|----------|-------|----------|-------|----------|-------|----------|-------|----------|-------|
| 30 | 0.000625 | 1 | 0.5 | 0.01 | 43.24 | 14.42 | 29.27 | 46.33 | 43.18 | 23.47 | 36.90 | 67.92 | 38.57 | 27.93 | 21.1 | 7.3 |
| 31 | 0.005 | 0.5 | 0.5 | 0.04 | 47.29 | | 48.09 | | 59.09 | 4.56 | 44.20 | 5.34 | 40.57 | | 32.3 | 14.2 |
| 32 | 0.000625 | 0.5 | 2.5 | 0.04 | 70.28 | 2.33 | 54.36 | 12.65 | 52.27 | 12.89 | 52.27 | 6.88 | 55.54 | 19.85 | 54.2 | 13.6 |
| 33 | 0.000625 | 2 | 0.5 | 0.04 | 64.86 | 2.01 | 46.00 | 23.72 | 44.70 | 12.42 | 42.50 | 13.71 | 51.30 | 10.72 | 50.3 | 28.0 |
| 34 | 0.000625 | 0.5 | 0.5 | 0.3 | 60.81 | 3.75 | 43.20 | 40.90 | 35.61 | 19.40 | 35.61 | 17.14 | 38.32 | 45.05 | 32.3 | 19.2 |
| 35 | 0.0005 | 1 | 0.5 | 0.04 | 48.66 | 23.17 | 42.51 | 23.36 | 52.27 | 6.96 | 35.60 | 46.32 | 33.08 | 5.08 | 26.3 | 38.4 |
| 36 | 0.00035 | 0.5 | 0.5 | 0.04 | 28.08 | 14.89 | 28.58 | 11.82 | 32.27 | 12.86 | 25.60 | 58.89 | 22.60 | 37.51 | 12.6 | 28.57 |
| 37 | 0.0025 | 0.5 | 0.5 | 0.04 | 61.85 | | 49.35 | 10.46 | 55.84 | 2.70 | 50.20 | 33.68 | 41.57 | 18.27 | 30.2 | 30.17 |

|    | TA-01 | L-Ala-L-Gln | Man | 18α GA | ALT-Pro% | ALT-Syn % | AST-Pro% | AST-Syn % | ALP-Pro% | ALP-Syn% | MCP-1 Pro | MCP-1% Syn | IL12Pro | IL12% Syn | Observed | % Syn |
|----|-------|-------------|-----|--------|----------|-----------|----------|-----------|----------|----------|-----------|------------|---------|-----------|----------|-------|
| 38 | | | | 0.04 | 1.26 | | 2.24 | | 2.50 | | 1.17 | | 1.89 | | 0.9 | |

EP 3 257 513 A1

| | TA-01 | L-Ala-L-Gln | Man | 18α GA | ALT-Pro% | ALT-Syn % | AST-Pro% | AST-Syn % | ALP-Pro% | ALP-Syn% | MCP-1 Pro | MCP-1% Syn | IL12Pro | IL12% Syn | Observed | % Syn |
|---|---|---|---|---|---|---|---|---|---|---|---|---|---|---|---|---|
| 39 | | | | 0.3 | 26.79 | | 21.53 | | 10.80 | | 16.89 | | 12.87 | | 17.3 | |
| 40 | 0.000625 | 0.5 | 0.5 | 0.04 | 40.57 | 18.44 | 15.33 | 23.26 | 23.48 | 13.87 | 20.60 | 12.48 | 23.35 | 20.17 | 12.9 | 13.2 |
| 41 | 0.000625 | 0.5 | 0.5 | 0.3 | 59.43 | 2.50 | 36.60 | 15.34 | 32.57 | 12.64 | 25.30 | | 31.09 | 12.38 | 28.6 | 2.9 |
| 42 | 0.00035 | 0.5 | 0.5 | 0.04 | 29.79 | 20.07 | 29.86 | 16.55 | 31.13 | 9.98 | 25.30 | 82.01 | 22.35 | 46.58 | 11.9 | 26.6 |
| 43 | 0.0025 | 0.5 | 0.5 | 0.04 | 62.33 | | 51.29 | 12.29 | 57.34 | 10.01 | 52.70 | 49.12 | 45.56 | 34.15 | 30.6 | 34.2 |
| 44 | 0.000625 | 1 | 1 | 0.04 | 52.88 | 2.98 | 40.41 | 27.20 | 47.37 | 2.22 | 28.73 | 14.04 | 42.31 | 7.56 | 32.6 | 21.2 |
| 45 | 0.000625 | 1.5 | 1.5 | 0.04 | 70.14 | 11.10 | 54.11 | 6.11 | 63.51 | 10.05 | 38.87 | 13.07 | 55.79 | 3.98 | 59.3 | 8.2 |

| | TA-01 | GSSG | Man | 18β GA | ALT-Pro% | ALT-Syn % | AST-Pro% | AST-Syn % | ALP-Pro% | ALP-Syn% | MCP-1Pro% | MCP-1% Syn | IL12Pro | IL12% Syn | ACHase Pro % | ACH % Syn |
|---|---|---|---|---|---|---|---|---|---|---|---|---|---|---|---|---|
| 46 | | 0.2 | | | 0.8 | | 4.6 | | 2.6 | | 3.10 | | 0.80 | | | |
| 47 | | 0.50 | | | 7.59 | | 6.27 | | 6.22 | | 5.16 | | 1.42 | | 5.5 | |
| 48 | | 1.00 | | | 19.7 | | 9.06 | | 7.69 | | 6.30 | | 2.14 | | 10.3 | |
| 49 | 0.000625 | 0.5 | 0.5 | 0.04 | 37.82 | 16.82 | 17.42 | 36.64 | 22.80 | 14.44 | 24.80 | 6.10 | 22.60 | 24.11 | 16.9 | 17.6 |
| 50 | 0.00035 | 0.5 | 0.5 | 0.04 | 28.56 | 24.55 | 27.92 | 7.66 | 31.13 | 12.75 | 28.60 | 35.08 | 23.10 | 51.83 | 15.3 | 23.4 |
| 51 | 0.000625 | 0.5 | 1 | 0.04 | 51.58 | 14.19 | 23.97 | 7.57 | 32.64 | 12.39 | 20.30 | | 30.59 | 19.90 | 23.5 | 11.9 |
| 52 | 0.000625 | 1 | 0.5 | 0.04 | 47.95 | 7.80 | 20.00 | 28.70 | 25.90 | 21.10 | 22.30 | | 26.59 | 40.49 | 22.6 | 17.7 |
| 53 | 0.00035 | 0.2 | 0.5 | 0.1 | 21.63 | 11.00 | 32.18 | 6.10 | 28.08 | 2.12 | 20.85 | 16.26 | 20.12 | 15.84 | 25.8 | 38.6 |
| 54 | 0.0025 | 0.5 | 0.5 | 0.04 | 51.36 | | 46.61 | | 48.80 | | 39.43 | | 28.84 | | 24.8 | |

| | TA-01 | L-Ala-L-Gln | Sor | 18β GA | ALT-Pro% | ALT-Syn % | AST-Pro% | AST-Syn % | ALP-Pro% | ALP-Syn% | MCP-1Pro | MCP-1% Syn | IL12Pro | IL12% Syn | ACHase Pro % | ACH % Syn |
|---|---|---|---|---|---|---|---|---|---|---|---|---|---|---|---|---|
| 55 | | | 0.50 | | 4.4 | | 5.10 | | 5.70 | | 6.75 | | 7.14 | | 2.83 | |
| 56 | | | 1 | | 18.10 | | 14.30 | | 15.05 | | 12.32 | | 15.10 | | 18.2 | |
| 57 | | | 1.5 | | 30.28 | | 22.10 | | 28.53 | | 16.52 | | 22.30 | | 31.6 | |

(continued)

| | Sample | L-Ala-L-Gln | Sor / Man | 18β GA | ALT-Pro% | ALT-Syn % | AST-Pro% | AST-Syn % | ALP-Pro% | ALP-Syn% | MCP-1Pro | MCP-1% Syn | IL12Pro | IL12% Syn | ACHase Pro % | ACH % Syn |
|---|---|---|---|---|---|---|---|---|---|---|---|---|---|---|---|---|
| | **TA-01** | | Sor | | | | | | | | | | | | | |
| 58 | 0.000625 | 0.5 | 0.5 | 0.04 | 34.68 | 1.33 | 15.69 | 19.03 | 24.24 | 11.17 | 22.50 | 13.78 | 24.60 | 14.47 | 16.4 | 59.1 |
| 59 | 0.0025 | 0.5 | 0.5 | 0.04 | 70.14 | 10.02 | 57.54 | 13.08 | 52.64 | | 37.18 | | 41.01 | 10.21 | 28.4 | 30.7 |
| 60 | 0.000625 | 1 | 1 | 0.04 | 56.33 | 6.93 | 41.79 | 29.85 | 45.62 | 25.65 | 36.05 | 11.33 | 44.81 | 3.61 | 39.4 | 13.9 |
| 61 | 0.000625 | 0.5 | 0.5 | 0.3 | 63.23 | 6.43 | 33.55 | 6.65 | 38.60 | | 34.36 | 7.87 | 33.08 | 16.16 | 27.4 | 6.9 |
| 62 | 0.000625 | 1.5 | 1.5 | 0.04 | 70.14 | 6.04 | 51.72 | 16.03 | 57.02 | | 38.87 | | 61.53 | 8.82 | 55.3 | |
| 63 | 0.00035 | 0.5 | 0.5 | 0.04 | 32.19 | 27.54 | 29.22 | 10.85 | 31.89 | 8.14 | 24.10 | 37.15 | 23.60 | 27.60 | 12.8 | 53.7 |
| | **TA-02** | | Man | | Observed | % Syn | Observed | % Syn | Observed | % Syn | Observed | % Syn | Observed | % Syn | Observed | % Syn |
| 64 | 0.00035 | | | | 8.29 | | 4.58 | | 8.39 | | 5.6 | | 4.4 | | 1.7 | |
| 65 | 0.000625 | | | | 17.67 | | 2.14 | | 5.89 | | 8.96 | | 7.87 | | 3.5 | |
| 66 | 0.0025 | | | | 47.68 | | 34.69 | | 40.68 | | 26.2 | | 22.5 | | 15.8 | |
| 67 | 0.000625 | 0.5 | 0.5 | 0.04 | 32.67 | 4.51 | 18.57 | 24.05 | 25.01 | 10.07 | 20.85 | 17.60 | 21.60 | 15.69 | 16.0 | 33.6 |
| 68 | 0.0025 | 0.5 | 0.5 | 0.04 | 64.41 | 5.13 | 55.48 | 17.87 | 55.27 | 4.31 | 38.31 | 9.54 | 41.07 | 23.32 | 31.5 | 29.6 |
| 69 | 0.000625 | 0.5 | 0.5 | 0.3 | 58.63 | 4.72 | 37.68 | 14.88 | 35.09 | 18.59 | 32.67 | 9.83 | 28.84 | 12.40 | 29.3 | 7.3 |
| 70 | 0.000625 | 1.5 | 1.5 | 0.04 | 65.54 | 8.97 | 58.91 | 10.89 | 57.90 | 0.15 | 58.03 | 7.31 | 52.30 | | 45.9 | |
| 71 | 0.00035 | 0.5 | 0.5 | 0.04 | 26.43 | 20.77 | 21.91 | 29.19 | 28.07 | 21.16 | 16.91 | 17.65 | 18.86 | 24.08 | 14.8 | 45.1 |
| 72 | 0.000625 | 1 | 0.75 | 0.1 | 51.36 | 5.19 | 44.92 | 22.43 | 51.55 | 11.15 | 29.86 | 22.33 | 41.57 | 8.56 | 33.9 | 12.6 |
| | **TA-B** | | Man | | ALT-Pro% | ALT-Syn % | AST-Pro% | AST-Syn % | ALP-Pro% | ALP-Syn% | MCP-1Pro | MCP-1% Syn | IL12Pro | IL12% Syn | ACHase Pro % | ACH % Syn |
| 73 | 0.00035 | | | | 11.74 | | 10.62 | | 6.95 | | 8.2 | | 6.9 | | 2.2 | |
| 74 | 0.000625 | | | | 21.34 | | 2.86 | | 7.72 | | 10.5 | | 8.8 | | 4.6 | |
| 75 | 0.0025 | | | | 53.17 | | 36.16 | | 40.87 | | 30.1 | | 25.3 | | 17.6 | |
| 76 | 0.0025 | 0.5 | 0.5 | 0.04 | 70.14 | 5.06 | 56.86 | 10.32 | 64.91 | 16.64 | 42.25 | 8.69 | 44.81 | 24.13 | 37.7 | 44.4 |
| 77 | 0.000625 | 0.5 | 0.5 | 0.3 | 63.23 | 5.98 | 42.46 | 26.68 | 42.11 | 34.02 | 36.05 | 15.00 | 31.83 | 19.72 | 31.3 | 10.2 |

| | TA-B | L-Ala-L-Gln | Man | 18β GA | ALT-Pro% | ALT-Syn % | AST-Pro% | AST-Syn % | ALP-Pro% | ALP-Syn% | MCP-1Pro | MCP-1% Syn | IL12Pro | IL12% Syn | ACHas e Pro % | ACH % Syn |
|---|---|---|---|---|---|---|---|---|---|---|---|---|---|---|---|---|
| 78 | 0.000625 | 1.5 | 1.5 | 0.04 | 71.31 | 11.75 | 59.87 | 11.19 | 64.04 | 7.37 | 40.56 | 8.04 | 55.29 | 0.51 | 50.6 | |
| 79 | 0.00035 | 0.5 | 0.5 | 0.04 | 31.03 | 22.49 | 29.45 | 28.03 | 28.95 | 27.35 | 19.72 | 16.22 | 20.61 | 16.42 | 16.2 | 51.4 |
| 80 | 0.000625 | 1 | 0.75 | 0.1 | 54.05 | 2.97 | 41.53 | 11.01 | 56.07 | 16.30 | 30.98 | 19.14 | 43.56 | 11.07 | 34.7 | 11.3 |

Man= D-Mannitol, Sor = D-Sorbitol, KS = Potassium Sorbate, Syn= Synergistic, Pro = Protection, GSSG= Oxidised-L-Glutathione, CP= Casein Protein Hydrolysate

**Claims**

1. A synergistic composition for reducing DNA and Hepatic damage, reducing/relieving hangover/vesalgia, enhancing DNA and Hepatic repair and modulating/alleviating immunology and CNS parameters comprising of:

   (a) hydrolyzable tannin or pyrogallol-type tannin compounds in mass concentration range of 0.00035% to 0.025% and at least two active ingredients selected from:
   (b) an amino acid derived molecule in mass concentration range of 0.1% to 2.0%;
   (c) sugar alcohol in mass concentration range of 0.5% to 2.5 %; and
   (d) saponin glycoside in mass concentration range of 0.01% to 0.3 %.

2. The composition as claimed in claim 1, wherein the composition is selected from:

   (a) a composition comprising hydrolyzable tannin or pyrogallol-type tannin compounds, amino acid derived molecule and sugar alcohol;
   (b) a composition comprising hydrolyzable tannin or pyrogallol-type tannin compounds, amino acid derived molecule and saponin glycoside;
   (c) a composition comprising hydrolyzable tannin or pyrogallol-type tannin compounds, sugar alcohol and saponin glycoside; and
   (d) a composition comprising hydrolyzable tannin or pyrogallol-type tannin compounds, amino acid derived molecule, sugar alcohol and saponin glycoside.

3. The composition as claimed in claim 1, wherein the hydrolyzable tannin or pyrogallol-type tannin compounds is selected from the group consisting of tannic acid, epigallitannins, gallotannins, ellagitannins, condensed tannin, a complex tannin and hydrolyzable tannin or a combination thereof.

4. The composition as claimed in claim 3, wherein tannic acid is selected from group consisting of Tanal 01, Tanal 02 and Tanal B.

5. The composition as claimed in claim 1, wherein amino-acid derived molecule is selected from the group consisting of an amino-acid monomer, a dipeptide, a tripeptide, an oligopeptide, a polypeptide and a protein hydrolysate or a combination thereof.

6. The composition as claimed in claim 5, wherein the amino-acid monomer is selected from the group consisting of alanine, glutamine, arginine, ornithine, arginine pryoglutamate, asparaginine, L-Aspartic acid, D-Asparatic acid, L-Carnitine, citruline, cysteine, gamma-aminobutyric acid (GABA), glutathione, glycine, histidine, L-isoleucine, L-leucine, L-lysine, methionine, phenylalanine, L-proline, pyroglutamate, serine, threonine, tyrptophan, tyrosine, L-valine, and L-Theanine or a combination thereof.

7. The composition as claimed in claim 5, wherein dipeptide (DP) is selected from the group consisting of L-alanyl-L-glutamine (L-Ala-L-Gln), glycyl-glycine (Gly-Gly) and L-glutamyl-L-alanine (Glu-Ala) or a combination thereof.

8. The composition as claimed in claim 5, wherein oligopeptide is selected from the group consisting of Oxidised L-Glutathione (GSSG), Reduced L-Glutathione and Glutathione or a combination thereof.

9. The composition as claimed in claim 5, wherein the protein hydrolysate is selected from hydrolysed casein protein (CP) and hydrolysed whey protein (WP) or a combination thereof.

10. The composition as claimed in claim 1, wherein sugar alcohol is selected from the group consisting of D-Glycerol, L-Glycerol, D-Mannitol, L-Mannitol, D-erythritol, L-erythritol, D-xylitol, or L-xylitol, L-Maltitol, D-Maltitol, L-Sorbitol, D-Sorbitol, L-Lactitol, D-Lactitol, L-Isomalt and D-Isomalt or a combination thereof.

11. The composition as claimed in claim 10, wherein sugar alcohol is selected from the group consisting from D-Mannitol , L-Mannitol, D-Sorbitol and L-Sorbitol.

12. The composition as claimed in claim 1, wherein saponin glycoside comprises Glycyrrhizin (GA) or Glycyrrhizin (GA) salt, or a combination thereof.

13. The composition as claimed in claim 12, wherein the Glycyrrhizin (GA) is selected from 18β-Glycyrrhizin and 18α-Glycyrrhizin, or a combination thereof.

14. The composition as claimed in claim 12, wherein the Glycyrrhizin(GA) salt is selected from 18α-Glycyrrhizin mono ammonium salt and 18β-Glycyrrhizin mono ammonium salt or a combination thereof.

15. The composition as claimed in claim 13, wherein the Glycyrrhizin (GA) comprises equal parts of 18β-Glycyrrhizin and 18-α-Glycyrrhizin.

16. The composition as claimed in claim 1, wherein the composition further comprises pH adjusting agent selected from the group consisting of potassium sorbate (KS), monobasic sodium phosphate, dibasic sodium phosphate and tribasic sodium phosphate in a mass concentration range of 0.01 to 0.2%.

17. The composition as claimed in claim 1, wherein the composition further comprises flavouring agent selected from the group consisting of extracts of herbs, spices, fruit, and artificial flavour, in mass concentration range of 0.01 to 0.2%.

18. The composition as claimed in claim 1, wherein the composition further comprises quantum sufficient of water or alcohol or mixture of water and alcohol.

19. The composition as claimed in claim 1, wherein the composition is selected from:

| Sr. No. | TA-01 | L-Ala-L-Gla | Man | 18β-GA | KS |
|---|---|---|---|---|---|
| 1 | 0.0075 | 0.5 | 0.5 | 0.1 | |
| 2 | 0.00125 | 1 | 0.5 | 0.1 | |
| 3 | 0.00125 | 1 | 2.5 | 0.1 | |
| 4 | 0.00125 | 2 | 0.75 | 0.1 | |
| 5 | 0.025 | 0.5 | 0.5 | 0.1 | |
| 6 | 0.00125 | 1 | 0.5 | 0.04 | |
| 7 | 0.00125 | 0.5 | 0.5 | 0.04 | |
| 8 | 0.00125 | 1 | 0 | 0.1 | |
| 9 | 0.00125 | 1 | 0.5 | 0 | |
| 10 | 0.00125 | 0 | 0.5 | 0.1 | |
| 11 | 0.000625 | 1 | 0.75 | 0.3 | |
| 12 | 0.00125 | 1 | 0.5 | 0.02 | |
| 13 | 0.00035 | 0.5 | 1.5 | 0.3 | |
| 14 | 0.00035 | 0.5 | 2.5 | 0.3 | |
| 15 | 0.00035 | 1 | 1 | 0.3 | |
| 16 | 0.00035 | 1 | 0.75 | 0.3 | |
| | TA-01 | L-Ala-L-Gla | Man | 18α-GA | KS |
| 17 | 0.00125 | 2 | 0.75 | 0.1 | |
| 18 | 0.00125 | 1 | 0.5 | 0.04 | |
| 19 | 0.00125 | 1 | 2.5 | 0.3 | |
| 20 | 0.0075 | 0.5 | 0.5 | 0.3 | |
| 21 | 0.0005 | 1 | 0.75 | 0.3 | |
| 22 | 0.00035 | 0.5 | 1.5 | 0.3 | |

(continued)

| | TA-01 | L-Ala-L-Gla | Man | 18α-GA | KS |
|---|---|---|---|---|---|
| 23 | 0.00035 | 0.5 | 2.5 | 0.3 | |
| 24 | 0.00035 | 1 | 0.75 | 0.3 | |
| | TA-01 | L-Ala-L-Gla | Man | 18βGA | KS |
| 25 | 0.00125 | 0 | 0.5 | 0.1 | 0.1 |
| 26 | 0.00125 | 1 | 0.5 | 0.1 | 0.1 |
| | TA-01 | GSSG | Man | 18βGA | KS |
| 27 | 0.00125 | 1 | 0.5 | 0.04 | |
| 28 | 0.0005 | 0.5 | 2.5 | 0.3 | |
| 29 | 0.00125 | 2 | 0.75 | 0.1 | |
| 30 | 0.0005 | 1 | 0.75 | 0.3 | |
| 31 | 0.0075 | 0.5 | 0.5 | 0.3 | |
| 32 | 0.0005 | 0.5 | 1.5 | 0.1 | |
| 33 | 0.0005 | 1.5 | 1 | 0.1 | |
| 34 | 0.0075 | 1 | 0.75 | 0.3 | |
| 35 | 0.00125 | 0.2 | 0.5 | 0.3 | |
| 36 | 0.00035 | 0.2 | 0.75 | 0.3 | |
| 37 | 0.00035 | 0.2 | 0.5 | 0.1 | |
| 38 | 0.000625 | 0.1 | 0.5 | 0.1 | |
| 39 | 0.0005 | 0.1 | 0.5 | 0.1 | |
| | TA-01 | L-Ala-L-Gln | Sor | 18βGA | KS |
| 40 | 0.0075 | 0.5 | 0.5 | 0.3 | |
| 41 | 0.00125 | 1 | 0.5 | 0.04 | |
| 42 | 0.0005 | 1 | 0.75 | 0.3 | |
| 43 | 0.00125 | 2 | 0.75 | 0.1 | |
| 44 | 0.00035 | 0.5 | 1.5 | 0.3 | |
| | TA-2 | L-Ala-L-Gln | Man | 18βGA | KS |
| 45 | 0.0075 | 0.5 | 0.5 | 0.3 | |
| 46 | 0.00125 | 2 | 0.75 | 0.1 | |
| 47 | 0.00035 | 0.5 | 1.5 | 0.3 | |
| 48 | 0.00125 | 1 | 0.5 | 0.04 | |
| 49 | 0.00035 | 1 | 0.75 | 0.3 | |
| | TA-B | L-Ala-L-Gln | Man | 18βGA | KS |
| 50 | 0.0075 | 0.5 | 0.5 | 0.3 | |
| 51 | 0.00125 | 2 | 0.75 | 0.1 | |
| 52 | 0.00035 | 0.5 | 1.5 | 0.3 | |
| 53 | 0.00125 | 1 | 0.5 | 0.04 | |
| 54 | 0.00035 | 1 | 0.75 | 0.3 | |

(continued)

| | TA-01 | CP | Man | 18βGA | KS |
|---|---|---|---|---|---|
| 55 | 0.00125 | 0.2 | 0.5 | 0.3 | |
| 56 | 0.00035 | 0.1 | 0.75 | 0.3 | |
| 57 | 0.00035 | 0.1 | 0.5 | 0.04 | |
| | **TA-01** | **L-Ala-L-Gln %** | **Man %** | **18β-GA %** | |
| 58 | 0.0025 | 1 | 0.5 | 0.1 | |
| | **TA-01 %** | **L-Ala-L-Gln %** | **Man %** | **18β-GA %** | |
| 59 | 0.00125 | 1 | 0.5 | 0.04 | |
| 60 | 0.000625 | 0.5 | 0.5 | 0.04 | |
| | **TA-01%** | **L-Ala-L-Gln %** | **Man** | **18β-GA %** | |
| 61 | 0.000625 | 1 | 0.5 | 0.02 | |
| 62 | 0.000625 | 1 | 0.5 | 0.01 | |
| 63 | 0.005 | 0.5 | 0.5 | 0.04 | |
| 64 | 0.000625 | 0.5 | 2.5 | 0.04 | |
| 65 | 0.000625 | 2 | 0.5 | 0.04 | |
| 66 | 0.000625 | 0.5 | 0.5 | 0.3 | |
| 67 | 0.0005 | 1 | 0.5 | 0.04 | |
| 68 | 0.00035 | 0.5 | 0.5 | 0.04 | |
| 69 | 0.0025 | 0.5 | 0.5 | 0.04 | |
| | **TA-01** | **L-Ala-L-Gln** | **Man** | **18αGA** | |
| 70 | 0.000625 | 0.5 | 0.5 | 0.04 | |
| 71 | 0.000625 | 0.5 | 0.5 | 0.3 | |
| 72 | 0.00035 | 0.5 | 0.5 | 0.04 | |
| 73 | 0.0025 | 0.5 | 0.5 | 0.04 | |
| 74 | 0.000625 | 1 | 1 | 0.04 | |
| 75 | 0.000625 | 1.5 | 1.5 | 0.04 | |
| | **TA-01** | **GSSG** | **Man** | **18βGA** | |
| 76 | 0.000625 | 0.5 | 0.5 | 0.04 | |
| 77 | 0.00035 | 0.5 | 0.5 | 0.04 | |
| 78 | 0.000625 | 0.5 | 1 | 0.04 | |
| 79 | 0.000625 | 1 | 0.5 | 0.04 | |
| 80 | 0.00035 | 0.2 | 0.5 | 0.1 | |
| 81 | 0.0025 | 0.5 | 0.5 | 0.04 | |
| | **TA-01** | **L-Ala-L-Gln** | **Sor** | **18βGA** | |
| 82 | 0.000625 | 0.5 | 0.5 | 0.04 | |
| 83 | 0.0025 | 0.5 | 0.5 | 0.04 | |
| 84 | 0.000625 | 1 | 1 | 0.04 | |
| 85 | 0.000625 | 0.5 | 0.5 | 0.3 | |
| 86 | 0.000625 | 1.5 | 1.5 | 0.04 | |

(continued)

|  | | TA-01 | L-Ala-L-Gln | Sor | 18βGA | |
|---|---|---|---|---|---|---|
| | 87 | 0.00035 | 0.5 | 0.5 | 0.04 | |
| | | TA-02 | L-Ala-L-Gln | Man | 18βGA | |
| | 88 | 0.000625 | 0.5 | 0.5 | 0.04 | |
| | 89 | 0.0025 | 0.5 | 0.5 | 0.04 | |
| | 90 | 0.000625 | 0.5 | 0.5 | 0.3 | |
| | 91 | 0.000625 | 1.5 | 1.5 | 0.04 | |
| | 92 | 0.00035 | 0.5 | 0.5 | 0.04 | |
| | 93 | 0.000625 | 1 | 0.75 | 0.1 | |
| | | TA-B | L-Ala-L-Gln | Man | 18βGA | |
| | 94 | 0.0025 | 0.5 | 0.5 | 0.04 | |
| | 95 | 0.000625 | 0.5 | 0.5 | 0.3 | |
| | 96 | 0.000625 | 1.5 | 1.5 | 0.04 | |
| | 97 | 0.00035 | 0.5 | 0.5 | 0.04 | |
| | 98 | 0.000625 | 1 | 0.75 | 0.1 | |

20. A synergistic composition for reducing DNA damage, reducing/relieving hangover, enhancing DNA repair and modulating immunology parameters comprising:

(a) hydrolyzable tannin or pyrogallol-type tannin compounds in mass a concentration range of 0.00035 to 0.025%; and at least two active ingredients selected from :
(b) amino acid derived molecule in mass concentration range of 0.1 to 2.0%;
(c) sugar alcohol in mass concentration range of 0.5 to 2.5 %; and
(d) saponin glycoside in mass concentration range of 0.01 to 0.3 %.

21. The composition as claimed in claim 20 comprising:

(a) Tanal 01 or Tanal 02 or Tanal B in mass a concentration range of 0.00035 to 0.025%; and at least two active ingredients selected from the group comprising:
(b) amino acid derived molecule selected from L-alanyl-L-glutamine (L-Ala-L-Gln),oxidised L-glutathione (GSSG) and hydrolysed casein protein (CP) in mass concentration range of 0.1 to 2.0%;
(c) sugar alcohol selected from D-Mannitol, L-Mannitol, D-Sorbitol and L-Sorbitol in mass concentration range of 0.5 to 2.5 %; and
(d) saponin glycoside selected from 18-α-Glycyrrhizin and 18-β-Glycyrrhizin or mixture of 18-α-Glycyrrhizin and 18-β-Glycyrrhizin in mass concentration range of 0.01 to 0.3 %.

22. A synergistic composition for reducing DNA damage and enhancing DNA repair wherein composition is selected from:

(i)a composition comprising Tanal 01in mass concentration range of 0.00035% to 0.025%, L-Ala-L-Gln in mass concentration range of 0.5% to 2.0%, D-Mannitol or L-Mannitol in mass concentration range of 0.5% to 2.5%, and 18β-Glycyrrhizin in mass concentration range of 0.02% to 0.3%;
(ii)a composition comprising Tanal 02 in mass concentration range of 0.00035% to 0.0075%, L-Ala-L-Glnin mass concentration range of 0.5% to 2.0%, D-Mannitol or L-Mannitol in mass concentration range of 0.5% to 0.75%, and 18β-Glycyrrhizin in mass concentration range of 0.04% to 0.3%;
(iii)a composition comprising Tanal Bin mass concentration range of 0.00035% to 0.0075%, L-Ala-L-Gln in mass concentration range of 0.5% to 2.0%, D-Mannitol or L-Mannitol in mass concentration range of 0.5% to 0.75%, and 18β-Glycyrrhizin in mass concentration range of 0.04% to 0.3%;
(iv)a composition comprising Tanal 01in mass concentration range of 0.00035% to 0.0075%, L-Ala-L-Gln in mass concentration range of 0.5% to 2.0%, D-Mannitolor L-Mannitol in mass concentration range of 0.5% to 2.5%, and 18α-Glycyrrhizin in mass concentration range of 0.04% to 0.3%;

(v) a composition comprising Tanal 01 in mass concentration range of 0.00035% to 0.000625%, Oxidised L-Glutathione (GSSG) in mass concentration range of 0.1% to 0.2%, D-Mannitol or L-Mannitol in mass concentration range of 0.5% to 0.75%, and 18β-Glycyrrhizin in mass concentration range of 0.1% to 0.3%;

(vi) a composition comprising Tanal 01 in mass concentration range of 0.00035% to 0.00125%, hydrolysed casein protein (CP) in mass concentration range of 0.1% to 0.2%, D-Mannitol or L-Mannitol in mass concentration range of 0.5% to 0.75%, and 18β-Glycyrrhizin in mass concentration range of 0.04% to 0.3%; and

(vii) a composition comprising Tanal 01 in mass concentration range of 0.00035% to 0.0075%, L-Ala-L-Gln in mass concentration range of 0.5% to 2.0%, D-Sorbitol L-Sorbitol in mass concentration range of 0.5% to 1.5%, and 18β-Glycyrrhizin in mass concentration range of 0.04% to 0.3 %.

23. A synergistic composition for reducing Hepatic damage, enhancing Hepatic repair and alleviating CNS parameters comprising:

(a) hydrolyzable tannin or pyrogallol-type tannin compounds in mass a concentration range of 0.00035 to 0.005%; and at least two active ingredients selected from group comprising:
(b) amino acid derived molecule in mass concentration range of 0.5 to 2.0%;
(c) sugar alcohol in mass concentration range of 0.5 to 2.5 %; and
(d) saponin glycoside in mass concentration range of 0.01 to 0.3 %.

24. The composition as claimed in claim 23, comprising:

(a) Tanal 01 or Tanal 02 or Tanal B in mass a concentration range of 0.00035 to 0.005%; and at least two other active ingredients selected from group comprising:
(b) amino acid derived molecule selected from L-alanyl-L-glutamine (L-Ala-L-Gln) or oxidised L-glutathione (GSSG) in mass concentration range of 0.5 to 2.0%;
(c) sugar alcohol selected from D-Mannitol or L-Mannitol or D-Sorbitol or L-Sorbitol in mass concentration range of 0.5 to 2.5 %; and
(d) saponin glycoside selected from 18-α-Glycyrrhizin or 18-β-Glycyrrhizin or mixture of 18-α-Glycyrrhizin and 18-β-Glycyrrhizin in mass concentration range of 0.01 to 0.3 %.

25. A synergistic composition for reducing hangover and alleviating hangover parameters wherein composition is selected from:

(i) a composition comprising Tanal 01 in mass concentration range of 0.00035% to 0.025%, L-Ala-L-Gln in mass concentration range of 0.5% to 2.0%, D-Mannitol or L-Mannitol in mass concentration range of 0.5% to 2.5%, and 18β-Glycyrrhizin in mass concentration range of 0.02% to 0.3%;

(ii) a composition comprising Tanal 02 in mass concentration range of 0.00035% to 0.00125%, L-Ala-L-Gln in mass concentration range of 0.5% to 2.0%, D-Mannitol or L-Mannitol in mass concentration range of 0.5% to 1.5%, and 18β-Glycyrrhizin in mass concentration range of 0.04% to 0.3%;

(iii) a composition comprising Tanal B in mass concentration range of 0.00035% to 0.0075%, L-Ala-L-Gln in mass concentration range of 0.5% to 2.0%, D-Mannitol or L-Mannitol in mass concentration range of 0.5% to 1.5%, and 18β-Glycyrrhizin in mass concentration range of 0.04% to 0.3%;

(iv) a composition comprising Tanal 01 in mass concentration range of 0.0005% to 0.0075%, L-Ala-L-Gln in mass concentration range of 0.5% to 1.0%, D-Mannitol or L-Mannitol in mass concentration range of 0.5% to 2.5%, and 18α-Glycyrrhizin in mass concentration range of 0.04% to 0.3%;

(v) a composition comprising Tanal 01 in mass concentration range of 0.00035% to 0.0075%, Oxidised L-Glutathione (GSSG) in mass concentration range of 0.1% to 1.5%, D-Mannitol or L-Mannitol in mass concentration range of 0.5% to 2.5%, and 18β-Glycyrrhizin in mass concentration range of 0.04% to 0.3%;

(vi) a composition comprising Tanal 01 in mass concentration range of 0.00035% to 0.00125%, hydrolysed casein protein (CP) in mass concentration range of 0.1% to 0.2%, D-Mannitol or L-Mannitol in mass concentration range of 0.5% to 0.75%, and 18β-Glycyrrhizin in mass concentration range of 0.04% to 0.3%; and

(vii) a composition comprising Tanal 01 in mass concentration range of 0.00125% to 0.0075%, L-Ala-L-Gln in mass concentration range of 0.5% to 2.0%, D-Sorbitol or L-Sorbitol in mass concentration range of 0.5% to 1.5%, and 18β-Glycyrrhizin in mass concentration range of 0.04% to 0.3%.

26. A synergistic composition for alleviating immunology parameters, wherein composition is selected from :

(i) a composition comprising Tanal 01 in mass concentration range of 0.00035% to 0.025%, L-Ala-L-Gln in mass concentration range of 0.5% to 2.0%, D-Mannitol or L-Mannitol in mass concentration range of 0.5% to 2.5%, and 18β-Glycyrrhizin in mass concentration range of 0.01% to 0.3%;

(ii) a composition comprising Tanal 02 in mass concentration range of 0.00035% to 0.0075%, L-Ala-L-Gln in mass concentration range of 0.5% to 2.0%, D-Mannitol or L-Mannitol in mass concentration range of 0.5% to 1.5%, and 18β-Glycyrrhizin in mass concentration range of 0.04% to 0.3%;

(iii) a composition comprising Tanal B in mass concentration range of 0.00035% to 0.0075%, L-Ala-L-Gln in mass concentration range of 0.5% to 2.0%, D-Mannitol or L-Mannitol in mass concentration range of 0.5% to 1.5%, and 18β-Glycyrrhizin in mass concentration range of 0.04% to 0.3%;

(iv) a composition comprising Tanal 01 in mass concentration range of 0.00035% to 0.0075%, L-Ala-L-Gln in mass concentration range of 0.5% to 2.0%, D-Mannitol or L-Mannitol in mass concentration range of 0.5% to 2.5%, and 18α-Glycyrrhizin in mass concentration range of 0.04% to 0.3%;

(v) a composition comprising Tanal 01 in mass concentration range of 0.00035% to 0.0075%, Oxidised L-Glutathione (GSSG) in mass concentration range of 0.1% to 2.0%, D-Mannitol or L-Mannitol in mass concentration range of 0.5% to 2.5%, and 18β-Glycyrrhizin in mass concentration range of 0.04% to 0.3%;

(vi) a composition comprising Tanal 01 in mass concentration range of 0.00035% to 0.00125%, hydrolysed casein protein (CP) in mass concentration range of 0.1% to 0.2%, D-Mannitol or L-Mannitol in mass concentration range of 0.5% to 0.75%, and 18β-Glycyrrhizin in mass concentration range of 0.04% to 0.3%; and

(vii) a composition comprising Tanal 01 in mass concentration range of 0.00035% to 0.0075%, L-Ala-L-Gln in mass concentration range of 0.5% to 2.0%, D-Sorbitol or L-Sorbitol in mass concentration range of 0.5% to 1.5%, and 18β-Glycyrrhizin in mass concentration range of 0.04% to 0.3%.

27. A synergistic composition for reducing hepatic damage and enhancing hepatic repair wherein composition is selected from:

(i) a composition comprising Tanal 01 in mass concentration range of 0.00035% to 0.0025%, L-Ala-L-Gln in mass concentration range of 0.5% to 2.0%, D-Mannitol or L-Mannitol in mass concentration range of 0.5% to 2.5%, and 18β-Glycyrrhizin in mass concentration range of 0.01% to 0.3%;

(ii) a composition comprising Tanal 01 in mass concentration range of 0.00035% to 0.0025%, L-Ala-L-Gln in mass concentration range of 0.5% to 1.5%, D-Mannitol or L-Mannitol in mass concentration range of 0.5% to 1.5%, and 18α-Glycyrrhizin in mass concentration range of 0.04% to 0.3%;

(iii) a composition comprising Tanal 01 in mass concentration range of 0.00035% to 0.000625%, Oxidised L-Glutathione (GSSG) in mass concentration range of 0.5% to 1.0%, D-Mannitol or L-Mannitol in mass concentration range of 0.5% to 1.0%, and 18β-Glycyrrhizin in mass concentration range of 0.04% to 0.1%;

(iv) a composition comprising Tanal 01 in mass concentration range of 0.00035% to 0.0025%, L-Ala-L-Gln in mass concentration range of 0.5% to 1.5%, D-Sorbitol or L- Sorbitol in mass concentration range of 0.5% to 1.5%, and 18β-Glycyrrhizin in mass concentration range of 0.04% to 0.3%;

(v) a composition comprising Tanal 02 in mass concentration range of 0.00035% to 0.0025%, L-Ala-L-Gln in mass concentration range of 0.5% to 1.0%, D-Mannitol or L-Mannitol in mass concentration range of 0.5% to 1.5%, and 18β-Glycyrrhizin in mass concentration range of 0.04% to 0.3%; and

(vi) a composition comprising Tanal B in mass concentration range of 0.00035% to 0.0025%, L-Ala-L-Gln in mass concentration range of 0.5% to 1.5%, D-Mannitol or L-Mannitol in mass concentration range of 0.5% to 1.5%, and 18β-Glycyrrhizin in mass concentration range of 0.04% to 0.3%.

28. A synergistic composition for reducing CNS stress and modulating and/or alleviating CNS parameters, wherein composition is selected from:

(i) a composition comprising Tanal 01 in mass concentration range of 0.00035% to 0.005%, L-Ala-L-Gln in mass concentration range of 0.5% to 2.0%, D-Mannitol or L-Mannitol in mass concentration range of 0.5% to 2.5%, and 18β-Glycyrrhizin in mass concentration range of 0.01% to 0.3%;

(ii) a composition comprising Tanal 01 in mass concentration range of 0.00035% to 0.0025%, L-Ala-L-Gln in mass concentration range of 0.5% to 1.5%, D-Mannitol or L-Mannitol in mass concentration range of 0.5% to 1.5%, and 18α-Glycyrrhizin in mass concentration range of 0.04% to 0.3%;

(iii) a composition comprising Tanal 01 in mass concentration range of 0.00035% to 0.000625%, Oxidised L-Glutathione (GSSG) in mass concentration range of 0.5% to 1.0%, D-Mannitol or L-Mannitol in mass concentration range of 0.5% to 1.0%, and 18β-Glycyrrhizin in mass concentration range of 0.04% to 0.1%;

(iv) a composition comprising Tanal 01 in mass concentration range of 0.00035% to 0.0025%, L-Ala-L-Gln in mass concentration range of 0.5% to 1.5%, D-Sorbitol or L- Sorbitol in mass concentration range of 0.5% to 1.5%, and 18β-Glycyrrhizin in mass concentration range of 0.04% to 0.3%;

(v) a composition comprising Tanal 02 in mass concentration range of 0.00035% to 0.0025%, L-Ala-L-Gln in mass concentration range of 0.5% to 1.0%, D-Mannitol or L-Mannitol in mass concentration range of 0.5% to 0.75%, and 18β-Glycyrrhizin in mass concentration range of 0.04% to 0.3%;

(vi) a composition comprising Tanal B in mass concentration range of 0.00035% to 0.0025%, L-Ala-L-Gln in mass concentration range of 0.5% to 1.0%, D-Mannitol or L-Mannitol in mass concentration range of 0.5% to 0.75%, and 18β-Glycyrrhizin in mass concentration range of 0.04% to 0.3%.

29. The composition as claimed in claim 1 wherein the composition is formulated as a beverage additive which is added to a beverage at the time of drinking or formulated as a ready to drink beverage wherein the beverage additive is mixed with the beverage at the time of preparation of the beverage.

30. The synergistic composition as claimed in claim 29, wherein the beverage is an alcoholic beverage.

31. A process for the preparation of synergistic composition as claimed in claim 1, comprising the steps of:

(a) obtaining water or alcohol or alcohol-water mixture;
(b) mixing two or more ingredients selected from group comprising sugar alcohol, hydrolysable tannin and amino acid derived molecules in a reactor portion wise in small quantities containing water or alcohol or alcohol-water mixture of step (a);
(c) after mixing above ingredients saponin glycoside is added portion wise;
(d) adjusting the pH of the resulting solution of step (c) between 4.0 - 10; and
(e) obtaining the required beverage composition, filtering and bottling.

| Figure-1 | Figure-2 | Figure-3 | Figure-4 |
| Figure-5 | Figure-6 | Figure-7 | Figure-8 |

## STABILITY RESULTS FOR 45 DAYS (TABLE 4)

| | | | |
|---|---|---|---|
| Figure-9 | Figure-10 | Figure-11 | Figure-12 |
| Figure-13 | Figure-14 | Figure-15 | |

**STABILITY RESULTS FOR 45 DAYS (TABLE 4)**

| | | | | |
|---|---|---|---|---|
| | | | | |
| Figure-16 | Figure-17 | Figure-18 | Figure-19 | Figure-20 |
| | | | | |
| Figure-21 | Figure-22 | Figure-23 | Figure-24 | Figure-25 |

**STABILITY RESULTS FOR 60 DAYS (TABLE 6)**

| | | | | |
|---|---|---|---|---|
| | | | | |
| Figure-26 | Figure-27 | Figure-28 | Figure-29 | Figure-30 |
| | | | | |
| Figure-31 | Figure-32 | Figure-33 | | |

**STABILITY RESULTS FOR 60 DAYS (TABLE 6)**

Europäisches
Patentamt

European
Patent Office

Office européen
des brevets

**EUROPEAN SEARCH REPORT**

Application Number

EP 17 17 6645

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A,P | WO 2016/203499 A1 (CHIGURUPATI HARSHA [IN]) 22 December 2016 (2016-12-22) * the whole document * * claims 1-23 * | 1-31 | INV. A61K31/7004 A61K31/198 A61K31/704 A61K38/00 A61P1/16 A61P25/00 A61P25/32 A61P37/04 |
| A | WO 2014/177989 A2 (CHIGURUPATI TECHNOLOGIES PRIVATE LTD [IN]) 6 November 2014 (2014-11-06) * the whole document * * claims 1-12 * | 1-31 | |
| A | ANURADHA SEHRAWAT ET AL: "Preventive effect of tannic acid on 2-acetylaminofluorene induced antioxidant level, tumor promotion and hepatotoxicity: a chemopreventive study", REDOX REPORT., vol. 11, no. 2, 1 April 2006 (2006-04-01), pages 85-95, XP055419983, GB ISSN: 1351-0002, DOI: 10.1179/135100006X101066 * the whole document * * abstract * * page 92 - page 93 * | 1-31 | |
| A | GULCIN ILHAMI ET AL: "Radical scavenging and antioxidant activity of tannic acid", ARABIAN JOURNAL OF CHEMISTRY, SAUDI CHEMICAL SOCIETY, SA, vol. 3, no. 1, 1 January 2010 (2010-01-01) , pages 43-53, XP026993097, ISSN: 1878-5352 [retrieved on 2010-01-01] * the whole document * * abstract * | 1-31 | |

TECHNICAL FIELDS
SEARCHED (IPC)

A61K

-/--

The present search report has been drawn up for all claims

| Place of search | Date of completion of the search | Examiner |
|---|---|---|
| Munich | 30 October 2017 | Economou, Dimitrios |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

**EUROPEAN SEARCH REPORT**

Application Number

EP 17 17 6645

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A,D | CHING-HSEIN CHEN ET AL: "The efficacy of protective effects of tannic acid, gallic acid, ellagic acid, and propyl gallate against hydrogen peroxide-induced oxidative stress and DNA damages in IMR-90 cells", MOLECULAR NUTRITION & FOOD RESEARCH, vol. 51, no. 8, 1 August 2007 (2007-08-01), pages 962-968, XP055419888, DE ISSN: 1613-4125, DOI: 10.1002/mnfr.200600230 * the whole document * * abstract * | 1-31 | |

TECHNICAL FIELDS SEARCHED (IPC)

The present search report has been drawn up for all claims

| Place of search | Date of completion of the search | Examiner |
|---|---|---|
| Munich | 30 October 2017 | Economou, Dimitrios |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 2 of 2

## ANNEX TO THE EUROPEAN SEARCH REPORT
## ON EUROPEAN PATENT APPLICATION NO.

EP 17 17 6645

This annex lists the patent family members relating to the patent documents cited in the above-mentioned European search report.
The members are as contained in the European Patent Office EDP file on
The European Patent Office is in no way liable for these particulars which are merely given for the purpose of information.

30-10-2017

| Patent document cited in search report | Publication date | Patent family member(s) | Publication date |
|---|---|---|---|
| WO 2016203499 A1 | 22-12-2016 | US 2016367478 A1<br>WO 2016203499 A1 | 22-12-2016<br>22-12-2016 |
| WO 2014177989 A2 | 06-11-2014 | AU 2014261082 A1<br>CA 2909855 A1<br>CN 105143428 A<br>EA 201592077 A1<br>EP 2992078 A2<br>JP 6194412 B2<br>JP 2016516442 A<br>US 2015141358 A1<br>US 2017071963 A1<br>WO 2014177989 A2 | 17-12-2015<br>06-11-2014<br>09-12-2015<br>31-05-2016<br>09-03-2016<br>06-09-2017<br>09-06-2016<br>21-05-2015<br>16-03-2017<br>06-11-2014 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- IN 201641020935
- EP 2218449 A
- WO 2007133076 A
- US 8911774 B
- US 8158608 B2
- US 4987123 A
- US 20100234308 A
- US 4596825 A
- US 20100037353 A1

### Non-patent literature cited in the description

- **TUTEJA, NARENDRA ; MOHAN B. SINGH ; MITHILESH K. MISRA ; PREM L. BHALLA ; RENU TUTEJA.** Molecular Mechanisms of DNA Damage and Repair: Progress in Plants. *Critical Reviews in Biochemistry and Molecular Biology,* 2001, vol. 36 (4), 337-97
- **JACKSON ; STEPHEN P. ; JIRI BARTEK.** The DNA-damage Response In Human Biology And Disease. *Nature,* 1071-078
- **TUTEJA ; NARENDRA ; MOHAN B. SINGH ; MITHILESH K. MISRA ; PREM L. BHALLA ; RENU TUTEJA.** Molecular Mechanisms of DNA Damage and Repair: Progress in Plants. *Critical Reviews in Biochemistry and Molecular Biology,* 2001, vol. 36 (4), 337-97
- **DIZDAROGLU ; MIRAL ; PAWEL JARUGA ; MUSTAFA BIRINCIOGLU ; HENRY RODRIGUEZ.** Free Radical-induced Damage to DNA: Mechanisms and Measurement.'' This Article Is Part of a Series of Reviews on ''Oxidative DNA Damage and Repair. *Free Radical Biology and Medicine,* 2002, vol. 32 (11), 1102-115
- DNA Repair, Human Diseases and Aging. **VAIDEHI KRISHNAN ; BAOHUA LIU ; ZHONGJUN ZHOU.** DNA Repair and Human Health. 612-6
- **AMES ; BRUCE N.** Micronutrient Deficiencies: A Major Cause of DNA Damage. *Annals NY Acad Sci Annals of the New York Academy of Sciences,* 87-106
- **SUNITA KUMARI ; RAJESH P. RASTOGI ; KANCHAN L. SINGH ; SHAILENDRA P. SINGH ; RAJESHWAR P. SINHA.** DNA Damage: Detection Strategies. *EXCLI Journal,* 2008, vol. 7, 44-62
- **COOKE, M. S.** Oxidative DNA Damage: Mechanisms, Mutation, And Disease. *The FASEB Journal,* 2003, vol. 17 (10), 1195-1214
- **SOUZA-PINTO ; NADJA C. DE ; DAVID M. WILSON ; TINNA V. STEVNSNER ; VILHELM A. BOHR.** Mitochondrial DNA, Base Excision Repair and Neurodegeneration. *DNA Repair,* 2008, vol. 7.7, 1098-109
- **SCHOPPET, M. ; MAISCH, B.** Alcohol and the heart. *Hertz,* 2001, vol. 26, 345-352
- **KUMAR ; COTRAN ; ROBBINS.** Robbins Basic Pathology. Saunders, 2003
- **NORDMANN, R. ; RIBIERE, C. ; ROUACH, H.** Implication of free radical mechanisms in ethanol-induced cellular injury. *Free Radic. Biol. Med.,* 1992, vol. 12, 219-240
- **MUTLU-TURKOGLU U ; DOGRU-ABBASOGLU S ; AYKAC-TOKER G ; MIRSAL H ; BEYAZYUREK M ; UYSAL M.** Increased lipid and protein oxidation and DNA damage in patients with chronic alcoholism. *J Lab Clin Med,* 2000, vol. 136, 287-291
- **YUE M ; YU CH ; REN K ; CHEN W ; LI Y.** Transient elevation of hepatic enzymes in volunteers after intake of alcohol. *Hepatobiliary and Pancreatic Diseases Interna-tional,* 2006, vol. 5 (1), 52-55
- **MANTLE, D. ; PREEDY, V.R.** Free radicals as mediators of alcohol toxicity. *Adverse Drug Reactions and Toxicological Reviews,* 1999, vol. 18 (4), 235-252
- **LIEBER, C. S. ; DECARLI, L. M.** The role of the hepatic microsomal ethanol oxidizing system (MEOS) for ethanol metabolism in vivo. *J. Pharmacol. Exp. Ther.,* 1972, vol. 181, 279-287
- **LIEBER, C. S.** Cytochrome P4502E1: its physiological and pathological role. *Physiol. Rev.,* 1997, vol. 77, 517-544
- **HALLIWELL, B.** Antioxidant defence mechanisms: from the beginning to the end. *Free Radic. Res.,* 1999, vol. 31, 261-272

- **CONDE DE LA ROSA et al.** Superoxide anions and hydrogen peroxide induce hepatocytes death by different mechanisms: Invovement of JNK and ERK MAP kinase. *J Hepatol,* 2006, vol. 44 (5), 918-929
- **ARAI M ; NAKANO S ; OKUNO F et al.** Endotoxin-induced hypercoagulability: a possitive aggravating factor of alcoholic liver disease. *Hepatology,* 1989, vol. 9 (6), 846-851
- **KIM et al.** Effects of Alcohol Hangover on Cytokine Production in Healthy Subjects. *Alcohol,* 2003, vol. 31 (3), 167-170
- **TERUNUMA, H et al.** Potential role of NK cells in the induction of immune responses: implications for NK cell-based immunotherapy for cancers and viral infections. *International Reviews of immunology,* 2008, vol. 27 (3), 93-110
- Effects of tannic acid and its related compounds on food mutagens or hydrogen peroxide-induced DNA strands breaks in human lymphocytes. *Mutation Research/Fundamental and Molecular Mechanisms of Mutagenesis,* 22 November 2004, vol. 556 (1-2), 75-82
- Suppressing effect of tannic acid on the frequencies of mutagen-induced sister-chromatid exchanges in mammalian cells. *Mutation Research/Fundamental and Molecular Mechanisms of Mutagenesis,* August 1989, vol. 213 (2), 195-203
- Effect of tannic acid, resveratrol and its derivatives, on oxidative damage and apoptosis in human neutrophils. *Food and Chemical Toxicology,* October 2015, vol. 84, 37-46
- The efficacy of protective effects of tannic acid, gallic acid, ellagic acid, and propyl gallate against hydrogen peroxide-induced oxidative stress and DNA damages in IMR-90 cells. *Molecular Nutrition & Food Research,* August 2007, vol. 51 (8), 962-968
- Ellagic acid induces apoptosis through inhibition of nuclear factor κB in pancreatic cancer cells. *World J Gastroenterol.,* 21 June 2008, vol. 14 (23), 3672-3680
- Oxyradicals and DNA damage. *Carcinogenesis,* vol. 21 (3), 361-370
- **GHARZOULI K.** *Exp. Toxic. Pathol.,* 2001, vol. 53, 175
- *J. Nutr.,* 1985, vol. 115, 890
- **W. XU-YINGA.** *Chemico-Biological Interactions,* 2009, vol. 181, 15-19
- **T, ZING.** *Chinese Journal of Modern Applied Pharmacy,* 2006, vol. 02, 15-19
- **XIAO-LAN LI et al.** *Int. J. Mol. Sci.,* 2011, vol. 12, 905
- **XIAO-LAN LI et al.** *Molecules,* 2012, vol. 17, 716-727
- **CHENG-HUNG CHUANG ; MIAO-LIN HU.** Use of whole blood directly for single-cell gel electrophoresis (comet) assay in vivo and white blood cells for in vitro assay. *Mutation Research,* 2004, vol. 564, 75-82
- **BEGMEYER HU ; BERNT E.** Methods of enzymatic analysis. VerlagChemie, Weinhelm, Academic press, 1974, vol. 2, 735
- **TIETZ ; NORBERT W.** Fundamentals of Clinical Chemistry. W.B. Saunders, 1970, 447
- **VARLEY, H. ; GOWENLOCK, A. H. ; BELL, M.** Practical Clinical Biochemistry. William Heinemann Medical Books Lts, 1980, 453
- **WOREK F et al.** Determination of acetylcholinesterase activity by the Ellman assay: A versatile tool for in vitro research on medical countermeasures against organophosphate poisoning. *Drug testing and analysis,* 2012, vol. 4, 282-291
- **WANG L ; LI Y ; CHEN J et al.** Ischemic Cerebral Tissue and MCP-1 Enhance Rat Bone Marrow Stromal Cell Migration in Interface Culture. *Experimental Hematology,* 2002, vol. 30 (7), 831-836
- **TRINCHIERI G.** Interleukin-12: A Cytokine at the Interface of Inflammation and Immunity. *Adv. Immunol.,* 1998, vol. 70, 83-243
- **ELLMAN GL et al.** A new and rapid colorimetric determination of acetylcholinesterase activity. *Biochem Pharmacol,* 1961, vol. 7, 88
- **COLLINS, A. R.** The comet assay for DNA damage and repair: principles, applications, and limitations. *Mol. Biotechnol.,* 2004, vol. 26 (3), 249-261
- **RICO EP et al.** *Toxicology Letters,* 2007, vol. 174, 25-30
- **VINOD TIWARI et al.** *Behavioural Brain Research,* 2009, vol. 203, 296-303